# EUROPEAN PATENT SPECIFICATION

(11) **EP 2 650 369 B1**
(45) Date of publication and mention of the grant of the patent: **29.04.2015**
(21) Application number: 13171674.8
(22) Date of filing: 31.07.2008
(51) Int. Cl.: C12N 15/11, A61K 31/711, A61K 39/39

(54) **Novel synthetic agonists of TLR9**
Neuartige synthetische TLR9-Agonisten
Nouveaux agonistes synthétiques de tlr9

(30) Priority: 01.08.2007 US 953251 P; 30.10.2007 US 983601 P; 12.11.2007 US 987151 P; 20.12.2007 US 15292 P
(43) Date of publication of application: 16.10.2013
(62) Divisional of application: 08796942.4
(73) Proprietor: Idera Pharmaceuticals, Inc., Cambridge, MA 02139 (US)
(72) Inventor: Kandimalla, Ekambar R., Hopkinton, MA 01748 (US); Reddy Putta, Mallikarjuna, Lexington, MA 02420 (US); Wang, Daqing, Bedford, MA 01730 (US); Yu, Dong, Westboro, MA 01581 (US); Lakshmi, Bhagat, Framingham, MA 01702 (US); Agrawal, Sudhir, Shrewsbury, MA 01545 (US)
(74) Representative: Schnappauf, Georg

(56) References cited:
- WO-A2-2005/001055
- WO-A2-2007/047396
- WO-A2-2007/055704
- WO-A2-2008/073959
- KANDIMALLA EKAMBAR R ET AL: "Immunomodulatory oligonucleotides containing a cytosine-phosphate-2'-deoxy-7-deazaguanosi ne motif as potent toll-like receptor 9 agonists", PROCEEDINGS OF THE NATIONAL ACADEMY OF SCIENCES, NATIONAL ACADEMY OF SCIENCES, US, vol. 102, no. 19, 10 May 2005 (2005-05-10) , pages 6925-6930, XP002523069, ISSN: 0027-8424, DOI: 10.1073/PNAS.0501729102

## Description

### BACKGROUND OF THE INVENTION

### Field of the invention

The invention relates to synthetic chemical compositions that are useful for modulation of Toll-Like Receptor (TLR)-mediated immune responses. In particular, the invention relates to agonists of Toll-Like Receptor 9 (TLR9) that generate unique cytokine and chemokine profiles.

### Summary of the related art

Toll-like receptors (TLRs) are present on many cells of the immune system and have been shown to be involved in the innate immune response (Hornung, V. et a.l, (2002) J. Immunol. 168:4531-4537). In vertebrates, this family consists of eleven proteins called TLR1 to TLR11 that are known to recognize pathogen associated molecular patterns from bacteria, fungi, parasites, and viruses (Poltorak, a. et al. (1998) Science 282:2085-2088; Underhill, D.M., et al. (1999) Nature 401:811-815; Hayashi, F. et. al (2001) Nature 410:1099-1103; Zhang, D. et al. (2004) Science 303:1522-1526; Meier, A. et al. (2003) Cell. Microbiol. 5:561-570; Campos, M.A. et al. (2001) J. Immunol. 167:416-423; Hoebe, K. et al. (2003) Nature 424: 743-748; Lund, J. (2003) J. Exp. Med. 198:513-520; Heil, F. et al. (2004) Science 303:1526-1529; Diebold, S.S., et al. (2004) Science 303:1529-1531; Hornung, V. et al. (2004) J. Immunol. 173:5935-5943).

TLRs are a key means by which vertebrates recognize and mount an immune response to foreign molecules and also provide a means by which the innate and adaptive immune responses are linked (Akira, S. et al. (2001) Nature Immunol. 2:675-680; Medzhitov, R. (2001) Nature Rev. Immunol. 1:135-145). Some TLRs are located on the cell surface to detect and initiate a response to extracellular pathogens and other TLRs are located inside the cell to detect and initiate a response to intracellular pathogens.

TLR9 is known to recognize unmethylated CpG motifs in bacterial DNA and in synthetic oligonucleotides. (Hemmi, H. et al. (2000) Nature 408:740-745). Other modifications of CpG-containing phosphorothioate oligonucleotides can also affect their ability to act as modulators of immune response through TLR9 (see, e.g., Zhao et al., Biochem. Pharmacol. (1996) 51:173-182; Zhao et al. (1996) Biochem Pharmacol. 52:1537-1544; Zhao et al. (1997) Antisense Nucleic Acid Drug Dev. 7:495-502; Zhao et al (1999) Bioorg. Med. Chem. Lett. 9:3453-3458; Zhao et al. (2000) Bioorg. Med. Chem. Lett. 10:1051-1054; Yu, D. et al. (2000) Bioorg. Med. Chem. Lett. 10:2585-2588; Yu, D. et al. (2001) Bioorg. Med. Chem. Lett. 11:2263-2267; and Kandimalla, E. et al. (2001) Bioorg. Med. Chem. 9:807-813). Naturally occurring agonists of TLR9 have been shown to produce anti-tumor activity (e.g. tumor growth and angiogenesis) resulting in an effective anti-cancer response (e.g. anti-leukemia) (Smith, J.B. and Wickstrom, E. (1998) J. Natl. Cancer Inst. 90:1146-1154). In addition, TLR9 agonists have been shown to work synergistically with other known anti-tumor compounds (e.g. cetuximab, irinotecan) (Vincenzo, D., et al. (2006) Clin. Cancer Res. 12(2):577-583).

Certain TLR9 agonists are comprised of 3'-3' linked DNA structures containing a core CpR dinucleotide, wherein the R is a modified guanosine (US Patent No. 7,276,489). In addition, specific chemical modifications have allowed the preparation of specific oligonucleotide analogs that generate distinct modulations of the immune response. In particular, structure activity relationship studies have allowed identification of synthetic motifs and novel DNA-based compounds that generate specific modulations of the immune response and these modulations are distinct from those generated by unmethylated CpG dinucleotides. (Kandimalla, E. et al. (2005) Proc. Natl. Acad. Sci. USA 102:6925-6930. Kandimalla, E. et al. (2003) Proc. Nat. Acad. Sci. USA 100:14303-14308; Cong, Y. et al. (2003) Biochem Biophys Res. Commun.310:1133-1139; Kandimalla, E. et al. (2003) Biochem. Biophys. Res. Commun. 306:948-953; Kandimalla, E. et al. (2003) Nucleic Acids Res. 31:2393-2400; Yu, D. et al. (2003) Bioorg. Med. Chem.11:459-464; Bhagat, L. et al. (2003) Biochem. Biophys. Res. Commun.300:853-861; Yu, D. et al. (2002) Nucleic Acids Res.30:4460-4469; Yu, D. et al. (2002) J. Med. Chem.45:4540-4548. Yu, D. et al. (2002) Biochem. Biophys. Res. Commun.297:83-90; Kandimalla. E. et al. (2002) Bioconjug. Chem.13:966-974; Yu, D. et al. (2002) Nucleic Acids Res. 30:1613-1619; Yu, D. et al. (2001) Bioorg. Med. Chem. 9:2803-2808; Yu, D. et al. (2001) Bioorg. Med. Chem. Lett. 11:2263-2267; Kandimalla, E. et al. (2001) Bioorg. Med. Chem. 9:807-813; Yu, D. et al. (2000) Bioorg. Med. Chem. Lett. 10:2585-2588; Putta, M. et al. (2006) Nucleic Acids Res. 34:3231-3238).

Kandimalla, E.R. et al. (2005) Proc. Natl. Acad. Sci. U S A 102:6925-6930 teaches 3 compounds comprising two oligonucleotides linked via a single glycerol linker wherein each oligonucleotide is 11 nucleotides in length. WO 2007/047396 teaches compounds that are antagonists of TLR9. WO 2007/055704 teaches compounds comprising two oligonucleotides linked via a single glycerol linker wherein each oligonucleotide is 11 nucleotides in length. WO 2005/001055 teaches immunostimulatory oligonucleotides having secondary structure (*e.g.,* 5' or 3' hairpin loops).

The inventors have surprisingly discovered that uniquely modifying the sequence flanking the core CpR dinucleotide, the linkages between nucleotides or the linkers connecting the oligonucleotides produces novel agonists of TLR9 that generate distinct in vitro and in vivo cytokine and chemokine profiles. This ability to "custom-tune" the cytokine and chemokine response to a CpR containing oligonucleotide provides the ability to prevent and/or treat various disease conditions in a disease-specific and even a patient-specific manner. Thus, there is a need for new oligonucleotide analog compounds to provide such custom-tuned responses.

### BRIEF SUMMARY OF THE INVENTION

The invention describes oligonucleotide-based compounds that individually provide distinct immune response profiles through their interactions as agonists with TLR9. The TLR9 agonists described herein are characterized by specific and unique chemical modifications, which provide their distinctive immune response activation profiles.

The TLR9 agonists described herein induce immune responses in various cell types and in various in vitro and in vivo experimental models, with each agonist providing a distinct immune response profile. The TLR9 agonists described herein are also useful in the prevention and/or treatment of various diseases, either alone, in combination with or co-administered with other drugs, or as adjuvants for antigens used as vaccines. As such, they are useful as tools to study the immune system, as well as to compare the immune systems of various animal species, such as humans and mice.

In a first aspect, the invention provides the immune modulatory compound 5'-TCG₂TCG₂TTU₁Y-M-YU₁TTG₂CTG₂CT-5' [5'-SEQ ID NO: 12-3'-M-3'-SEQ ID NO: 12-5']; wherein G₂ = AraG; U₁ = 2'-deoxy-U; M = cis,cis-1,3,5-cyclohexanetriol linker; Y = 1,3-propanediol linker.

In a second aspect, the invention provides a composition such as a pharmaceutical formulation comprising the immune modulatory compound according to the invention and a pharmaceutically acceptable carrier.

The invention also provides a vaccine comprising a pharmaceutical formulation according to the invention and further comprising an antigen.

In a third aspect, the invention provides a compound according to the invention for use in a method for generating an immune response in an individual. Generating a TLR9-mediated immune response in an individual may comprise administering to the individual a compound, pharmaceutical formulation or vaccine according to the invention.

In a fourth aspect, the invention provides a compound according to the invention for use in a method for therapeutically treating an individual having a disease or disorder where modulating an immune response would be beneficial. Therapeutically treating a patient having a disease or disorder may comprise administering to the patient a compound, pharmaceutical formulation or vaccine according to the invention.

In a fifth aspect, the invention provides a compound according to the invention for use in a method for prophylactically treating an individual having a disease or disorder where modulating an immune response would be beneficial. Preventing a disease or disorder may comprise administering to the patient a compound, pharmaceutical formulation or vaccine according to the invention.

### BRIEF DESCRIPTION OF THE DRAWINGS

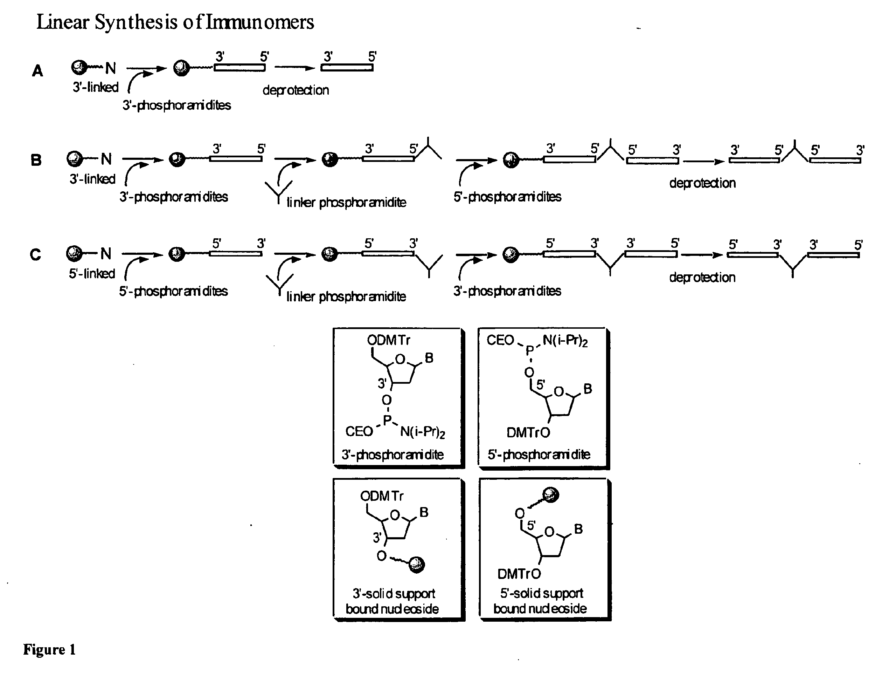

Figure 1 is a synthetic scheme for the linear synthesis of immune modulatory compounds described herein. DMTr = 4,4'-dimethoxytrityl; CE = cyanoethyl.

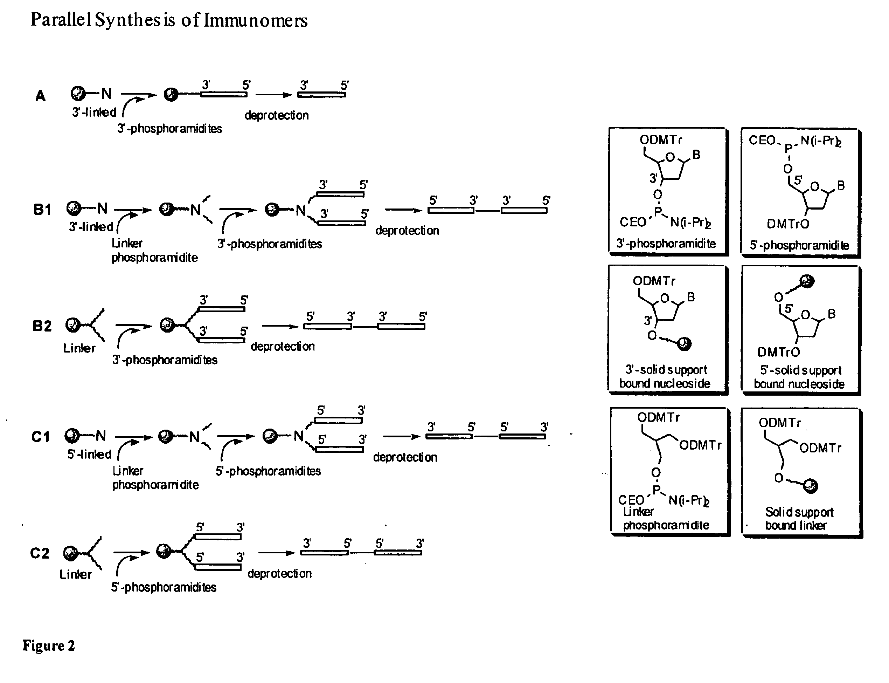

Figure 2 is a synthetic scheme for the parallel synthesis of immune modulatory compounds described herein. DMTr = 4,4'-dimethoxytrityl; CE = cyanoethyl.

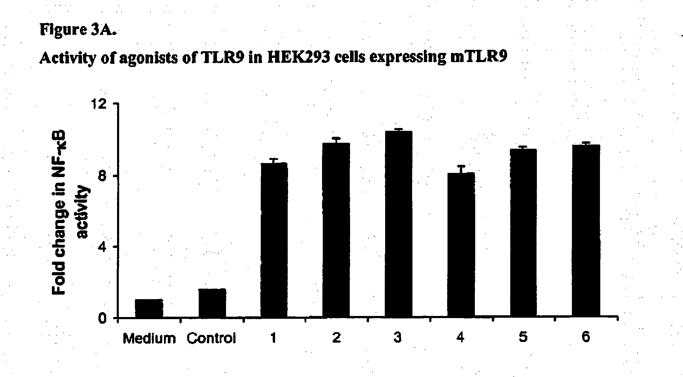

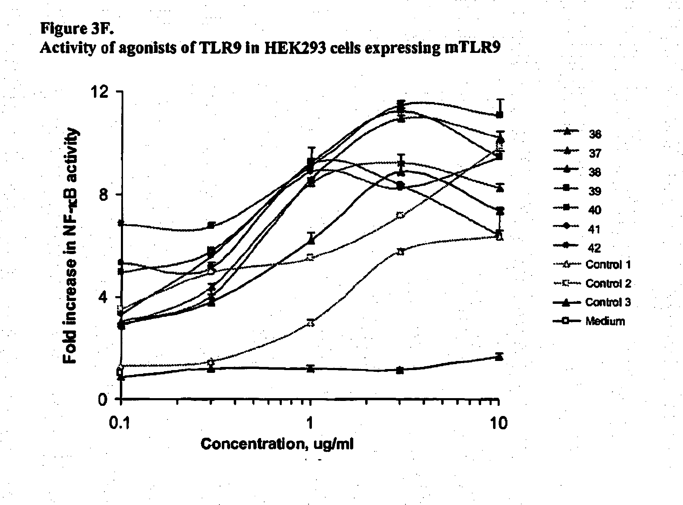

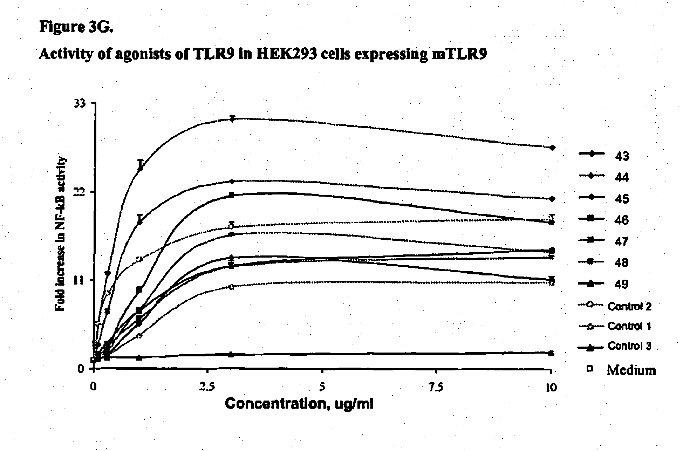

Figures 3A-3C depict NF-kB activity in HEK293 cells expressing TLR9 that were cultured, treated and analyzed according to Example 2 below. Briefly, the HEK293 cells were stimulated with 10 µg/ml of immune modulatory oligonucleotides described herein for 18 hours, and the levels of NF-κB were determined using SEAP (secreted form of human embryonic alkaline phosphatase) assay.

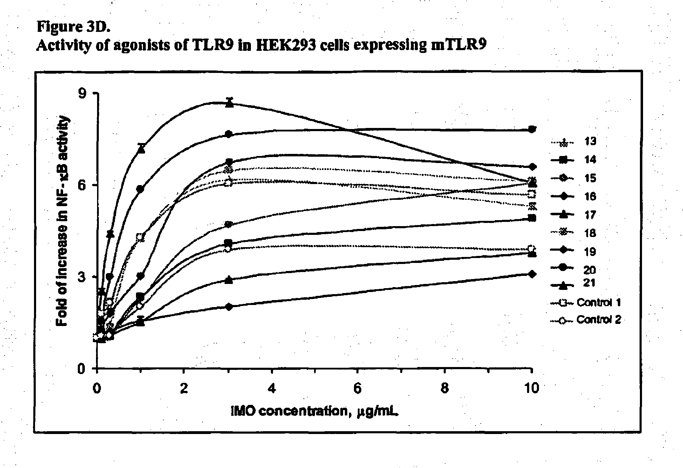

Figures 3D-3G depicts NF-kB activity in HEK293 cells expressing TLR9 that were cultured, treated and analyzed according to Example 2 below. Briefly, the HEK293 cells were stimulated with 0 (PBS/Media), 0.1, 0.3, 1.0, 3.0, or 10.0 µg/ml of immune modulatory oligonucleotides described herein for 18 hours, and the levels of NF-κB were determined using SEAP (secreted form of human embryonic alkaline phosphatase) assay. Figures 3A - 3G more generally demonstrate that administration of immune modulatory oligonucleotides containing novel bases, linkers, and/or unique modifications described herein generates distinct TLR9 activation profiles.

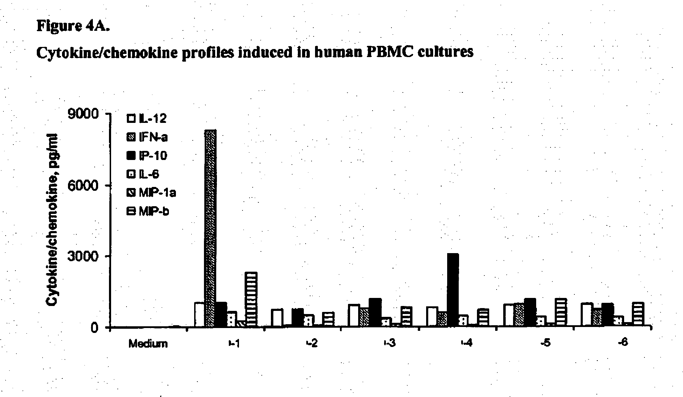

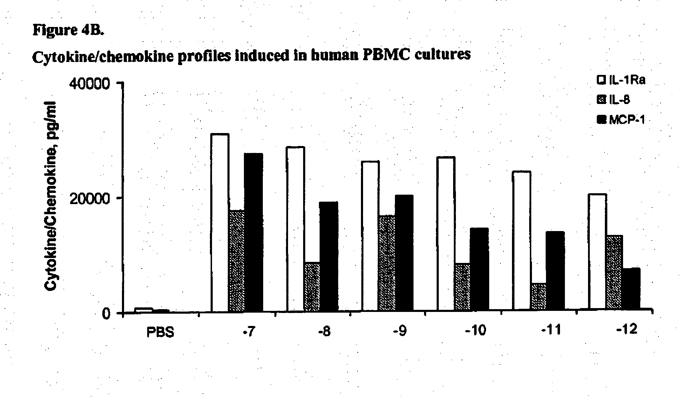

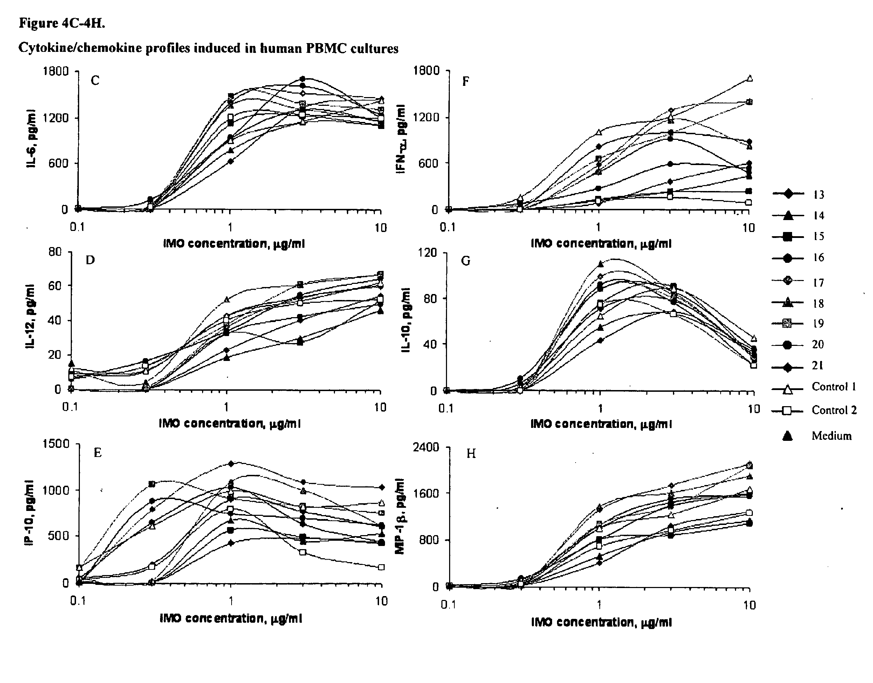

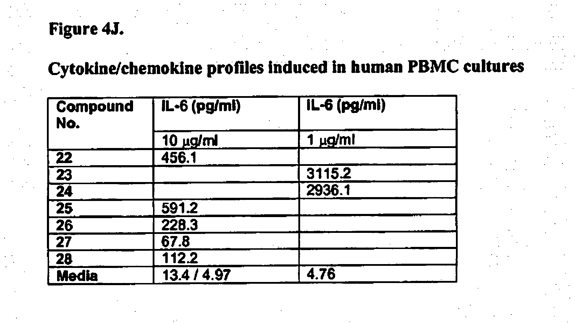

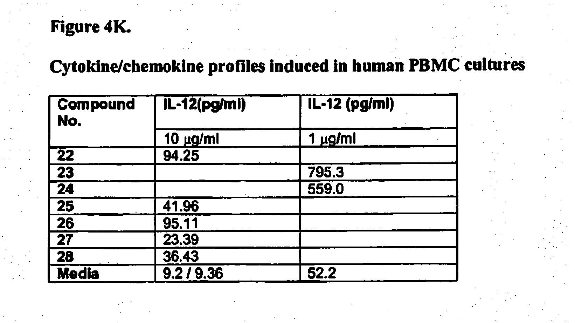

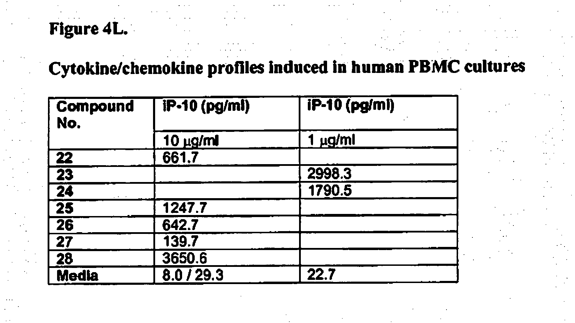

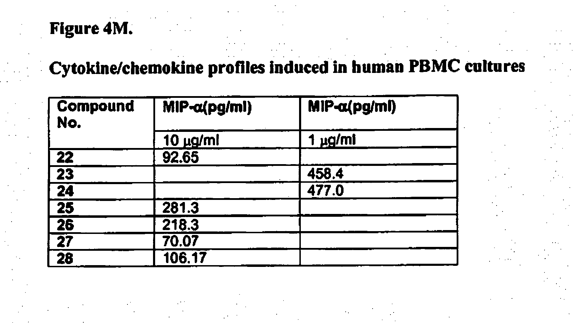

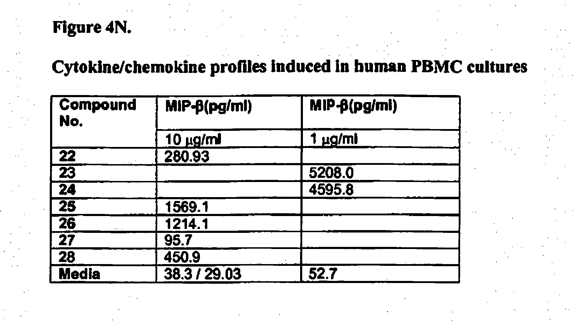

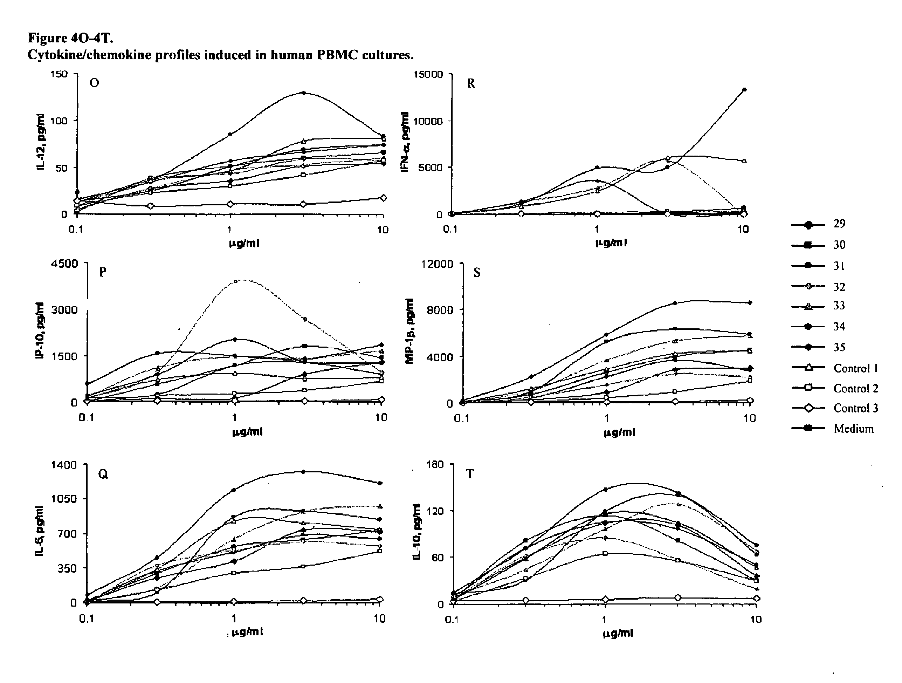

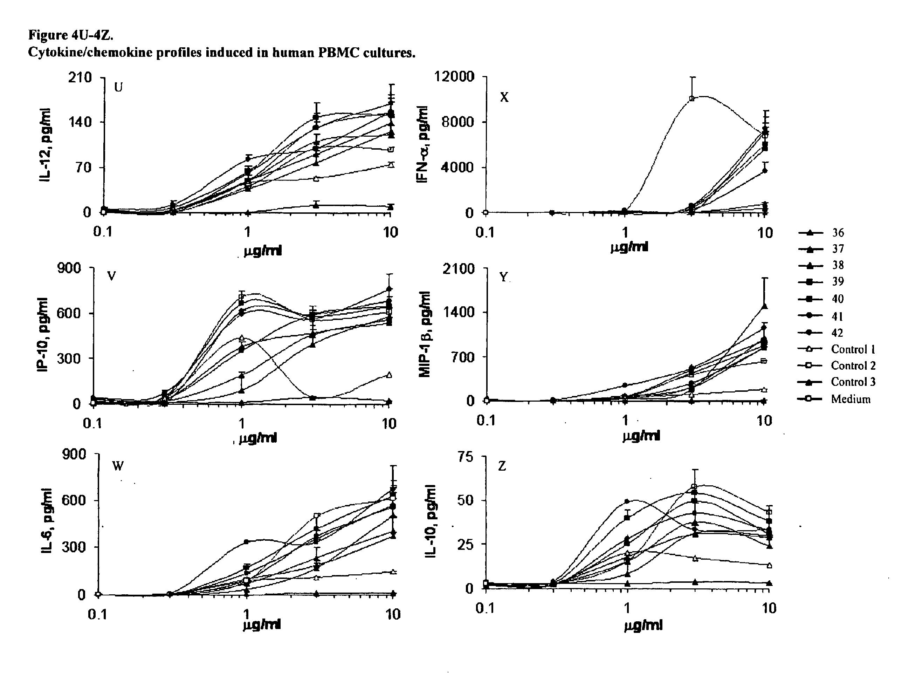

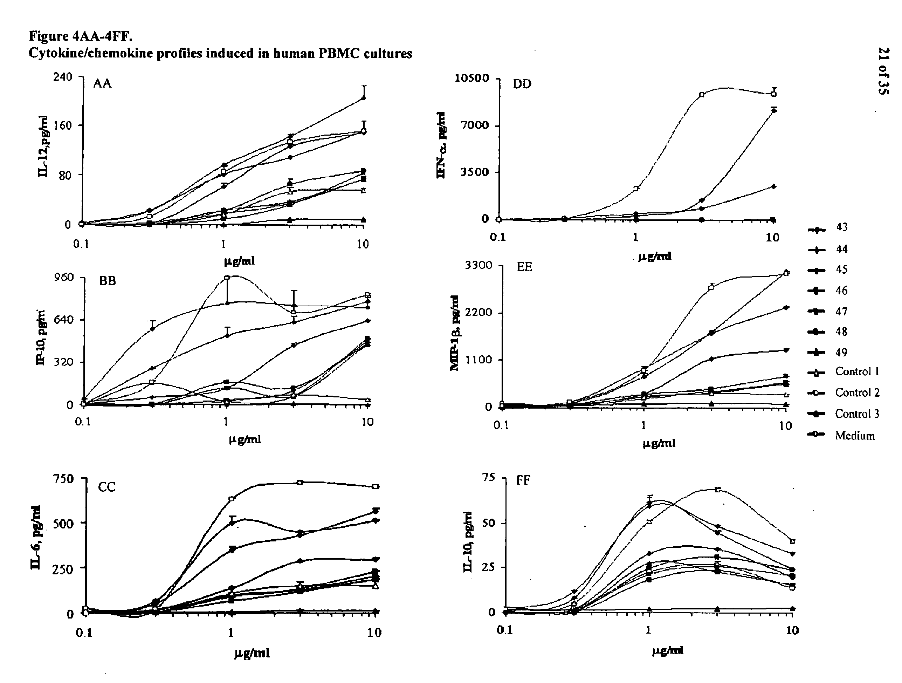

Figures 4A and 4B depict cytokine and chemokine concentrations from human PBMCs that were isolated, cultured, treated and analyzed according to Example 3 below. Briefly, the PBMCs were isolated from freshly obtained healthy human volunteer's blood and cultured with 10 µg/ml dose of immune modulatory oligonucleotides described herein for 24 hr. Supernatants were collected and analyzed by Luminex multiplex assay cytokine and chemokine levels. Figures 4A and 4B more generally demonstrate that administration of immune modulatory oligonucleotides containing novel bases, linkers, and/or unique modifications described herein generates distinct cytokine and chemokine profiles.
Figures 4C - 4H depict cytokine and chemokine concentrations from human PBMCs that were isolated, cultured, treated and analyzed according to Example 3 below. Briefly, the PBMCs were isolated from freshly obtained healthy human volunteer's blood and cultured with 0 (PBS), 0.1, 0.3, 1.0, 3.0, or 10.0 µg/ml dose of immune modulatory oligonucleotides described herein for 24 hours. Supernatants were collected and analyzed by Luminex multiplex assay for cytokine and chemokine levels. Figures 4C - 4H more generally demonstrate that administration of immune modulatory oligonucleotides containing novel bases, linkers, and/or unique modifications described herein generates distinct cytokine and chemokine profiles.

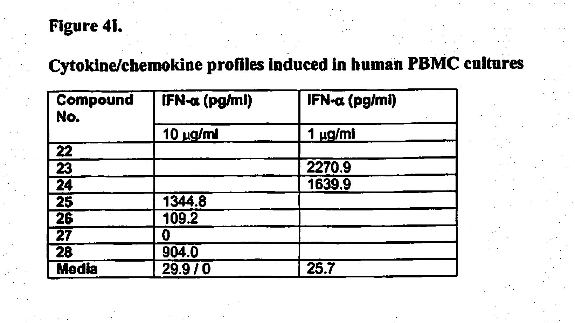

Figures 4I - 4N depict cytokine and chemokine concentrations from human PBMCs that were isolated, cultured, treated and analyzed according to Example 3 below. Briefly, the PBMCs were isolated from freshly obtained healthy human volunteer's blood and cultured with 0 (PBS), 1.0, or 10.0 µg/ml dose of immune modulatory oligonucleotides described herein for 24 hours. Supernatants were collected and analyzed by Luminex multiplex assay for cytokine and chemokine levels. Figures 4I - 4N more generally demonstrate that administration of immune modulatory oligonucleotides containing novel bases, linkers, and/or unique modifications described herein generates distinct cytokine and chemokine profiles.
Figures 4O - 4FF depict cytokine and chemokine concentrations from human PBMCs that were isolated, cultured, treated and analyzed according to Example 3 below. Briefly, the PBMCs were isolated from freshly obtained healthy human volunteer's blood and cultured with 0 (PBS), 0.1, 0.3, 1.0, 3.0, or 10.0 µg/ml dose of immune modulatory oligonucleotides described herein for 24 hours. Supernatants were collected and analyzed by Luminex multiplex assay for cytokine and chemokine levels. Figures 4O - 4FF more generally demonstrate that administration of immune modulatory oligonucleotides containing novel bases, linkers, and/or unique modifications described herein generates distinct cytokine and chemokine profiles.

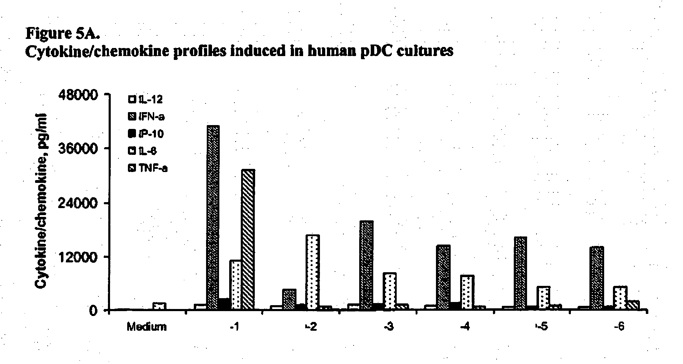

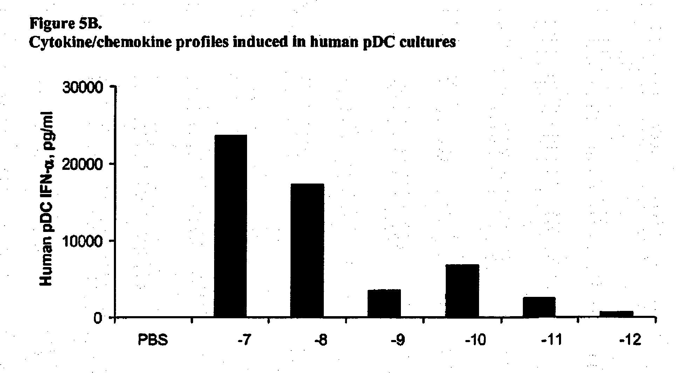

Figures 5A and 5B depict cytokine and chemokine concentrations from human plasmacytoid dendritic cells (pDCs) that were isolated, cultured, treated and analyzed according to Example 3 below. Briefly, the pDCs were isolated from freshly obtained healthy human volunteer's blood PBMCs and cultured with 10 µg/ml dose of immune modulatory oligonucleotides described herein for 24 hr. Supernatants were collected and analyzed by Luminex multiplex assay for cytokine and chemokine levels. Figures 5A and 5B more generally demonstrate that administration of immune modulatory oligonucleotides containing novel bases, linkers, and/or unique modifications described herein generates distinct cytokine and chemokine profiles.

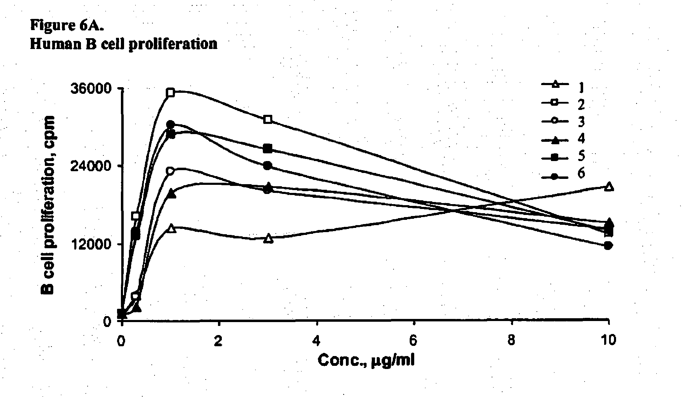

Figures 6A - 6F depict human B-cell proliferation induced by immune modulatory oligonucleotides described herein. The human B-cells were isolated, cultured, treated and analyzed according to Example 4 below. Briefly, the Human B cells isolated from freshly obtained healthy human volunteer's PBMCs were cultured with different doses of immune modulatory oligonucleotides described herein for 68 hours and pulsed with ³H-thymidine for 6-8 hours. ³H-Thymidine uptake was determined using a liquid scintillation counter. Figures 6A - 6F more generally demonstrate that administration of immune modulatory oligonucleotides containing novel bases, linkers, and/or unique modifications described herein generates distinct cell proliferation profiles, which vary with the base composition, unique modification, and the amount of the oligonucleotide administered.

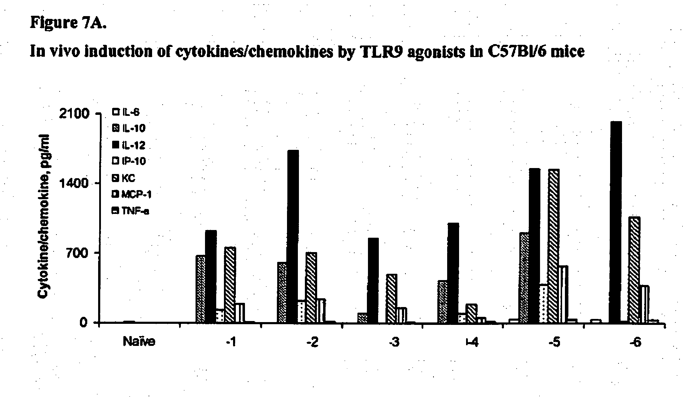

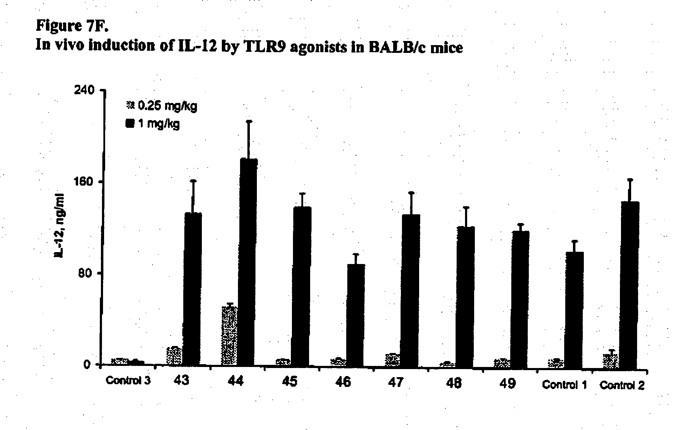

Figure 7A depicts serum cytokine and chemokine induction in C57BL/6 mice that were treated according to Example 5 below. Briefly, 2 hours after the mice were injected subcutaneously with 1 mg/kg dose of immune modulatory oligonucleotides described herein, serum was collected and analyzed by Luminex multiplex assay for cytokine and chemokine levels.

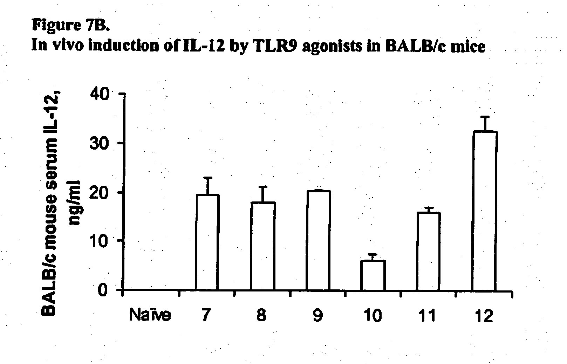

Figure 7B depicts serum cytokine induction in BALB/c mice that were treated according to Example 5 below. Briefly, 2 hours after the mice were injected subcutaneously with 1 mg/kg dose of immune modulatory oligonucleotides described herein, serum was collected and analyzed by ELISA for IL-12 levels.

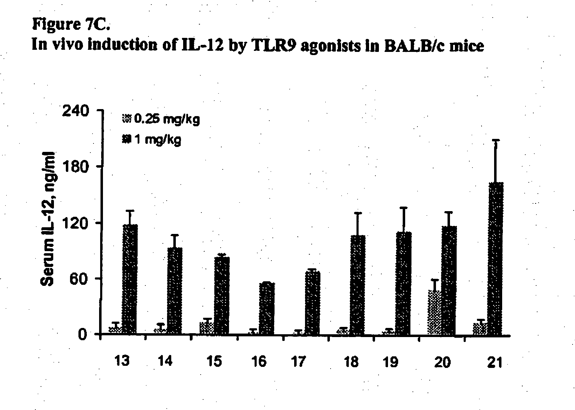

Figures 7C - 7F depict serum cytokine induction in BALB/c mice that were treated according to Example 5 below. Briefly, 2 hours after the mice were injected subcutaneously with 0.25 or 1 mg/kg dose of immune modulatory oligonucleotides described herein, serum was collected and analyzed by ELISA for IL-12 levels. Figures 7A - 7F more generally demonstrate that in vivo administration of immune modulatory oligonucleotides containing novel bases, linkers, and/or unique modifications described herein generates distinct TLR9 activation profiles, which will find application in a variety of diseases.

### DETAILED DESCRIPTION OF THE PREFERRED EMBODIMENTS

The invention describes oligonucleotide-based compounds that individually provide distinct immune response profiles through their interactions as agonists with TLR9. The TLR9 agonists described herein are characterized by unique chemical modifications, which provide their distinct immune response activation profiles. All publications cited herein reflect the level of skill in the art and are hereby incorporated by reference in their entirety. Any conflict between the teachings of these references and this specification shall be resolved in favor of the latter.

The TLR9 agonists described herein induce immune responses in various cell types and in various in vivo and in vitro experimental models, with each agonist providing a distinct immune response profile. As such, they are useful as tools to study the immune system, as well as to compare the immune systems of various animal species, such as humans and mice. The TLR9 agonists described herein are also useful in the prevention and/or treatment of various diseases, either alone, in combination with or co-administered with other drugs, or as adjuvants for antigens used as vaccines.

### DEFINITIONS

The term "2'-substituted nucleoside" or "2'-substituted arabinoside" generally includes nucleosides or arabinonucleosides in which the hydroxyl group at the 2' position of a pentose or arabinose moiety is substituted to produce a 2'-substituted or 2'-O-substituted ribonucleoside. In certain embodiments, such substitution is with a lower hydrocarbyl group containing 1-6 saturated or unsaturated carbon atoms, with a halogen atom, or with an aryl group having 6-10 carbon atoms, wherein such hydrocarbyl, or aryl group may be unsubstituted or may be substituted, e.g., with halo, hydroxy, trifluoromethyl, cyano, nitro, acyl, acyloxy, alkoxy, carboxyl, carboalkoxy, or amino groups. Examples of 2'-O-substituted ribonucleosides or 2'-O-substituted-arabinosides include, without limitation 2'-amino, 2'-fluoro, 2'-allyl, 2'-O-alkyl and 2'-propargyl ribonucleosides or arabinosides, 2'-O-methylribonucleosides or 2'-O-methylarabinosides and 2'-O-methoxyethoxyribonucleosides or 2'-O-methoxyethoxyarabinosides.

The term " 3' ", when used directionally, generally refers to a region or position in a polynucleotide or oligonucleotide 3' (toward the 3' position of the oligonucleotide) from another region or position in the same polynucleotide or oligonucleotide.

The term " 5' ", when used directionally, generally refers to a region or position in a polynucleotide or oligonucleotide 5' (toward the 5' position of the oligonucleotide) from another region or position in the same polynucleotide or oligonucleotide.

The term "about" generally means that the exact number is not critical. Thus, the number of nucleoside residues in the oligonucleotides is not critical, and oligonucleotides having one or two fewer nucleoside residues, or from one to several additional nucleoside residues are contemplated as equivalents of each of the embodiments described above.

The term "airway inflammation" generally includes, without limitation, inflammation in the respiratory tract caused by allergens, including asthma.

The term "allergen" generally refers to an antigen or antigenic portion of a molecule, usually a protein, which elicits an allergic response upon exposure to a subject. Typically the subject is allergic to the allergen as indicated, for instance, by the wheal and flare test or any method known in the art. A molecule is said to be an allergen even if only a small subset of subjects exhibit an allergic (e.g., IgE) immune response upon exposure to the molecule.

The term "allergy" generally includes, without limitation, food allergies, respiratory allergies and skin allergies.

The term "antigen" generally refers to a substance that is recognized and selectively bound by an antibody or by a T cell antigen receptor. Antigens may include but are not limited to peptides, proteins, nucleosides, nucleotides and combinations thereof. Antigens may be natural or synthetic and generally induce an immune response that is specific for that antigen.

The term "autoimmune disorder" generally refers to disorders in which "self" antigen undergo attack by the immune system. Such term includes, without limitation, lupus erythematosus, multiple sclerosis, type I diabetes mellitus, irritable bowel syndrome, Chron's disease, rheumatoid arthritis, septic shock, alopecia universalis, acute disseminated encephalomyelitis, Addison's disease, ankylosing spondylitis, antiphospholipid antibody syndrome, autoimmune hemolytic anemia, autoimmune hepatitis, Bullous pemphigoid, chagas disease, chronic obstructive pulmonary disease, coeliac disease, dermatomyositis, endometriosis, Goodpasture's syndrome, Graves' disease, Guillain-Barré syndrome, Hashimoto's disease, hidradenitis suppurativa, idiopathic thrombocytopenic purpura, interstitial cystitis, morphea, myasthenia gravis, narcolepsy, neuromyotonia, pemphigus, pernicious anaemia, polymyositis, primary biliary cirrhosis, schizophrenia, Sjögren's syndrome, temporal arteritis ("giant cell arteritis"), vasculitis, vitiligo, vulvodynia and Wegener's granulomatosis autoimmune asthma, septic shock, psoriasis and malaria.

The term "cancer" generally refers to, without limitation, any malignant growth or tumor caused by abnormal or uncontrolled cell proliferation and/or division. Cancers may occur in humans and/or animals and may arise in any and all tissues. Treating a patient having cancer with the invention may include administration of a compound, pharmaceutical formulation or vaccine according to the invention such that the abnormal or uncontrolled cell proliferation and/or division is affected.

The term "carrier" generally encompasses any excipient, diluent, filler, salt, buffer, stabilizer, solubilizer, oil, lipid, lipid containing vesicle, microspheres, liposomal encapsulation, or other material well known in the art for use in pharmaceutical formulations. It will be understood that the characteristics of the carrier, excipient, or diluent will depend on the route of administration for a particular application. The preparation of pharmaceutically acceptable formulations containing these materials is described in, *e.g*., Remington's Pharmaceutical Sciences, 18th Edition, ed. A. Gennaro, Mack Publishing Co., Easton, PA, 1990.

The term "pharmaceutically acceptable" or "physiologically acceptable" generally refers to a material that does not interfere with the effectiveness of a compound according to the invention, and that is compatible with a biological system such as a cell, cell culture, tissue, or organism. Preferably, the biological system is a living organism, such as a vertebrate.

The term "co-administration" or "co-administered" generally refers to the administration of at least two different substances sufficiently close in time to modulate an immune response. Preferably, co-administration refers to simultaneous administration of at least two different substances.

The term a "pharmaceutically effective amount" generally refers to an amount sufficient to affect a desired biological effect, such as a beneficial result. Thus, a "pharmaceutically effective amount" will depend upon the context in which it is being administered. A pharmaceutically effective amount may be administered in one or more prophylactic or therapeutic administrations.

The term "in combination with" generally means administering a compound described herein and another agent useful for treating the disease or condition that does not abolish TLR9 antagonist effect of the compound in the course of treating a patient. Such administration may be done in any order, including simultaneous administration, as well as temporally spaced order from a few seconds up to several days apart. Such combination treatment may also include more than a single administration of the compound according to the invention and/or independently the other agent. The administration of the compound described herein and the other agent may be by the same or different routes.

The term "individual" or "subject" generally refers to a mammal, such as a human. Mammals generally include, but are not limited to, humans, non-human primates, rats, mice, cats, dogs, horses, cattle, cows, pigs, sheep and rabbits.

The term "kinase inhibitor" generally refers to molecules that antagonize or inhibit phosphorylation-dependent cell signaling and/or growth pathways in a cell. Kinase inhibitors may be naturally occurring or synthetic and include small molecules that have the potential to be administered as oral therapeutics. Kinase inhibitors have the ability to rapidly and specifically inhibit the activation of the target kinase molecules. Protein kinases are attractive drug targets, in part because they regulate a wide variety of signaling and growth pathways and include many different proteins. As such, they have great potential in the treatment of diseases involving kinase signaling, including cancer, cardiovascular disease, inflammatory disorders, diabetes, macular degeneration and neurological disorders. Examples of kinase inhibitors include sorafenib (Nexavar®), Sutent®, dasatinib, Dasatinib™, Zactima™, Tykerb™ and STI571.

The term "linear synthesis" generally refers to a synthesis that starts at one end of an oligonucleotide and progresses linearly to the other end. Linear synthesis permits incorporation of either identical or non-identical (in terms of length, base composition and/or chemical modifications incorporated) monomeric units into an oligonucleotide.

The term "mammal" is expressly intended to include warm blooded, vertebrate animals, including, without limitation, humans.

The term "modified nucleoside" generally is a nucleoside that includes a modified heterocyclic base, a modified sugar moiety, or any combination thereof. In some embodiments, the modified nucleoside is a non-natural pyrimidine or purine nucleoside, as herein described. For purposes of the invention, a modified nucleoside, a pyrimidine or purine analog or non-naturally occurring pyrimidine or purine can be used interchangeably and refers to a nucleoside that includes a non-naturally occurring base and/or non-naturally occurring sugar moiety. For purposes of the invention, a base is considered to be non-natural if it is not guanine, cytosine, adenine, thymine or uracil.

The term "modulation" or "modulatory" generally refers to change, such as an increase in a response or qualitative difference in a TLR9-mediated response.

The term "linker" generally refers to any moiety that can be attached to an oligonucleotide by way of covalent or non-covalent bonding through a sugar, a base, or the backbone. The linker can be used to attach two or more nucleosides or can be attached to the 5' and/or 3' terminal nucleotide in the oligonucleotide. In certain embodiments of the invention, such linker may be a non-nucleotidic linker.

The term "non-nucleotidic linker" generally refers to a chemical moiety other than a nucleotidic linkage that can be attached to an oligonucleotide by way of covalent or non-covalent bonding. Preferably such non-nucleotidic linker is from about 2 angstroms to about 200 angstroms in length, and may be either in a cis or trans orientation.

The term "nucleotidic linkage" generally refers to a chemical linkage to join two nucleosides through their sugars (e.g. 3'-3', 2'-3', 2'-5', 3'-5') consisting of a phosphorous atom and a charged, or neutral group (e.g., phosphodiester, phosphorothioate or phosphorodithioate) between adjacent nucleosides.

The term "oligonucleotide-based compound" refers to a polynucleoside formed from a plurality of linked nucleoside units. The nucleoside units may be part of or may be made part of viruses, bacteria, cell debris, siRNA or microRNA. Such oligonucleotides can also be obtained from existing nucleic acid sources, including genomic or cDNA, but are preferably produced by synthetic methods. In preferred embodiments each nucleoside unit includes a heterocyclic base and a pentofuranosyl, trehalose, arabinose, 2'-deoxy-2'-substituted nucleoside, 2'-deoxy-2'-substituted arabinose, 2'-O-substitutedarabinose or hexose sugar group. The nucleoside residues can be coupled to each other by any of the numerous known internucleoside linkages. Such internucleoside linkages include, without limitation, phosphodiester, phosphorothioate, phosphorodithioate, alkylphosphonate, alkylphosphonothioate, phosphotriester, phosphoramidate, siloxane, carbonate, carboalkoxy, acetamidate, carbamate, morpholino, borano, thioether, bridged phosphoramidate, bridged methylene phosphonate, bridged phosphorothioate, and sulfone internucleoside linkages. The term "oligonucleotide-based compound" also encompasses polynucleosides having one or more stereospecific internucleoside linkage (e.g., (*R_{P}*)*-* or (*S_{P}*)-phosphorothioate, alkylphosphonate, or phosphotriester linkages). As used herein, the terms "oligonucleotide" and "dinucleotide" are expressly intended to include polynucleosides and dinucleosides having any such internucleoside linkage, whether or not the linkage comprises a phosphate group. In certain preferred embodiments, these internucleoside linkages may be phosphodiester, phosphorothioate or phosphorodithioate linkages, or combinations thereof.

The term "peptide" generally refers to polypeptides that are of sufficient length and composition to affect a biological response, e.g., antibody production or cytokine activity whether or not the peptide is a hapten. The term "peptide" may include modified amino acids (whether or not naturally or non-naturally occurring), where such modifications include, but are not limited to, phosphorylation, glycosylation, pegylation, lipidization and methylation.

The term "TLR9 agonist" generally refers to an oligonucleotide-based compound that is able to enhance, induce or modulate an immune stimulation mediated by TLR9.

The term "treatment" generally refers to an approach intended to obtain a beneficial or desired result, which may include alleviation of symptoms, or delaying or ameliorating a disease progression.

TLR9 agonists are shown in Table I below. TLR9 agonists which are not encompassed by the subject-matter of the claims are described for comparative purposes only. In this table, the oligonucleotide-based TLR9 agonists have all phosphorothioate (PS) linkages, except where indicated. Those skilled in the art will recognize, however, that phosphodiester (PO) linkages, or a mixture of PS and PO linkages can be used. Except where indicated, all nucleotides are deoxyribonucleotides.

**Table I**

| Seq. ID. No./Compound No. | Sequence and Modifications |
|---|---|
| **1** (1) | 5'- TCG₁TACG₁TACG₁-X-G₁CATG₁CATG₁CT -5' |
| **2** (2) | 5'- TCTGT_{O}CG₂TTGT-X-TGTTG₂C_{O}TGTCT -5' |
| **3** (3) | 5'- TCAGT_{O}CG₂TTAC-Z-CATTG₂C_{O}TGACT -5' |
| **4** (4 & 170) | 5'- TCTG_{O}T_{O}CG₂TAG-M-GATTG₂C_{O}T_{O}GTCT -5' |
| **5** (5) | 5'- TCG₁TCG₁TTT-L-M-L-TTTG₁CTG₁CT -5' |
| **6** (6) | 5'- TCG₁TCG₁TTT-L-X-HTTG₁CTG₁CT -5' |
| **7** (7) | 5'- TCG₁AACG₁TTCoG-Z-G_{O}CTTG₁CAAG₁CT -5' |
| **8** (8 & 18) | 5'- TCG₁TCG₁TTL-Y-G₁CTTG₁CAAG₁CT -5' |
| **9** (9) | 5'- TCTGTCG₂TTCU-X-UCTTG₂CTGTCT -5' |
| **10** (10) | 5'- TCG₁TCG₁TTTUU-X-UUTTTG₁CTG₁CT -5' |
| **11** (11) | TCG₁TCG₁TTU₁Y-Z-YU₁TTG₁CTG₁CT -5' |
| **12** (12) | 5'- TCG₂TCG₂TTU₁Y-M-YU₁TTG₂CTG₂CT -5' |
| **13** (13) | 5'-TAGTCG₁TTCTC-X-CTCTTG₁CTGAT-5' |
| **14** (14) | 5'-TCUTGTCG₁TTC-X-CTTG₁CTGTUCT-5' |
| **15** (15 & 171) | 5'-TCG₁TCG₁TTTTT-Y-TCTTG₁CTGUCT-5' |
| **16** (16 & 172) | 5'-TCG₁TCG₁TTTTT-Y-TCTTG₁CTGTCTTG₁CT-5' |
| **17** (17 & 192) | 5'-TCTGTCG₁TTCT-Y-TCTTG₁CTGYYTTG₁CT-5' |
| **18** (18 & 172) | 5'-TCG₁AACG₁TTCG₁-Y-TCTTG₁CTGTCTTG₁CT-5' |
| **19** (19 & 193) | 5'-TCG₁AACG₁TTCG₁-Y-TCTTG₁CTGLLTTG₁CT-5' |
| **20** (20 & 171) | 5'-TCG₁AACG₁TTCG₁-Y-TCTTG₁CTGUCT-5' |
| **21** (21 & 173) | 5'-TCG₁AACG₁TTCG₁-Y-GACAG₁CTGTCT-5' |
| **22** (22) | 5'-TCTGTCG₁TTCT-m-TCTTG₁CTGTCT-5' |
| **23** (23) | 5'-CAGTC_{O}G₂TTCAG-M-GACTTG_{2O}CTGAC-5' |
| **24** (24 & 18) | 5'-CAGTC_{O}G₂TTCAG-Y-G₁CTTG₁CAAG₁CT-5' |
| **25** (25) | 5'-TCG₁AACG₁TTCG-Z-GCTTG₁CAAG₁CT-5' |
| **26** (26 & 174) | 5'-TCG₁TCG₁TTTTT-Y-TCTTG₁CTGUCT-5' |
| **27** (27) | 5'-TCG₂TC_{O}G₂TTU₁Y-X-YU₁TTG_{2O}CTG₂CT-5' |
| **28** (28) | 5-TCG₁AACG₁U₁U₁CG-X-GcU₁U₁G₁CAAG₁CT-5' |
| **29** (29) | 5'-TCTGTCG₁TTCT-L₁-TCTTG₁CTGTCT-5' |
| **30** (30 & 18) | 5'-CAGTCG₂TTCAG-Y-G₁CTTG₁CAAG₁CT-5' |
| **31** (31) | 5'-CAGTCoG₂TTCAG-Z-GACTTG₂oCTGAC-5' |
| **32** (32 & 175) | 5'-TCTGTCG₁TTCT-Y-TCTTG₁CTGUCTTG₁CT-5' |
| **33** (33) | 5'-TCG₁AACG₁U₁U₁CoG-M-GoCU₁U₁G₁CAAG₁CT-5' |
| **34** (34) | 5'-TCG₁AACG₁ToTCoG-m-GoCToTG₁CAAG₁CT-5' |
| **35** (35) | 5'-TCG₁AACG₁TTCG₁-L₁-G₁CTTG₁CAAG₁CT-5' |
| **36** (36) | 5'-CAGTCG₂TTCAG-X₂-GACTTG₂CTGAC-5' |
| **37** (37) | 5'-CAGTCG₂TTCAG-X₂-GACTTG₂CTGAC-5' |
| **38** (38) | 5-psCAGTCG₂TTCAG-X-GACTTG₂CTGACps-5' |
| **39** (39 & 30) | 5'-TCG₁AACG₁TTCoG₁-Y₂-GACTTG₂CTGAC-5' |
| **40** (40 & 30) | 5'-TCG₁AACG₁TTCG-Y₂-GACTTG₂CTGAC-5' |
| **41** (41 & 30) | 5'-TCG₁AACG₁TTCoG-Y₂-GACTTG₂CTGAC-5' |
| **42** (42 & 176) | 5'-TCG₁AACG₁TTCoG-Y₂-CTTG₂CTGACUTG₁CT-5' |
| **43** (43 & 17) | 5'-CAGTCG₂TTCAG-Y₂-TCTTG₁CTGTCT-5' |
| **44** (44 & 177) | 5'-TCG₁AACG₁TTCoG-Y2-CTTG₂CTGApmCTTG₁CT-5' |
| **45** (45 & 178) | 5'-TCG₁AACG₁TTCG1-Y₃-TGTTG₁CTGTCTTG₁CT-5' |
| **46** (46) | 5'-TCAGTCG₁TTAC-X-CATTG₁CTGACT-5' |
| **47** (47) | 5'-TCTGTCG₁TTTT-X-TTTTG₁CTGTCT-5' |
| **48** (48) | 5'-TCAGTCG₁TTACY₁-X3-Y₁CATTG₁CTGACT-5' |
| **49** (49) | 5'-TCTGTCG₁TTTTY₁-X₃-Y₁TTTTG₁CTGTCT-5' |
| **50** (50 & 18) | 5'-TCG₁TCG₁TTY3-Y-G₁CTTG₁CAAG₁CT-5' |
| **51** (51) | 5'-TCG₁TCG₁TTdUY-Z-YdUTTG₁CTG₁CT-5' |
| **52** (52) | 5'-TCG₁TCG₁TTdUY-X-YdUTTG₁CTG₁CT-5' |
| **53** (53) | 5'-TCG₁TCG₁TTdUY-M-YdUTTG₁CTG₁CT-5' |
| **54** (54) | 5'-TCG₁TCG₁TTdUY-m-YdUTTG₁CTG₁CT-5' |
| **55** (55) | 5'-TCG₂TCG₂TTdUY-Z-YdUTTG₂CTG₂CT-5' |
| **56** (56) | 5'-TCG₂TCG₂TTdUY-X-YdUTTG₂CTG₂CT-5' |
| **57** (57) | 5'-TCG₂TCG₂TTdUY-M-YdUTTG₂CTG₂CT-5' |
| **58** (58) | 5'-TCG₂TCG₂TTdUY-m-YdUTTG₂CTG₂CT-5' |
| **59** (59) | 5'-TCG₁TCG₁ACG₁AT-Z-TAG₁CAG₁CTG₁CT-5' |
| **60** (60) | 5'-TCAGTCG₂TTAC-X-CATTG₂CTGACT-5' |
| **61** (61) | 5'-TCG₁ATCG₁ATCG₁-X-G₁CTAG₁CTAG₁CT-5' |
| **62** (62) | 5'-TCG₁AACG₁TTCG₁-Z-G₁CTTG₁CAAG₁CT-5' |
| **63** (63) | 5'-TCG₁AACG₁TTCG-Z-GCTTG₁CAAG₁CT-5' |
| **64** (64 & 173) | 5'-TCG₁AACG₁TTCG₁-Y-GACAG₁CTGTCT-5' |
| **65** (65 & 179) | 5'-TAGTCG₁TTTTT-X-TTTTTG₁CGTAT-5' |
| **66** (66) | 5'-TCGTCGTTCTT-X-TTCTTGCTGCT-5' |
| **67** (67) | 5'-TGGTCG₂TTCTT-X-TTCTTG₂CTGGT-5' |
| **68** (68) | 5'-TAGTCG₂TTCTC-X-CTCTTG₂CTGAT-5' |
| **69** (69 & 180) | 5'-TCG₁TCG₁TTTTT-Y-TCTTG₁CTGTCT-5' |
| **70** (70 & 174) | 5'-TCG₁TCG₁TTTTT-Y-TCTTG₁CTGUCT-5' |
| **71** (71 & 181) | 5'-TCG₁TCG₁TTTTT-Y-TCTTG₁CTGTCTTCCT-5' |
| **72** (72) | 5'-TCTTGTCG₁TTC-X-CTTG₁CTGTTCT-5' |
| **73** (73) | 5'-TCTTGTCG₁TTC-X-CTTG₁CTGTTCT-5' |
| **74** (74) | 5'-TCTGTCG₃TTCT-X-TCTTG₃CTGTCT-5' |
| **75** (75) | 5'-TCG₃AACG₃TTCG₃-X-G₃CTTG₃CAAG₃CT-5' |
| **76** (76 & 175) | 5'-TCG₁TCG₁TTTTT-X-TCTTG₁CTGUCTTG₁CT-5' |
| **77** (77 & 192) | 5'-TCG₁TCG₁TTTTT-X-TCTTG₁CTGYYTTG₁CT-5' |
| **78** (78 & 193) | 5'-TCG₁TCG₁TTTTT-X-TCTTG₁CTGLLTTG₁CT-5' |
| **79** (79 & 193) | 5'-TCTGTCG₁TTCT-Y-TCTTG₁CTGLLTTG₁CT-5' |
| **80** (80 & 171) | 5'-TCG₁AACG₁TTCG₁-L-TCTTG₁CTGUCT-5' |
| **81** (81) | 5'-CAGTCG₂TTCAG-X-GACTTG₂CTGAC-5' |
| **82** (82 & 182) | 5'-CAGTCG₂TTCAG-Z-GACTTG₂CTTAC-5' |
| **83** (83 & 182) | 5'-CAGTCG₂TTCAG-M-GACTTG₂CTTAC-5' |
| **84** (84 & 182) | 5'-CAGTCG₂TTCAG-m-GACTTG₂CTTAC-5' |
| **85** (85 & 183) | 5'-CAGTCoG₂TTCAG-X-GACTTG₂CoTTAC-5' |
| **86** (86 & 183) | 5'-CAGTCoG₂TTCAG-M-GACTTG₂CoTTAC-5' |
| **87** (87 & 183) | 5'-CAGTCoG₂TTCAG-m-GACTTG₂CoTTAC-5' |
| **88** (88 & 18) | 5'-CAGTCoG₂TTCAG-Y-G₁CTTG₁CAAG₁CT-5' |
| **89** (89) | 5'-TCG₁AACG₁ToTCoG₁-m-G₁oCToTG₁CAAG₁CT-5' |
| **90** (90) | 5'-TCG₁AACG₁oTTCG₁-Z-G₁CTToG₁CAAG₁CT-5' |
| **91** (91) | 5'-TCG₁AACG₁oToToCoG-Z-GoCoToToG₁CAAG₁CT-5' |
| **92** (92) | 5'-TCG₁AACoG₁TTCG₁-X-G₁CTTG₁oCAAG₁CT-5' |
| **93** (93) | 5'-TCG₁AACG₁dUdUCG₁-X-G₁CdUdUG₁CAAG₁CT-5' |
| **94** (94) | 5'-TCG₁AACG₁dUdUCoG-X-GoCdUdUG₁CAAG₁CT-5' |
| **95** (95) | 5'-TCG₁AACG₁dUdUCoG-Z-GoCdUdUG₁CAAG₁CT-5' |
| **96** (96) | 5'-TCG₁AACG₁dUdUCoG-m-GoCdUdUG₁CAAG₁CT-5' |
| **97** (97) | 5'-TCG₁AACG₁dUdUCG-X-GCdUdUG₁CAAG₁CT-5' |
| **98** (98) | 5'-TCG₁AACG₁TTCG₁-L₂-G₁CTTG₁CAAG₁CT-5' |
| **99** (99 & 184) | 5'-TCG₁TCG₁TTCT-L₃-TCTTG₁CTGG₁CT-5' |
| **100**(100&185) | 5'-TCTG₁TCG₁TTCG₁-L₃-M-L₃-G₁CTTG₁CTGTCT-5' |
| **101** (101) | 5'-TCG₁GTCG₁TTCG₁-L₃-m-L₃-G₁CTTG₁CTGG₁CT-5' |
| **102** (102) | 5'-TCTGTCG₁TTCT-L₂-TCTTG₁CTGTCT-5' |
| **103** (103) | 5'-TCTGTCG₁TTCT-L₃-TCTTG₁CTGTCT-5' |
| **104** (104) | 5'-TCTGTCG₁TTCT-L₃-M-L₃-TCTTG₁CTGTCT-5' |
| **105** (105) | 5'-TCTGTCG₁TTCT-L₃-m-L₃-TCTTG₁CTGTCT-5' |
| **106** (106) | 5'-CAGTCG₃TTCAG-X-GACTTG₃CTGAC-5' |
| **107** (107) | 5'-TCTGTCG₃TTCT-X-TCTTG₃CTGTCT-5' |
| **108** (108) | 5'-TCTGTCG₁TTCT-X₁-TCTTG₁CTGTCT-5' |
| **109** (109) | 5'-TCTGTCG₁TTCT-X₂-TCTTG₁CTGTCT-5' |
| **110** (110) | 5'-TCTGTCG₁TTCT-Z-TCTTG₁CTGTCT-5' |
| **111** (111) | 5'-TCTGTCG₁TTCT-M-TCTTG₁CTGTCT-5' |
| **112** (112) | 5'-TCTGTCG₁TTCT-m-TCTTG₁CTGTCT-5' |
| **113** (113) | 5'-TCG₁AACG₁TTCG₁-Z-G₁CTTG₁CAAG₁CT-5' |
| **114** (114) | 5'-TCG₁AACG₁TTCG₁-M-G₁CTTG₁CAAG₁CT-5' |
| **115** (115) | 5'-TCG₁AACG₁TTCG₁-m-G₁CTTG₁CAAG₁CT-5' |
| **116** (116) | 5'-TCG₁AACG₁TTCG₁-X₂-G₁CTTG₁CAAG₁CT-5' |
| **117** (117) | 5'-TCG₁AACG₁TTCG₁-X₁-G₁CTTG₁CAAG₁CT-5' |
| **118** (118) | 5'-Y₁CAGTCG₂TTCAG-X-GACTTG₂CTGACY₁-5' |
| **119** (119) | 5'-YCAGTCC₂TTCAG-X-GACTTC₂CTGACY-5' |
| **120** (120) | 5'-poCAGTCG₂TTCAG-X-GACTTG₂CTGACpo-5' |
| **121** (121&22) | 5'-CAGTCG₂TTCAG-Y₂-TCTTG₁CTGTCT-5' |
| **122**(122&186) | 5'-TCG₁AACG₁TTCoG-Y₂-CTTG₂CTGACTTG₁CT-5' |
| **123** (123) | 5'-TCAGTCG₁TTAC-X-CATTG₁CTGACT-5' |
| **124** (124) | 5'-TCTGTCG₁TTAG-X-GATTG₁CTGTCT-5' |
| **125** (125) | 5'-TCTGTCG₁TTTT-X- TTTTG₁CTGTCT-5' |
| **126** (126) | 5'-TCTGTCG₁TTGT-X-TGTTG₁CTGTCT-5' |
| **127**(127&186) | 5'-TCG₁AACG₁TTCoG-Y₂-CTTG₂CTGACTTG₁CT-5' |
| **128** (128) | 5'-TCG₁AACG₁oTTCG₁-Z-G₁CTToG₁CAAG₁CT-5' |
| **129** (129) | 5'-TCG₁AACG₁TTCG₁-X₂-G₁CTTG₁CAAG₁CT-5' |
| **130**(130&177) | 5'-TCG₁AACG₁TTCoG-Y₂-CTTG₂CTGApmCTTG₁CT-5' |
| **131**(131&187) | 5'-TCG₁AACG₁TTCoG-Y₂-CTTG₂CTGApmCpmTTG₁CT-5' |
| **132**(132&180) | 5'-TCG₁TCG₁TTTTT-Y₃-TCTTG₁CTGTCT-5' |
| **133**(133&181) | 5'-TCG₁TCG₁TTTTT-Y₃-TCTTG₁CTGTCTTCCT-5' |
| **134** (134) | 5'-TCG₂TCG₂TTdUY₃-X₃-Y₃dUTTG₂CTG₂CT-5' |
| **135** (135) | 5'-TCG₁AACG₁oTTCG₁-X₃-G₁CTToG₁CAAG₁CT-5' |
| **136**(136&194) | 5'-TCTGTCG₁TTAC-Y₃-CATTG₁CTGYYTTG₁CT-5' |
| **137**(137&195) | 5'-TCTGTCG₁TTGT-Y₃-TGTTG₁CTGYYTTG₁CT-5' |
| **138**(139&194) | 5'-TCAGTCG₁TTCT-Y₃-CATTG₁CTGYYTTG₁CT-5' |
| **139**(139&188) | 5'-TCG₁AACG₁TTCG₁-Y₃-CATTG₁CTGTCTTG₁CT |
| **140**(140&178) | 5'-TCG₁AACG₁TTCG₁-Y₃-TGTTG₁CTGTCTTG₁CT |
| **141**(141&189) | 5'-TCTGTCG₁TTAC-Y₃-TGTTG₁CTGUCTTG₁CT-5' |
| **142**(142&190) | 5'-TCTGTCG₁TTGT-Y₃₋AGTTG₁CTGUCTTG₁CT-5' |
| **143**(143&191) | 5'-TCAGTCG₁TTAG-Y₃-CATTG₁CTGUCTTG₁CT-5' |
| **144** (144) | 5'-TCAGTCG₁TTAC-X-CATTG₁CTGACT-5' |
| **145** (145) | 5'-TCTGTCG₁TTAG-X-GATTG₁CTGTCT-5' |
| **146** (146) | 5'-TCTGTCG₁TTTT-X-TTTTG₁CTGTCT-5' |
| **147** (147) | 5'-TCTGTCG₁TTGT-X-TGTTG₁CTGTCT-5' |
| **148** (147) | 5'-Y₁CAGTCG₂TTCAG-X-GACTTG₂CTGACY₁-5' |
| **149** (149) | 5'-TCAGTCG₁TTACY₁-X₃-Y₁CATTG₁CTGACT-5' |
| **154** (150) | 5'-TCTGTCG₁TTAGY₁-X₃-Y₁GATTG₁CTGTCT-5' |
| **151** (151) | 5'-TCTGTCG₁TTTTY₁-X₃-Y₁TTTTG₁CTGTCT-5' |
| **152** (152) | 5'-TCTGTCG₁TTGTY₁-X₃-Y₁TGTTG₁CTGTCT-5' |
| **153** (153) | 5'-TCG₁AACG₁oTTCG₁-Z-G₁CTToG₁CAAG₁CT-5' |
| **154** (154) | 5'-TCG₁AACG₁TTCG₁-X₂-G₁CTTG₁CAAG₁CT-5' |
| **155** (155) | 5'-TCG₁TACG₁TACG₁-X₁-G₁CATG₁CATG₁CT-5' |
| **156** (156) | 5'-TCG₁TACG₁TACG₁-X₃-G₁CATG₁CATG₁CT-5' |
| **157** (157) | 5'-TCG₁TACG₁TACG₁-X-G₁CATG₁CATG₁CT-5' |
| **158** (158&29) | 5'-CAGTCG₂TTCAG-Y₂-TCTTG₁CTGTCT-5' |
| **159**(159&179) | 5'-TAGTCG₁TTTTT-X-TTTTTG₁CGTAT-5' |
| **160** (160) | 5'-TCTGTCG₁TTCT-Z-TCTTG₁CTGTCT-5' |
| **161** (161) | 5'-TCTGTCG₁TTCT-M-TCTTG₁CTGTCT-5' |
| **162** (162) | 5'-TCTGTCG₁TTCT-L₂-TCTTG₁CTGTCT-5' |
| **163** (163) | 5'-TCG₁AACG₁oTTCG₁-Z-G₁CTToG₁CAAG₁CT-5' |
| **164** (164) | 5'-TCG₁AACG₁TTCG₁-X₂-G₁CTTG₁CAAG₁CT-5' |
| **165** (165) | 5'-TCG₁AACG₁TTCG₁-m-G₁CTTG₁CAAG₁CT-5' |
| **166** (166) | 5'-TCG₁AACG₁ToTCoG-m-GoCToTG₁CAAG₁CT-5' |
| **167** (167) | 5'-TCG₁AACG₁dUdUCoG-M-GCodUdUG₁CAAG₁CT-5' |
| **168** (168) | 5'-TCG₁TCG₁ACG₁AT-X-TAG₁CAG₁CTG₁CT-5' |
| **169** (169) | 5'-TCG₁ATCG₁ATCG₁-X-G₁CTAG₁CTAG₁CT-5' |

G₁ = 7-deaza-dG; G₂ = AraG; G₃ = 7-deaza-araG; A/G/C/U = 2'-O-methylribonucleotides; dU = U₁ = 2'-deoxy-U; o = phosphodiester linkage; po = 5' -mono-phosphate; ps = 5'phosphorothiate linkage; pm = methyl phosphonate (non-ionic linkage); L = 1,5-pentanediol linker; L = 1,2-dideoxyribose; L₁ = triethylene glycol linker; L₂ = tetraethylene glycol linker; L₃ = hexaethylene glycol linker M = cis,cis-1,3,5-cyclohexanetriol linker; m = cis,trans-1,3,5-cyclohexanetriol linker; X = glycerol linker; X₁ = 1,2,4-butanetriol linker; X₂ = 1,3,5-tris(2-hydroxyethyl)cyanuric acid linker; X₃ = isobutanetriol linker; Y = 1,3-propanediol linker; Y₁ = 1,2-ethylenediol linker; Y₂ = 1,4-butanediol linker; Y₃ = 1,5-pentandiol linker; Z = 1,3,5-pentanetriol linker.

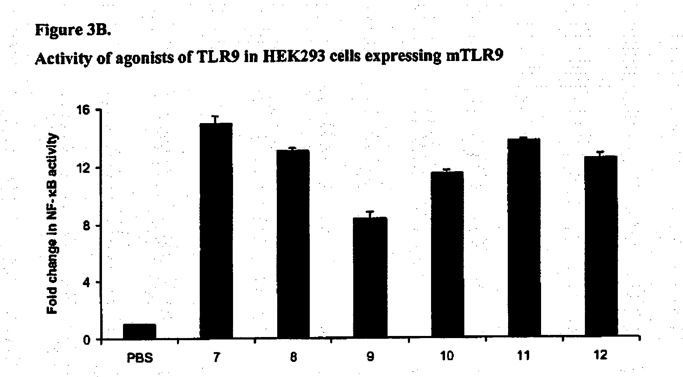

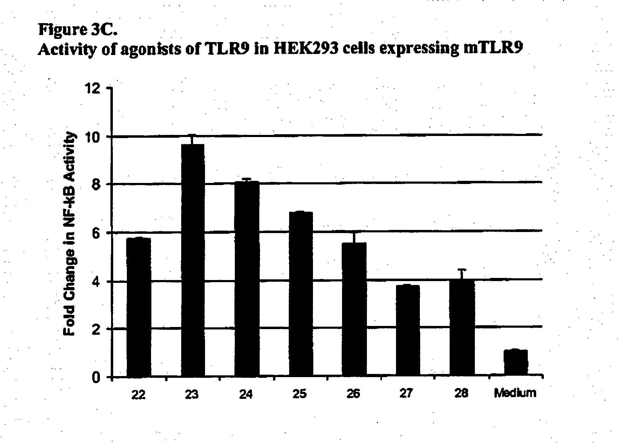

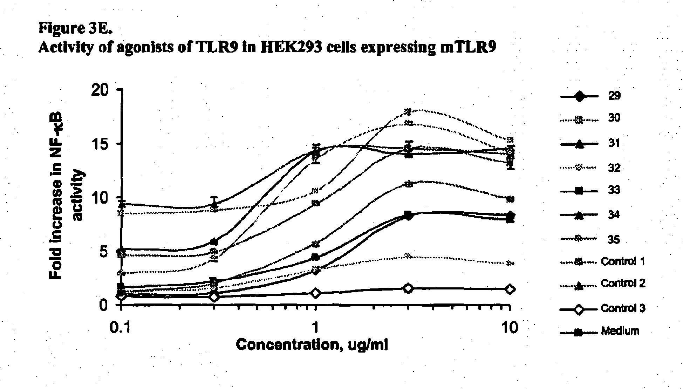

Exemplar TLR9 agonists from Table I were tested for immune stimulatory activity in HEK293 cells expressing TLR9, as described in Example 2. The results shown in Figure 3A, 3B, 3C, 3D, and 3E demonstrate that specific chemical modifications to 3'-3' linked oligonucleotides will alter their TLR9 mediated NF-kB activation profile 18 hours after administration. More generally, these data demonstrate that specific chemical modifications to 3'-3' linked oligonucleotides can be used to increase or decrease NF-kB activation.

Exemplar TLR9 agonists from Table I were tested for immune stimulatory activity in the human PBMC assay for IL-12, IL-10, IL-8, IL-6, IFN-α, IP-10, MIP-1α, MIP-1β, IL-1Rα, IL-2R, and MCP-1, as described in Example 3. The results shown in Figures 4A through 4FF demonstrate that specific chemical modifications to 3'-3' linked oligonucleotides will alter their TLR9 mediated IL-12, IL-10, IL-8, IL-6, IFN-α, IP-10, MIP-1α, MIP-1β, IL-IRα, IL-2R, and/or MCP-1 activation profile in human PBMCs. More generally, these data demonstrate that specific chemical modifications to 3'-3' linked oligonucleotides can be used to increase or decrease IL-12, IL-10, IL-8, IL-6, IFN-α, IP-10, MIP-1α, MIP-1β, IL-1Rα, IL-2R, and MCP-1 activation.

Exemplar TLR9 agonists from Table I were tested for immune stimulatory activity in the human pDC assays for IL-12, IL-6, IFN-α, IP-10, MIP-1α, MIP-1β, and TNFα, as described in Example 3. The results shown in Figures 5A and 5B demonstrate that specific chemical modifications to 3'-3' linked oligonucleotides will alter their TLR9 mediated immune activation profile in human pDCs. More generally, these data demonstrate that specific chemical modifications to 3'-3' linked oligonucleotides can be used to increase or decrease IL-12, IL-6, IFN-α, IP-10, MIP-1α, MIP-1β, and TNFα activation.

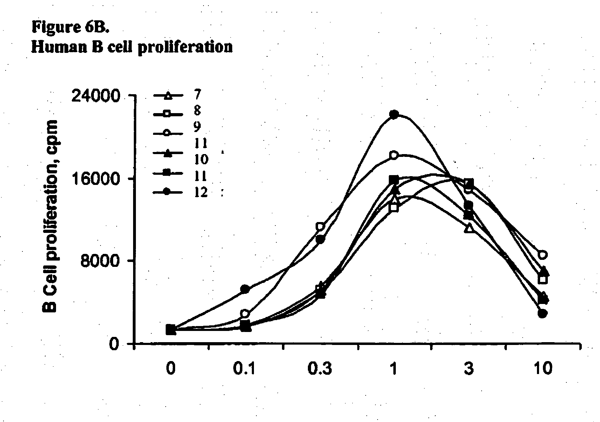

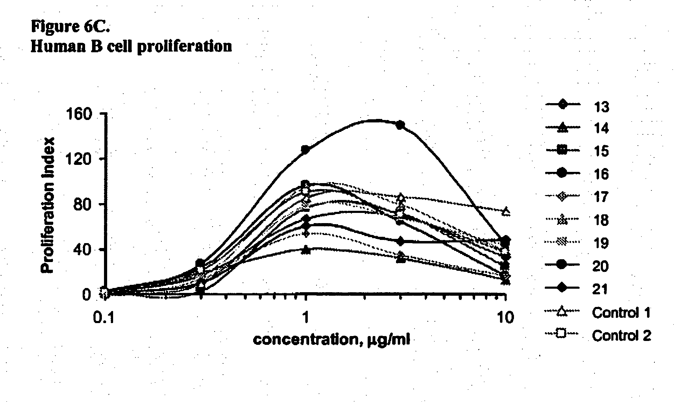

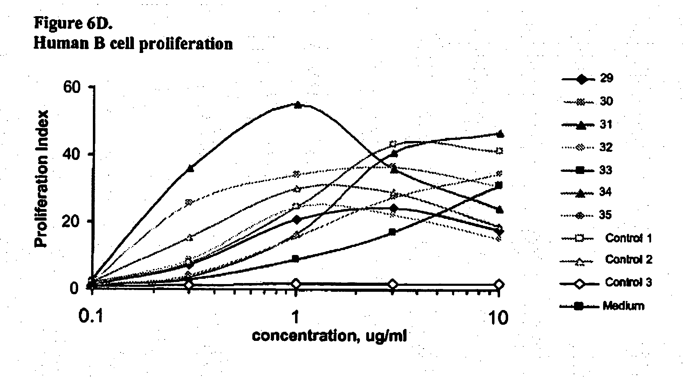

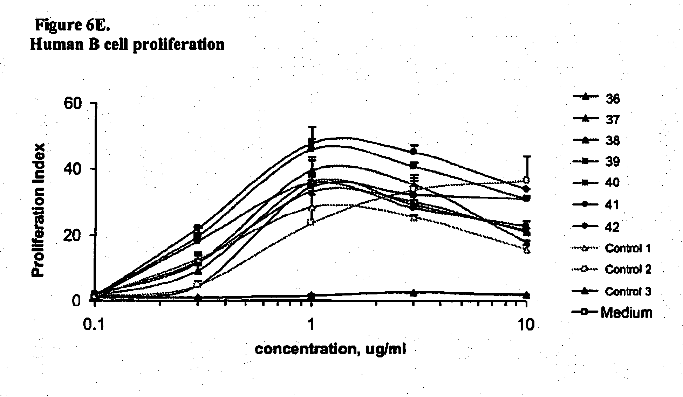

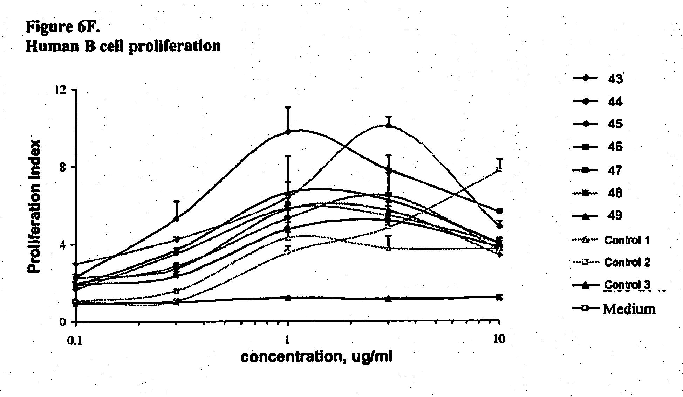

Exemplar TLR9 agonists from Table I were tested for immune stimulatory activity in the human B-cell proliferation assay, as described in Example 4. The results shown in Figures 6A, 6B, 6C, 6D, 6E and 6F demonstrate that specific chemical modifications to 3'-3' linked oligonucleotides will alter their TLR9 mediated B-cell proliferation activity and that this activation profile may be dose dependent depending on the chemical modification. More generally, these data demonstrate that specific chemical modifications to 3'-3' linked oligonucleotides can be used to regulate B-cell proliferation.

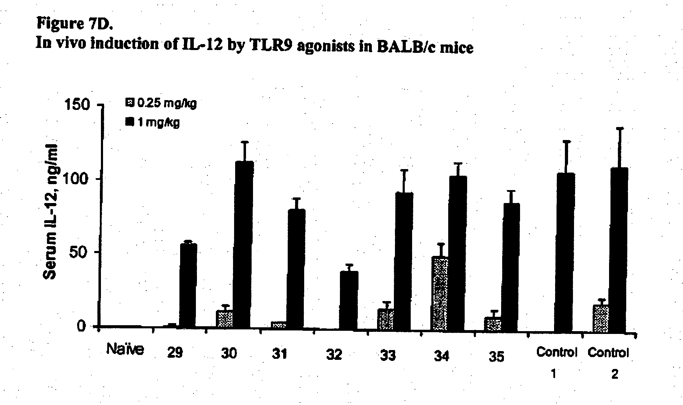

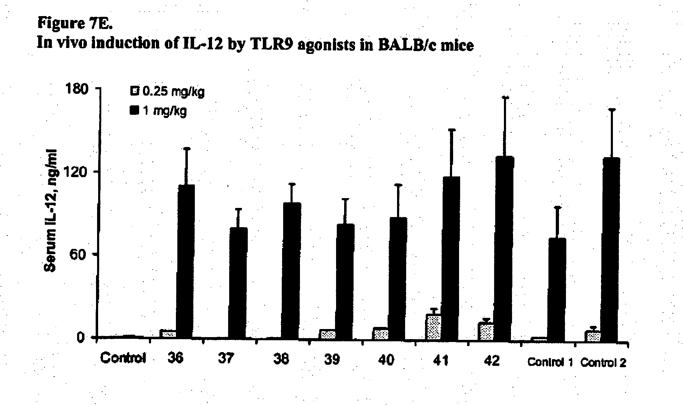

Exemplar TLR9 agonists from Table I were tested for *in vivo* immune stimulatory activity in C57Bl/6 and BALB/c mice, as described in Example 5. The results shown in Figures 7A, 7B, 7D, 7E and 8F demonstrate that specific chemical modifications to 3'-3' linked oligonucleotides will alter their in vivo TLR9 medicated immune activation profile in mouse models. More generally, these data demonstrate that specific chemical modifications to 3'-3' linked oligonucleotides can alter *in vivo* cytokine and/or chemokine concentrations, which will find application in many diseases.

The invention describes oligonucleotide-based synthetic agonists of TLR9. Based upon certain chemical modifications to the base, sugar, linkage or linker, the agonists of TLR9 may possess increased stability when associated and/or duplexed with other of the TLR9 agonist molecules, while retaining an accessible 5'-end.

Non-nucleotidic linkers include, but are not limited to, those listed in Table II.

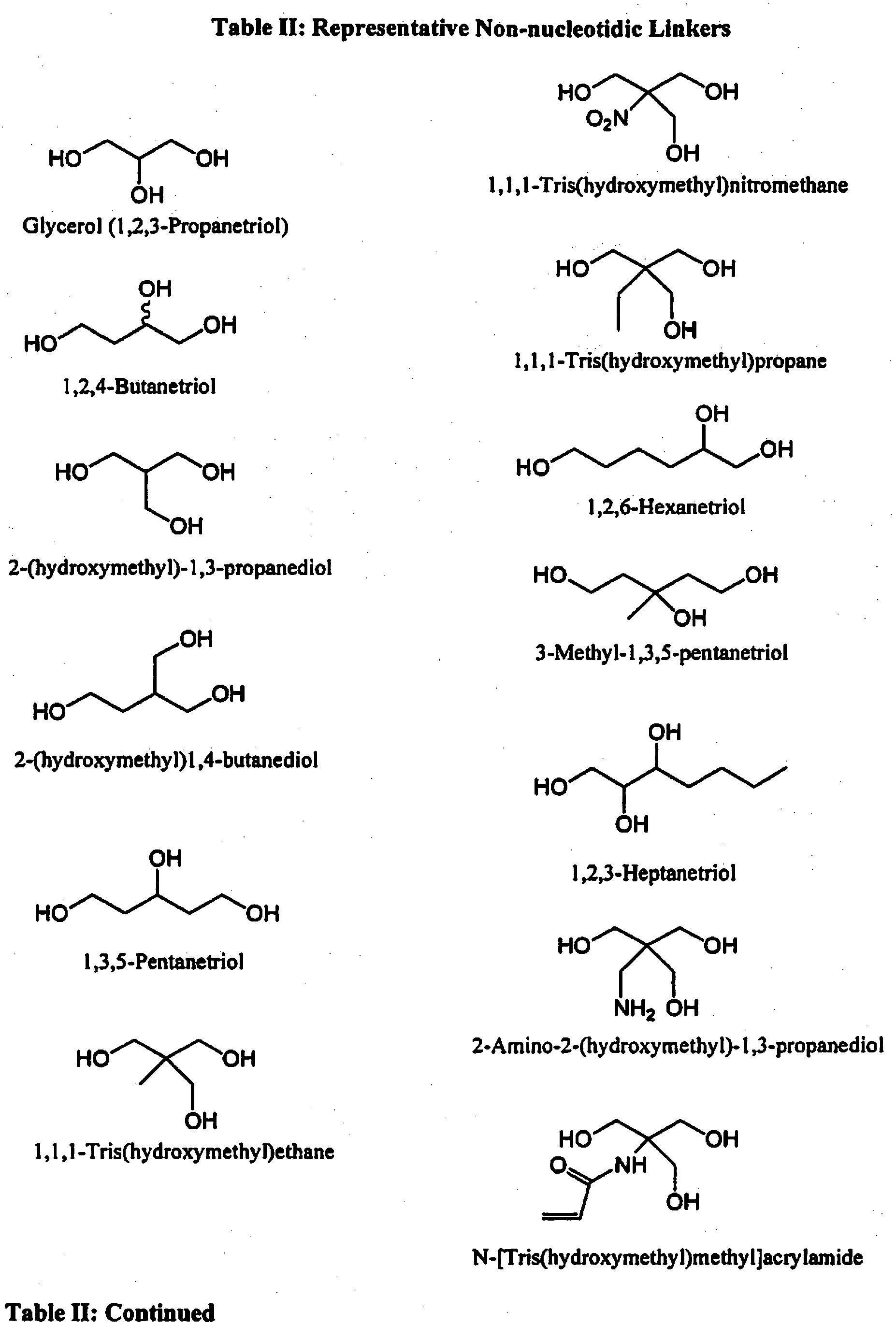

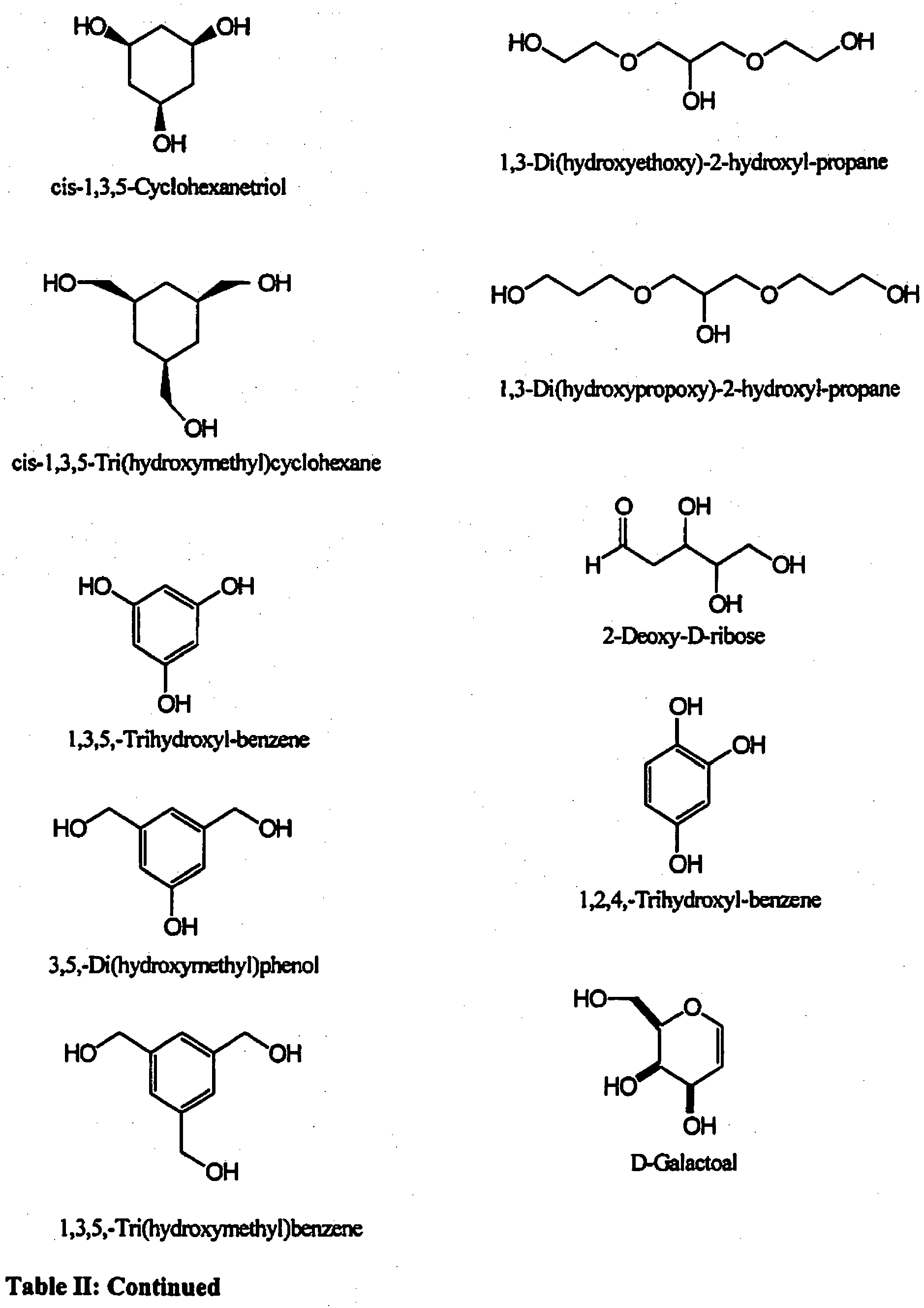

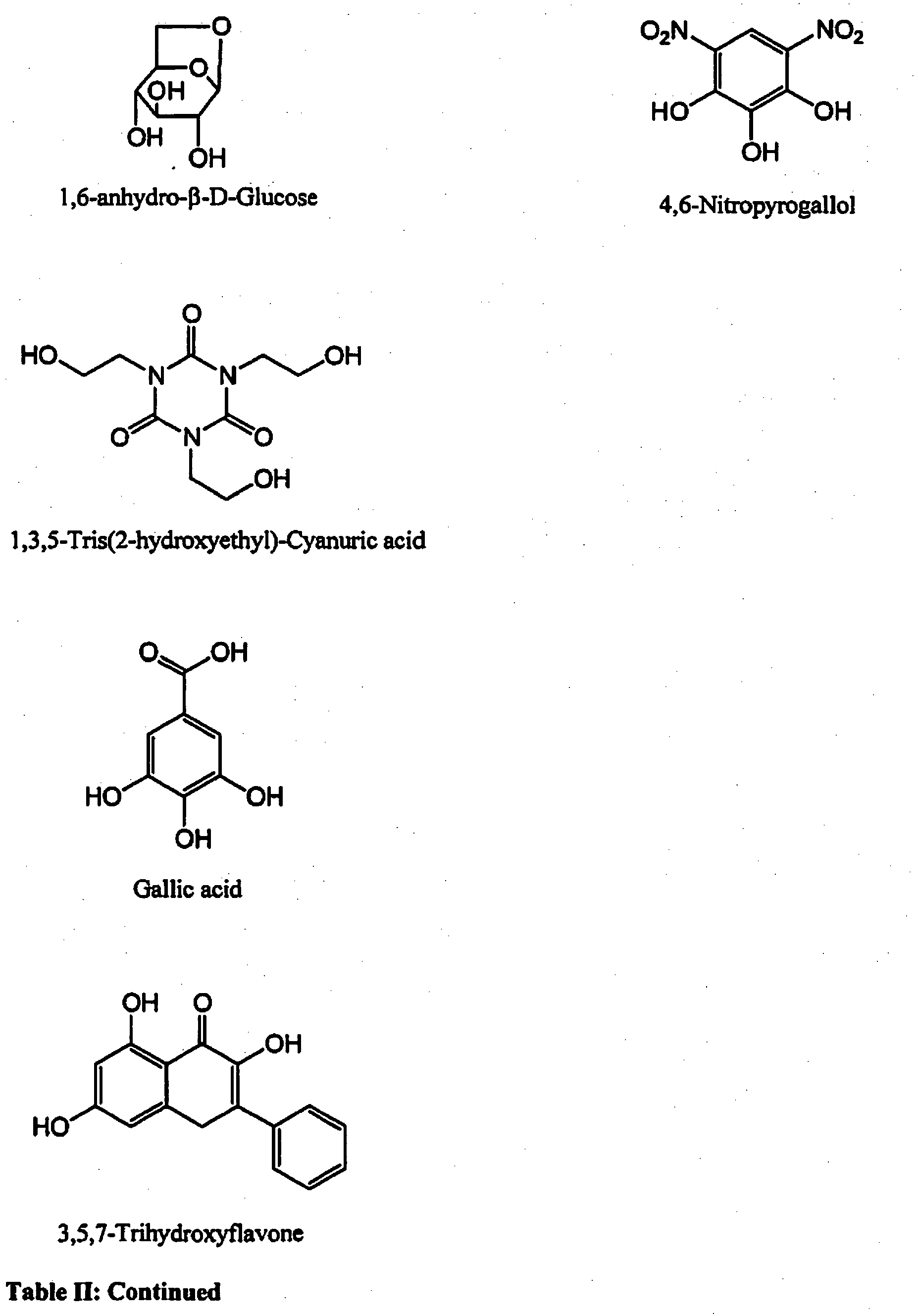

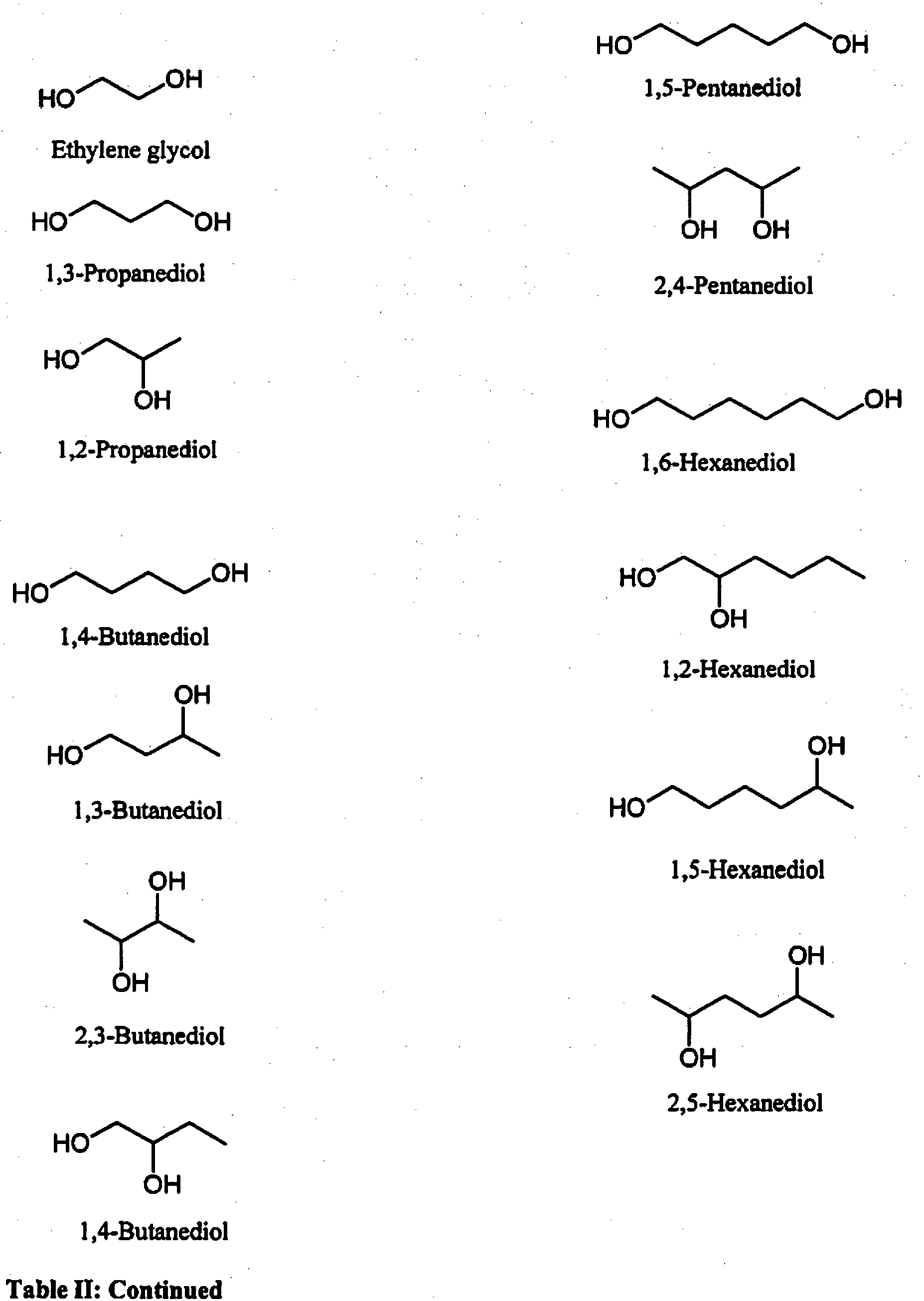

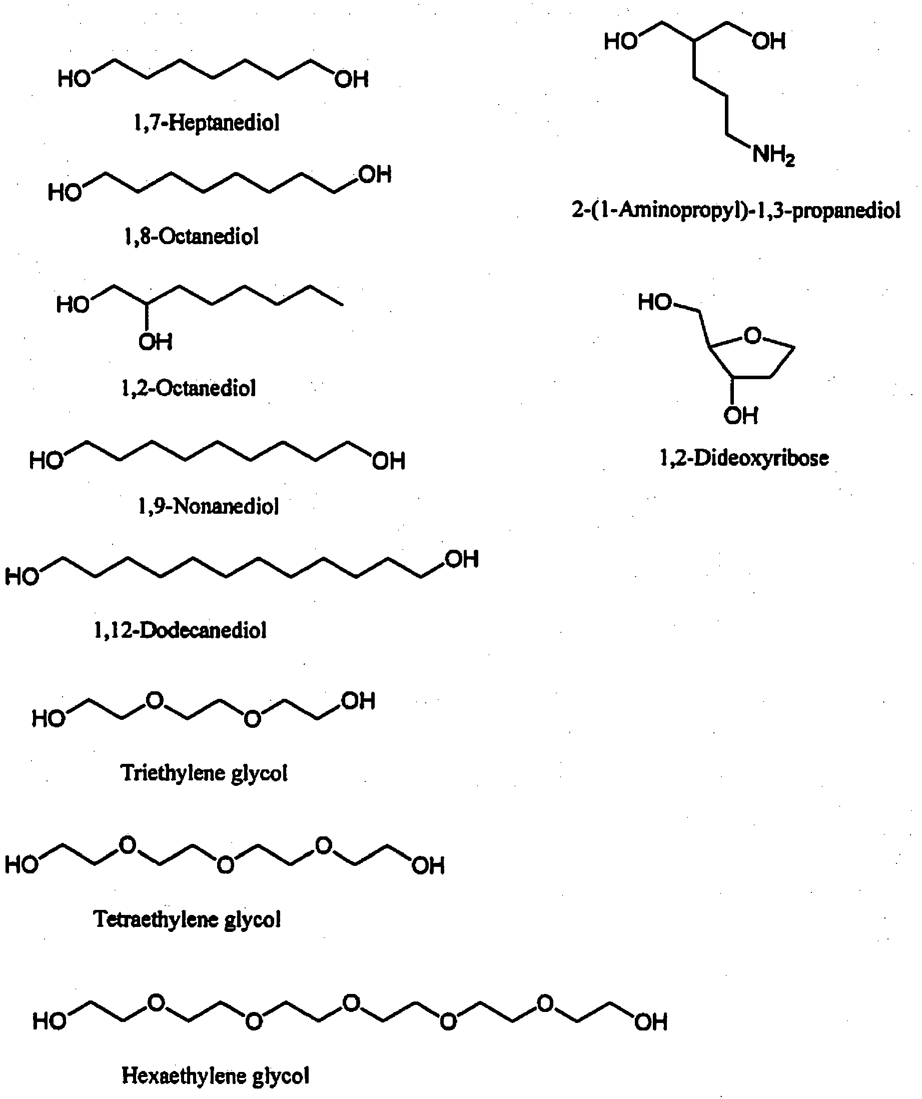

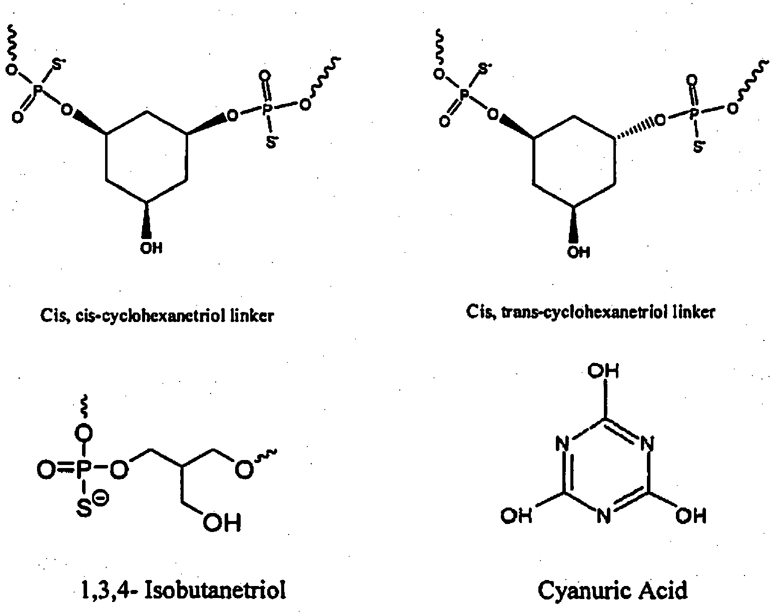

In a first aspect, the invention provides the immune modulatory compound 5'-TCG₂TCG₂TTU₁Y-M-YU₁TTG₂CTG₂CT-5' [5'-SEQ ID NO: 12-3'-M-3'-SEQ ID NO: 12-5']; wherein G₂ = AraG; U₁ = 2'-deoxy-U; M = cis,cis-1,3,5-cyclohexanetriol linker; Y = 1,3-propanediol linker.

In a second aspect, the invention provides a composition such as a pharmaceutical formulation comprising the immune modulatory compound according to the invention and a pharmaceutically acceptable carrier.

The active compound is included in the pharmaceutically acceptable carrier or diluent in an amount sufficient to deliver to a patient a pharmaceutically effective amount without causing serious toxic effects in the patient treated. The effective dosage range of the pharmaceutically acceptable derivatives can be calculated based on the weight of the parent compound to be delivered, or by other means known to those skilled in the art. If the derivative exhibits activity in itself, the effective dosage can be estimated as above using the weight of the derivative, or by other means known to those skilled in the art.

The invention also provides a vaccine comprising a pharmaceutical formulation according to the invention, and further comprising an antigen. An antigen is a molecule that elicits a specific immune response. Such antigens include, without limitation, proteins, peptides, nucleic acids, carbohydrates and complexes or combinations of any of the same. Any such antigen may optionally be linked to an immunogenic protein or peptide, such as keyhole limpet hemocyanin (KLH), cholera toxin B subunit, or any other immunogenic carrier protein.

Vaccines according to the invention may further include any of the plethora of known adjuvants, including, without limitation, Freund's complete adjuvant, Keyhole Limpet Hemocyanin (KLH), monophosphoryl lipid A (MPL), alum, and saponins, including QS-21, imiquimod, R848, TLR agonists or combinations thereof.

In a third aspect, the invention provides a compound according to the invention for use in a method for generating an immune response in an individual. Generating a TLR9-mediated immune response in an individual may comprise administering to the individual a compound, pharmaceutical formulation or vaccine according to the invention. In some embodiments, the individual is a mammal. In preferred embodiments, the compound, pharmaceutical formulation or vaccine is administered to an individual in need of immune stimulation.

Administration of a compound, pharmaceutical formulation or vaccine according to the invention can be by any suitable route, including, without limitation, parenteral, oral, intratumoral, sublingual, transdermal, topical, intranasal, aerosol, intraocular, intratracheal, intrarectal, mucosal, vaginal, by gene gun, dermal patch or in eye drop or mouthwash form. Administration of the compound, pharmaceutical formulation or vaccine can be carried out using known procedures at dosages and for periods of time effective to reduce symptoms or surrogate markers of the disease. When administered systemically, the compound, pharmaceutical formulation or vaccine is preferably administered at a sufficient dosage to attain a blood level of a compound according to the invention from about 0.0001 micromolar to about 10 micromolar. For localized administration, much lower concentrations than this may be effective, and much higher concentrations may be tolerated without serious toxic effects. Preferably, a total dosage of a compound according to the invention ranges from about 0.001 mg per patient per day to about 200 mg per kg body weight per day. It may be desirable to administer simultaneously, or sequentially a therapeutically effective amount of one or more of the therapeutic compositions of the invention to an individual as a single treatment episode.

In certain preferred embodiments, a compound, pharmaceutical formulation or vaccine according to the invention is co-administered or administered in combination with another agent, including without limitation antibodies, cytotoxic agents, allergens, antibiotics, antisense oligonucleotides, siRNA, aptamers, ribozymes, targeted therapies, kinase inhibitors, peptides, proteins, gene therapy vectors, DNA vaccines, and/or adjuvants to enhance the specificity or magnitude of the immune response.

The compound according to the invention is useful for the prophylactic or therapeutic treatment of human or animal disease. For example, the compound is useful for pediatric and veterinary vaccine applications. The compound is also useful for model studies of the immune system.

In a fourth aspect, the invention provides a compound according to the invention for use in a method for therapeutically treating an individual having a disease or disorder where modulating an immune response would be beneficial. Therapeutically treating a patient having a disease or disorder may comprise administering to the patient a compound, pharmaceutical formulation or vaccine according to the invention. In various embodiments, the disease or disorder to be treated is cancer, an autoimmune disorder, infectious disease, airway inflammation, inflammatory disorders, allergy, asthma or a disease caused by a pathogen or allergen. Pathogens include for example bacteria, parasites, fungi, viruses, viroids, and prions. Administration is carried out as described for the third aspect of the invention.

In a fifth aspect, the invention provides a compound according to the invention for use in a method for prophylactically treating an individual having a disease or disorder where modulating an immune response would be beneficial. Preventing a disease or disorder may comprise administering to the patient a compound, pharmaceutical formulation or vaccine according to the invention. In various embodiments, the disease or disorder to be prevented is cancer, an autoimmune disorder, airway inflammation, inflammatory disorders, infectious disease, allergy, asthma or a disease caused by a pathogen. Pathogens include, without limitation, bacteria, parasites, fungi, viruses, viroids, and prions. Administration is carried out as described for the third aspect of the invention.

The compound, pharmaceutical formulation or vaccine according to the invention can be co-administered or administered in combination with any other agent useful for preventing or treating the disease or condition that does not abolish the immune stimulatory effect of the compound, pharmaceutical formulation or vaccine according to the invention. The agent useful for preventing or treating the disease or condition includes, but is not limited to, vaccines, antigens, antibodies, cytotoxic agents, allergens, antibiotics, antisense oligonucleotides, TLR agonists, kinase inhibitors, peptides, proteins, gene therapy vectors, DNA vaccines and/or adjuvants to enhance the specificity or magnitude of the immune response, or co-stimulatory molecules such as cytokines, chemokines, protein ligands, trans-activating factors, peptides and peptides comprising modified amino acids. For example, in the prevention and/or treatment of cancer, it is contemplated that the compound, pharmaceutical formulation or vaccine according to the invention may be co-administered or administered in combination with a chemotherapeutic compound or a monoclonal antibody. Preferred chemotherapeutic agents include, without limitation Gemcitabine methotrexate, vincristine, adriamycin, cisplatin, non-sugar containing chloroethylnitrosoureas, 5-fluorouracil, mitomycin C, bleomycin, doxorubicin, dacarbazine, Taxol®, fragyline, Meglamine GLA, valrubicin, carmustaine and poliferposan, MMI270, BAY 12-9566, RAS famesyl transferase inhibitor, famesyl transferase inhibitor, MMP, MTA/LY231514, LY264618/Lometexol, Glamolec, CI-994, TNP-470, Hycamtin/Topotecan, PKC412, Valspodar/PSC833, Novantrone®/Mitroxantrone, Metaret/Suramin, Batimastat, E7070, BCH-4556, CS-682, 9-AC, AG3340, AG3433, Incel/VX-710, VX-853, ZD0101, ISI641, ODN 698, TA 2516/Marmistat, BB2516/Marmistat, CDP 845, D2163, PD183805, DX8951 f, Lemonal DP 2202, FK 317, imatinib mesylate/Gleevec®, Picibanil/OK-432, AD 32/Valrubicin, Metastron®/strontium derivative, Temodal/Temozolomide, Evacet/liposomal doxorubicin, Yewtaxan/Placlitaxel, Taxol®/Paclitaxel, Xeload/Capecitabine, Furtulon/Doxifluridine, Cyclopax/oral paclitaxel, Oral Taxoid, SPU-077/Cisplatin, HMR 1275/Flavopiridol, CP-358 (774)/EGFR, CP-609 (754)/RAS oncogene inhibitor, BMS-182751/oral platinum, UFT™(Tegafur/Uracil), Ergamisol®/Levamisole, Eniluracil/776C85/5FU enhancer, Campto/Levamisole, Camptosar®/Irinotecan, Tumodex/Ralitrexed, Leustatin®/Cladribine, Paxex/Paclitaxel, Doxil®/Iiposomal doxorubicin, Caelyx/liposomal doxorubicin, Fludara®/Fludarabine, Pharmarubicin/Epirubicin, DepoCyt®, ZD 1839, LU 79553/Bis-Naphtalimide, LU 103793/Dolastain, Caetyx/liposomal doxorubicin, Gemzar®/Gemcitabine, ZD 0473/Anormed®, YM 116, Iodine seeds, CDK4 and CDK2 inhibitors, PARP inhibitors, D4809/Dexifosamide, Ifes/Mesnex®/Ifosamide, Vumon®/Teniposide, Paraplatin®/Carboplatin, Plantinol/cisplatin, Vepeside/Etoposide, ZD 9331, Taxotere®/Docetaxel, prodrug of guanine arabinoside, Taxane Analog, nitrosoureas, alkylating agents such as melphelan and cyclophosphamide, Aminoglutethimide, Asparaginase, Busulfan, Carboplatin, Chlorombucil, Cytarabine HCl, Dactinomycin, Daunorubicin HCl, Estramustine phosphate sodium, Etoposide (VP16-213), Floxuridine, Fluorouracil (5-FU), Flutamide, Hydroxyurea (hydroxycarbamide), Ifosfamide, Interferon Alfa-2a, Alfa-2b, Leuprolide acetate (LHRH-releasing factor analogue), Lomustine (CCNU), Mechlorethamine HCl (nitrogen mustard), Mercaptopurine, Mesna, Mitotane (o.p'-DDD), Mitoxantrone HCl, Octreotide, Plicamycin, Procarbazine HCl, Streptozocin, Tamoxifen citrate, Thioguanine, Thiotepa, Vinblastine sulfate, Amsacrine (m-AMSA), Azacitidine, Erthropoietin, Hexamethylmelamine (HMM), Interleukin 2, Mitoguazone (methyl-GAG; methyl glyoxal bis-guanylhydrazone; MGBG), Pentostatin (2'deoxycoformycin), Semustine (methyl-CCNU), Teniposide (VM-26) and Vindesine sulfate. Preferred monocloncal antibodies include, but are not limited to, Panorex® (Glaxo-Welicome), Rituxan® (IDEC/Genentech/Hoffman la Roche), Mylotarg® (Wyeth), Campath® (Millennium), Zevalin® (IDEC and Schering AG), Bexxar® (Corixa/GSK), Erbitux® (Imclone/BMS), Avastin® (Genentech) Herceptin® (Genentech/Hoffman la Roche), Tarceva®(OSI Pharmaceuticals/Genentech).

Alternatively, the agent useful for preventing or treating the disease or condition can include DNA vectors encoding for antigen or allergen. In these embodiments, the compound, pharmaceutical formulation or vaccine according to the invention can variously act as adjuvants and/or produce direct immunomodulatory effects.

The following examples are intended to further illustrate certain preferred embodiments of the invention. Compounds which are not encompassed by the subject-matter of the claims are described for comparative purposes only.

### Example 1:

### Synthesis of oligonucleotide-based compounds containing immune stimulatory moieties

Chemical entities according to the invention were synthesized on a 1 µmol to 0.1 mM scale using an automated DNA synthesizer (OligoPilot II, AKTA, (Amersham) and/or Expedite 8909 (Applied Biosystem)), following the linear synthesis or parallel synthesis procedures outlined in Figures 1 and 2.

5'-DMT dA, dG, dC and T phosphoramidites were purchased from Proligo (Boulder, CO). 5'-DMT 7-deaza-dG and araG phosphoramidites were obtained from Chemgenes (Wilmington, MA). DiDMT-glycerol linker solid support was obtained from Chemgenes. 1-(2'-deoxy-β-D-ribofuranosyl)-2-oxo-7-deaza-8-methyl-purine amidite was obtained from Glen Research (Sterling, VA), 2'-O-methylribonuncleoside amidites were obtained from Promega (Obispo, CA). All compounds according to the invention were phosphorothioate backbone modified.

All nucleoside phosphoramidites were characterized by ³¹P and ¹H NMR spectra. Modified nucleosides were incorporated at specific sites using normal coupling cycles recommended by the supplier. After synthesis, compounds were deprotected using concentrated ammonium hydroxide and purified by reverse phase HPLC, detritylation, followed by dialysis. Purified compounds as sodium salt form were lyophilized prior to use. Purity was tested by CGE and MALDI-TOF MS. Endotoxin levels were determined by LAL test and were below 1.0 EU/mg.

### Example 2:

### Cell Culture Conditions and Reagents

HEK293 or HEK293XL cells expressing mouse TLR9 (Invivogen, San Diego, CA) were cultured in 48-well plates in 250 µl/well DMEM supplemented with 10% heat-inactivated FBS in a 5% CO₂ incubator. At 80% confluence, cultures were transiently transfected with 400 ng/ml of SEAP (secreted form of human embryonic alkaline phosphatase) reporter plasmid (pNifty2-Seap) (Invivogen) in the presence of 4 µl/ml of lipofectamine (Invitrogen, Carlsbad, CA) in culture medium. Plasmid DNA and lipofectamine were diluted separately in serum-free medium and incubated at room temperature for 5 minutes. After incubation, the diluted DNA and lipofectamine were mixed and the mixtures were incubated at room temperature for 20 minutes. Aliquots of 25 µl of the DNA/lipofectamine mixture containing 100 ng of plasmid DNA and 1 µl of lipofectamine were added to each well of the cell culture plate, and the cultures were continued for 4 hours.

### Cytokine induction by exemplar compounds from Table I in HEK293 cells expressing mouse TLR9

After transfection, medium was replaced with fresh culture medium, exemplar compounds from Table I were added to the cultures at concentrations of 0, 0.1, 0.3, 1.0, 3.0, or 10.0 µg/ml, and the cultures were continued for 18 hours. At the end of compounds treatment, the levels of NF-κB were determined using SEAP (secreted form of human embryonic alkaline phosphatase) assay according to the manufacturer's protocol (Invivogen). Briefly, 30 µl of culture supernatant was taken from each treatment and incubated with p-nitrophynyl phosphate substrate and the yellow color generated was measured by a plate reader at 405 nm (Putta MR et al, Nucleic Acids Res., 2006, 34:3231-8).

### Example 3:

### Cytokine induction by exemplar compounds from Table I in human PBMCs, pDCs, and mouse splenocytes

### Human PBMC isolation

Peripheral blood mononuclear cells (PBMCs) from freshly drawn healthy volunteer blood (CBR Laboratories, Boston, MA) were isolated by Ficoll density gradient centrifugation method (Histopaque-1077, Sigma).

### Human pDC isolation

Human plasmacytoid dendritic cells (pDCs) were isolated from freshly obtained healthy human volunteer's blood PBMCs by positive selection using the BDCA4 cell isolation kits (Miltenyi Biotec) according to the manufacturer's instructions.

### Mouse splenocyte isolation

Human PBMCs were plated in 48-well plates using 5x10⁶ cells/ml. Human pDCs were plated in 96-well dishes using 1X10⁶ cells/ml. The exemplar compounds from Table I dissolved in DPBS (pH 7.4; Mediatech) were added to the cell cultures at doses of 0, 0.1,0.3,1.0,3.0, or 10.0 µg/ml. The cells were then incubated at 37 °C for 24 hours and the supernatants were collected for luminex multiplex or ELISA assays. In certain experiments, the levels of IFN-α, IL-6, and/or IL-12 were measured by sandwich ELISA. The required reagents, including cytokine antibodies and standards, were purchased from PharMingen.

### Cytokine Luminex Multiplex

In certain experiments, the levels of IL-1Rα, IL-6, IL-10, IL-12, IFN-α, IFN-y, MIP-1α, MIP-β, MCP-1, and IL-12p40p70 in culture supernatants were measured by Luminex multiplex assays, which were performed using Biosource human multiplex cytokine assay kits on Luminex 100 instrument and the data were analyzed using StarStation software supplied by Applied Cytometry Systems (Sacramento, CA).

### Activation of Human Immune Cells.

Human plasmacytoid dendritic cells (pDCs) were isolated from freshly obtained healthy human blood PBMCs and cultured with 50 µg/ml of TLR9 agonists or control for 24 hr. Cells were stained with fluorescently-conjugated Abs (CD123, CD80, CD86) and data were collected on an FC500 MPL cytometer. Mean fluorescence intensity of CD80 and CD86 on CD123⁺ cells was analyzed using FlowJo software and is expressed as fold change over PBS control.

Human myeloid dendritic cells (mDC) were isolated from freshly obtained healthy human blood PBMCs and cultured with 50 µg/ml of TLR9 agonists or control for 24 hr. Cells were stained with fluorescently-conjugated Abs (CD11c, CD80, CD40) and data were collected on an FC500 MPL cytometer. Mean fluorescence intensity of CD80 and CD40 on CD11c⁺ cells was analyzed using FlowJo software and is expressed as fold change over PBS control.

### Example 4:

### Human B cell proliferation assay in the presence of exemplar compounds from Table I

Human B cells were isolated from PBMCs by positive selection using the CD19 Cell Isolation Kit (Miltenyi Biotec, Auburn, CA) according to the manufacturer's instructions.

The culture medium used for the assay consisted of RPMI 1640 medium supplemented with 1.5 mM glutamine, 1 mM sodium pyruvate, 0.1 mM non-essential amino acids, 50 µM 2-mercaptoethanol, 100 IU/ml penicillin-streptomycin mix and 10% heat-inactivated fetal bovine serum.

A total of 0.5 X 10⁶ B cells per ml (i.e. 1X 10⁵/200 µl/well) were stimulated in 96 well flat bottom plates with different concentrations of exemplar compounds from Table I in triplicate for a total period of 68 hours. After 68 hours, cells were pulsed with 0.75 µCi of [³H]-thymidine (1Ci = 37 GBq; Perkin Elmer Life Sciences) in 20 µl RPMI 1640 medium (no serum) per well and harvested 6-8 hours later. The plates were then harvested using a cell harvester and radioactive incorporation was determined using standard liquid scintillation technique. In some cases the corresponding [³H]-T (cpm) was converted into a proliferation index and reported as such.

### Example 5:

### In vivo cytokine secretion in mouse model treated with TLR9 agonist compounds

C57BL/6 mice and BALB/c mice, 5-6 weeks old, were obtained from Taconic Farms, Germantown, NY and maintained in accordance with Idera Pharmaceutical's IACUC approved animal protocols. Mice (n=3) were injected subcutaneously (s.c) with individual immune modulatory compounds from Table I at 0.25 or 1.0 mg/kg (single dose). Serum was collected by retro-orbital bleeding 2 hours after immune modulatory compound administration and IL-12, IL-10, IL-6, IP-10, KC, MCP1, MIG, MIP-1α and TNF-α concentrations were determined by sandwich ELISA or Luminex multiplex assays. The results are shown in Figures 7A, 7B, 7C, 7D, 7E and 8F and demonstrate that *in vivo* administration of immune modulatory compounds containing novel chemical compositions generates unique cytokine and chemokine profiles. All reagents, including cytokine and chemokine antibodies and standards were purchased from PharMingen. (San Diego, CA)

## Claims

1. The immune modulatory compound
5'-TCG₂TCG₂TTU₁Y-M-YU₁TTG₂CTG₂CT-5'
[5'-SEQ ID NO: 12-3'-M-3'-SEQ ID NO: 12-5'];
wherein G₂ = AraG; U₁ = 2'-deoxy-U; M = cis,cis-1,3,5-cyclohexanetriol linker; Y = 1,3-propanediol linker.

2. A composition comprising the immune modulatory compound of claim 1 and a physiologically acceptable carrier.

3. A compound according to claim 1 for use in a method for generating an immune response in an individual.

4. A compound according to claim 1 for use in a method for therapeutically treating an individual having a disease or disorder where modulating an immune response would be beneficial.

5. A compound according to claim 1 for use in a method for therapeutically treating an individual having a disease or disorder where modulating an immune response would be beneficial wherein the disease or disorder is cancer, an autoimmune disorder, airway inflammation, inflammatory disorder, infectious disease, skin disorder, allergy, asthma or a disease caused by a pathogen or allergen.

6. A compound according to claim 1 for use in a method for therapeutically treating an individual having a disease or disorder where modulating an immune response would be beneficial, wherein the method further comprises administering one or more chemotherapeutic compounds, targeted therapeutic agents, vaccines, antigens, antibodies, cytotoxic agents, allergens, antibiotics, antisense oligonucleotides, TLR agonists, kinase inhibitors, peptides, proteins, DNA vaccines, adjuvants, co-stimulatory molecules or combinations thereof.

7. A compound according to claim 1 for use in a method for prophylactically treating an individual having a disease or disorder where modulating an immune response would be beneficial.

8. A compound according to claim 1 for use in a method for prophylactically treating an individual having a disease or disorder where modulating an immune response would be beneficial wherein the disease or disorder is cancer, an autoimmune disorder, airway inflammation, inflammatory disorders, infectious disease, skin disorders, allergy, asthma or a disease caused by a pathogen or allergen.

9. A compound according to claim 1 for use in a method for prophylactically treating an individual having a disease or disorder where modulating an immune response would be beneficial, wherein the method further comprises administering one or more chemotherapeutic compounds, targeted therapeutic agents, vaccines, antigens, antibodies, cytotoxic agents, allergens, antibiotics, antisense oligonucleotides, TLR agonists, kinase inhibitors, peptides, proteins, DNA vaccines, adjuvants, co-stimulatory molecules or combinations thereof.

## Patentansprüche

1. Die immunmodulatorische Verbindung
5'-TCG₂TCG₂TTU₁Y-M-YU₁TTG₂CTG₂CT-5'
[5'-SEQ ID NO: 12-3'-M-3'-SEQ ID NO: 12-5'],
worin G₂ = AraG; U₁ = 2'-Desoxy-U; M = cis,cis-1,3,5-Cyclohexantriollinker; Y = 1,3-Propandiollinker.

2. Zusammensetzung, die die immunmodulatorische Verbindung nach Anspruch 1 und einen physiologisch verträglichen Träger umfasst.

3. Verbindung nach Anspruch 1 zur Verwendung in einem Verfahren zur Erzeugung einer Immunantwort in einem Individuum.

4. Verbindung nach Anspruch 1 zur Verwendung in einem Verfahren zur therapeutischen Behandlung eines Individuums mit einer Erkrankung oder Störung, bei der eine Modulation einer Immunantwort günstig wäre.

5. Verbindung nach Anspruch 1 zur Verwendung in einem Verfahren zur therapeutischen Behandlung eines Individuums mit einer Erkrankung oder Störung, bei der eine Modulation einer Immunantwort günstig wäre, wobei die Erkrankung oder Störung Krebs, eine Autoimmunerkrankung, Atemwegsentzündung, entzündliche Erkrankung, Infektionserkrankung, Hauterkrankung, Allergie, Asthma oder eine Erkrankung, die durch ein Pathogen oder Allergen hervorgerufen wird, ist.

6. Verbindung nach Anspruch 1 zur Verwendung in einem Verfahren zur therapeutischen Behandlung eines Individuums mit einer Erkrankung oder Störung, bei der eine Modulation einer Immunantwort günstig wäre, wobei das Verfahren ferner ein Verabreichen eines oder mehrerer Chemotherapeutika, zielgesteuerter therapeutischer Mittel, Impfstoffe, Antigene, Antikörper, cytotoxischer Mittel, Allergene, Antibiotika, Antisense-Oligonukleotide, TLR-Agonisten, Kinase-Inhibitoren, Peptide, Proteine, DNA-Impfstoffe, Adjuvantien, co-stimulierender Moleküle oder Kombinationen davon umfasst.

7. Verbindung nach Anspruch 1 zur Verwendung in einem Verfahren zur prophylaktischen Behandlung eines Individuums mit einer Erkrankung oder Störung, bei der eine Modulation einer Immunantwort günstig wäre.

8. Verbindung nach Anspruch 1 zur Verwendung in einem Verfahren zur prophylaktischen Behandlung eines Individuums mit einer Erkrankung oder Störung, bei der eine Modulation einer Immunantwort günstig wäre, wobei die Erkrankung oder Störung Krebs, eine Autoimmunerkrankung, Atemwegsentzündung, entzündliche Erkrankungen, Infektionserkrankung, Hauterkrankungen, Allergie, Asthma oder eine Erkrankung, die durch ein Pathogen oder Allergen hervorgerufen wird, ist.

9. Verbindung nach Anspruch 1 zur Verwendung in einem Verfahren zur prophylaktischen Behandlung eines Individuums mit einer Erkrankung oder Störung, bei der eine Modulation einer Immunantwort günstig wäre, wobei das Verfahren ferner ein Verabreichen eines oder mehrerer Chemotherapeutika, zielgesteuerter therapeutischer Mittel, Impfstoffe, Antigene, Antikörper, cytotoxischer Mittel, Allergene, Antibiotika, Antisense-Oligonukleotide, TLR-Agonisten, Kinase-Inhibitoren, Peptide, Proteine, DNA-Impfstoffe, Adjuvantien, co-stimulierender Moleküle oder Kombinationen davon umfasst.

## Revendications

1. Composé modulateur immunitaire
5'-TCG₂TCG₂TTU₁Y-M-YU₁TTG₂CTG₂CT-5'
[5'-SEQ ID NO:12-3'-M-3'-SEQ ID NO:12-5'];
dans lequel G₂ = AraG; U₁ = 2'-désoxy-U; M = lieur cis,cis-1,3,5-cyclohexanetriol; Y = lieur 1,3-propanediol.

2. Composition comprenant le composé modulateur immunitaire selon la revendication 1 et un support physiologiquement acceptable.

3. Composé selon la revendication 1 pour utilisation dans un procédé pour générer une réponse immunitaire chez un individu.

4. Composé selon la revendication 1 pour utilisation dans un procédé pour le traitement thérapeutique d'un individu ayant une maladie ou un désordre dans laquelle/lequel la modulation d'une réponse immunitaire serait bénéfique.

5. Composé selon la revendication 1 pour utilisation dans un procédé pour le traitement thérapeutique d'un individu ayant une maladie ou un désordre dans laquelle/lequel la modulation d'une réponse immunitaire serait bénéfique, tandis que la maladie ou le désordre est un cancer, un désordre auto-immun, une inflammation des voies supérieures, un désordre inflammatoire, une maladie infectieuse, un désordre cutané, une allergie, l'asthme ou une affection provoquée par un agent pathogène ou un allergène.

6. Composé selon la revendication 1 pour utilisation dans un procédé pour le traitement thérapeutique d'un individu ayant une maladie ou un désordre dans laquelle/lequel la modulation d'une réponse immunitaire serait bénéfique, tandis que le procédé comprend en outre l'administration d'un ou plusieurs composés chimiothérapeutiques, agents thérapeutiques ciblés, vaccins, antigènes, anticorps, agents cytotoxiques, allergènes, antibiotiques, oligonucléotides antisens, agonistes de TLR, inhibiteurs de kinase, peptides, protéines, vaccins à ADN, adjuvants, molécules co-stimulatrices, ou combinaisons de ceux-ci.

7. Composé selon la revendication 1 pour utilisation dans un procédé pour le traitement prophylactique d'un individu ayant une maladie ou un désordre dans laquelle/lequel la modulation d'une réponse immunitaire serait bénéfique.

8. Composé selon la revendication 1 pour utilisation dans un procédé pour le traitement prophylactique d'un individu ayant une maladie ou un désordre dans laquelle/lequel la modulation d'une réponse immunitaire serait bénéfique, tandis que la maladie ou le désordre est un cancer, un désordre auto-immun, une inflammation des voies aériennes, un désordre inflammatoire, une maladie infectieuse, un désordre cutané, une allergie, l'asthme ou une maladie provoquée par un agent pathogène ou un allergène.

9. Composé selon la revendication 1 pour utilisation dans un procédé pour le traitement prophylactique d'un individu ayant une maladie ou un désordre dans laquelle/lequel la modulation d'une réponse immunitaire serait bénéfique, tandis que le procédé comprend en outre l'administration d'un ou plusieurs composés chimiothérapeutiques, agents thérapeutiques ciblés, vaccins, antigènes, anticorps, agents cytotoxiques, allergènes, antibiotiques, oligonucléotides antisens, agonistes de TLR, inhibiteurs de kinase, peptides, protéines, vaccins à ADN, adjuvants, molécules co-stimulatrices, ou combinaisons de ceux-ci.


### SEQUENCE LISTING

<110> Idera Pharmaceuticals, Inc.
<120> NOVEL SYNTHETIC AGONISTS OF TLR9
<130> 725-1 T1
<150> US 60/953,251
   <151> 2007-08-01
<150> US 60/983,601
   <151> 2007-10-30
<150> US 60/987,151
   <151> 2007-11-12
<150> US 61/015,292
   <151> 2007-12-20
<160> 191
<170> PatentIn version 3.5
<210> 1
   <211> 11
   <212> DNA
   <213> Artificial Sequence
<220>
   <223> Description of Artificial Sequence: Synthetic oligonucleotide
<220>
   <221> modified_base
   <222> (3) .. (3)
   <223> 7-deaza-dG
<220>
   <221> modified_base
   <222> (7)..(7)
   <223> 7-deaza-dG
<220>
   <221> modified_base
   <222> (11)..(11)
   <223> 7-deaza-dG
<400> 1
   tcgtacgtac g 11
<210> 2
   <211> 11
   <212> DNA
   <213> Artificial Sequence
<220>
   <223> Description of Artificial Sequence: Synthetic oligonucleotide
<220>
   <221> modified_base
   <222> (7)..(7)
   <223> AraG
<400> 2
   tctgtcgttg t 11
<210> 3
   <211> 11
   <212> DNA
   <213> Artificial Sequence
<220>
   <223> Description of Artificial Sequence: Synthetic oligonucleotide
<220>
   <221> modified_base
   <222> (7)..(7)
   <223> AraG
<400> 3
   tcagtcgtta c 11
<210> 4
   <211> 10
   <212> DNA
   <213> Artificial Sequence
<220>
   <223> Description of Artificial Sequence: Synthetic oligonucleotide
<220>
   <221> modified_base
   <222> (7)..(7)
   <223> AraG
<400> 4
   tctgtcgtag 10
<210> 5
   <211> 9
   <212> DNA
   <213> Artificial Sequence
<220>
   <223> Description of Artificial Sequence: Synthetic oligonucleotide
<220>
   <221> modified_base
   <222> (3)..(3)
   <223> 7-deaza-dG
<220>
   <221> modified_base
   <222> (6).. (6)
   <223> 7-deaza-dG
<400> 5
   tcgtcgttt 9
<210> 6
   <211> 9
   <212> DNA
   <213> Artificial Sequence
<220>
   <223> Description of Artificial Sequence: Synthetic oligonucleotide
<220>
   <221> modified_base
   <222> (3)..(3)
   <223> 7-deaza-dG
<220>
   <221> modified_base
   <222> (6)..(6)
   <223> 7-deaza-dG
<400> 6
   tcgtcgttt 9
<210> 7
   <211> 11
   <212> DNA
   <213> Artificial Sequence
<220>
   <223> Description of Artificial Sequence: Synthetic oligonucleotide
<220>
   <221> modified_base
   <222> (3) .. (3)
   <223> 7-deaza-dG
<220>
   <221> modified_base
   <222> (7)..(7)
   <223> 7-deaza-dG
<220>
   <221> modified_base
   <222> (11) .. (11)
   <223> 2'-O-methylribonucleotide
<400> 7
   tcgaacgttc g 11
<210> 8
   <211> 8
   <212> DNA
   <213> Artificial Sequence
<220>
   <223> Description of Artificial Sequence: Synthetic oligonucleotide
<220>
   <221> modified_base
   <222> (3)..(3)
   <223> 7-deaza-dG
<220>
   <221> modified_base
   <222> (6)..(6)
   <223> 7-deaza-dG
<400> 8
   tcgtcgtt 8
<210> 9
   <211> 11
   <212> DNA
   <213> Artificial Sequence
<220>
   <223> Description of Combined DNA/RNA molecule: Synthetic oligonucleotide
<220>
   <223> Description of Artificial Sequence: Synthetic oligonucleotide
<220>
   <221> modified_base
   <222> (7)..(7)
   <223> AraG
<220>
   <221> modified_base
   <222> (10)..(11)
   <223> 2'-O-methylribonucleotide
<400> 9
   tctgtcgttc u 11
<210> 10
   <211> 11
   <212> DNA
   <213> Artificial Sequence
<220>
   <223> Description of Combined DNA/RNA molecule: Synthetic oligonucleotide
<220>
   <223> Description of Artificial Sequence: Synthetic oligonucleotide
<220>
   <221> modified_base
   <222> (3)..(3)
   <223> 7-deaza-dG
<220>
   <221> modified_base
   <222> (6) .. (6)
   <223> 7-deaza-dG
<220>
   <221> modified_base
   <222> (10) .. (11)
   <223> 2'-O-methylribonucleotide
<400> 10
   tcgtcgtttu u 11
<210> 11
   <211> 9
   <212> DNA
   <213> Artificial Sequence
<220>
   <223> Description of Combined DNA/RNA molecule: Synthetic oligonucleotide
<220>
   <223> Description of Artificial Sequence: Synthetic oligonucleotide
<220>
   <221> modified_base
   <222> (3) .. (3)
   <223> 7-deaza-dG
<220>
   <221> modified_base
   <222> (6)..(6)
   <223> 7-deaza-dG
<220>
   <221> modified_base
   <222> (9)..(9)
   <223> 2'-deoxy-U
<400> 11
   tcgtcgttu 9
<210> 12
   <211> 9
   <212> DNA
   <213> Artificial Sequence
<220>
   <223> Description of Combined DNA/RNA molecule: Synthetic oligonucleotide
<220>
   <223> Description of Artificial Sequence: Synthetic oligonucleotide
<220>
   <221> modified_base
   <222> (3)..(3)
   <223> AraG
<220>
   <221> modified_base
   <222> (6)..(6)
   <223> AraG
<220>
   <221> modified_base
   <222> (9)..(9)
   <223> 2'-deoxy-U
<400> 12
   tcgtcgttu 9
<210> 13
   <211> 11
   <212> DNA
   <213> Artificial Sequence
<220>
   <223> Description of Artificial Sequence: Synthetic oligonucleotide
<220>
   <221> modified_base
   <222> (6)..(6)
   <223> 7-deaza-dG
<400> 13
   tagtcgttct c 11
<210> 14
   <211> 11
   <212> DNA
   <213> Artificial Sequence
<220>
   <223> Description of Combined DNA/RNA molecule: Synthetic oligonucleotide
<220>
   <223> Description of Artificial Sequence: Synthetic oligonucleotide
<220>
   <221> modified_base
   <222> (3)..(3)
   <223> 2'-O-methylribonucleotide
<220>
   <221> modified_base
   <222> (8)..(8)
   <223> 7-deaza-dG
<400> 14
   tcutgtcgtt c 11
<210> 15
   <211> 11
   <212> DNA
   <213> Artificial Sequence
<220>
   <223> Description of Artificial Sequence: Synthetic oligonucleotide
<220>
   <221> modified_base
   <222> (3)..(3)
   <223> 7-deaza-dG
<220>
   <221> modified_base
   <222> (6)..(6)
   <223> 7-deaza-dG
<400> 15
   tcgtcgtttt t 11
<210> 16
   <211> 11
   <212> DNA
   <213> Artificial Sequence
<220>
   <223> Description of Artificial Sequence: Synthetic oligonucleotide
<220>
   <221> modified_base
   <222> (3)..(3)
   <223> 7-deaza-dG
<220>
   <221> modified_base
   <222> (6)..(6)
   <223> 7-deaza-dG
<400> 16
   tcgtcgtttt t 11
<210> 17
   <211> 11
   <212> DNA
   <213> Artificial Sequence
<220>
   <223> Description of Artificial Sequence: Synthetic oligonucleotide
<220>
   <221> modified_base
   <222> (7)..(7)
   <223> 7-deaza-dG
<400> 17
   tctgtcgttc t 11
<210> 18
   <211> 11
   <212> DNA
   <213> Artificial Sequence
<220>
   <223> Description of Artificial Sequence: Synthetic oligonucleotide
<220>
   <221> modified_base
   <222> (3)..(3)
   <223> 7-deaza-dG
<220>
   <221> modified_base
   <222> (7)..(7)
   <223> 7-deaza-dG
<220>
   <221> modified_base
   <222> (11)..(11)
   <223> 7-deaza-dG
<400> 18
   tcgaacgttc g 11
<210> 19
   <211> 11
   <212> DNA
   <213> Artificial Sequence
<220>
   <223> Description of Artificial Sequence: Synthetic oligonucleotide
<220>
   <221> modified_base
   <222> (3)..(3)
   <223> 7-deaza-dG
<220>
   <221> modified_base
   <222> (7)..(7)
   <223> 7-deaza-dG
<220>
   <221> modified_base
   <222> (11)..(11)
   <223> 7-deaza-dG.
<400> 19
   tcgaacgttc g 11
<210> 20
   <211> 11
   <212> DNA
   <213> Artificial Sequence
<220>
   <223> Description of Artificial Sequence: Synthetic oligonucleotide
<220>
   <221> modified_base
   <222> (3)..(3)
   <223> 7-deaza-dG
<220>
   <221> modified_base
   <222> (7)..(7)
   <223> 7-deaza-dG
<220>
   <221> modified_base
   <222> (11)..(11)
   <223> 7-deaza-dG
<400> 20
   tcgaacgttc g 11
<210> 21
   <211> 11
   <212> DNA
   <213> Artificial Sequence
<220>
   <223> Description of Artificial Sequence: Synthetic oligonucleotide
<220>
   <221> modified_base
   <222> (3)..(3)
   <223> 7-deaza-dG
<220>
   <221> modified_base
   <222> (7)..(7)
   <223> 7-deaza-dG
<220>
   <221> modified_base
   <222> (11) .. (11)
   <223> 7-deaza-dG
<400> 21
   tcgaacgttc g 11
<210> 22
   <211> 11
   <212> DNA
   <213> Artificial Sequence
<220>
   <223> Description of Artificial Sequence: Synthetic oligonucleotide
<220>
   <221> modified_base
   <222> (7)..(7)
   <223> 7-deaza-dG
<400> 22
   tctgtcgttc t 11
<210> 23
   <211> 11
   <212> DNA
   <213> Artificial Sequence
<220>
   <223> Description of Artificial Sequence: Synthetic oligonucleotide
<220>
   <221> modified_base
   <222> (6)..(6)
   <223> AraG
<400> 23
   cagtcgttca g 11
<210> 24
   <211> 11
   <212> DNA
   <213> Artificial Sequence
<220>
   <223> Description of Artificial Sequence: Synthetic oligonucleotide
<220>
   <221> modified_base
   <222> (6) .. (6)
   <223> AraG
<400> 24
   cagtcgttca g 11
<210> 25
   <211> 11
   <212> DNA
   <213> Artificial Sequence
<220>
   <223> Description of Artificial Sequence: Synthetic oligonucleotide
<220>
   <221> modified_base
   <222> (3) .. (3)
   <223> 7-deaza-dG
<220>
   <221> modified_base
   <222> (7)..(7)
   <223> 7-deaza-dG
<220>
   <221> modified_base
   <222> (11)..(11)
   <223> 2'-O-methylribonucleotide
<400> 25
   tcgaacgttc g 11
<210> 26
   <211> 11
   <212> DNA
   <213> Artificial Sequence
<220>
   <223> Description of Artificial Sequence: Synthetic oligonucleotide
<220>
   <221> modified_base
   <222> (3) .. (3)
   <223> 7-deaza-dG
<220>
   <221> modified_base
   <222> (6)..(6)
   <223> 7-deaza-dG
<400> 26
   tcgtcgtttt t 11
<210> 27
   <211> 9
   <212> DNA
   <213> Artificial Sequence
<220>
   <223> Description of Combined DNA/RNA molecule: Synthetic oligonucleotide
<220>
   <223> Description of Artificial Sequence: Synthetic oligonucleotide
<220>
   <221> modified_base
   <222> (3) .. (3)
   <223> AraG
<220>
   <221> modified_base
   <222> (6)..(6)
   <223> AraG
<220>
   <221> modified_base
   <222> (9)..(9)
   <223> 2'-deoxy-U
<400> 27
   tcgtcgttu 9
<210> 28
   <211> 11
   <212> DNA
   <213> Artificial Sequence
<220>
   <223> Description of Combined DNA/RNA molecule: Synthetic oligonucleotide
<220>
   <223> Description of Artificial Sequence: Synthetic oligonucleotide
<220>
   <221> modified_base
   <222> (3)..(3)
   <223> 7-deaza-dG
<220>
   <221> modified_base
   <222> (7)..(7)
   <223> 7-deaza-dG
<220>
   <221> modified_base
   <222> (8)..(9)
   <223> 2'-deoxy-U
<220>
   <221> modified_base
   <222> (11)..(11)
   <223> 2'-O-methylribonucleotide
<400> 28
   tcgaacguuc g 11
<210> 29
   <211> 11
   <212> DNA
   <213> Artificial Sequence
<220>
   <223> Description of Artificial Sequence: Synthetic oligonucleotide
<220>
   <221> modified_base
   <222> (7)..(7)
   <223> 7-deaza-dG
<400> 29
   tctgtcgttc t 11
<210> 30
   <211> 11
   <212> DNA
   <213> Artificial Sequence
<220>
   <223> Description of Artificial Sequence: Synthetic oligonucleotide
<220>
   <221> modified_base
   <222> (6)..(6)
   <223> AraG
<400> 30
   cagtcgttca g 11
<210> 31
   <211> 11
   <212> DNA
   <213> Artificial Sequence
<220>
   <223> Description of Artificial Sequence: Synthetic oligonucleotide
<220>
   <221> modified_base
   <222> (6)..(6)
   <223> AraG
<400> 31
   cagtcgttca g 11
<210> 32
   <211> 11
   <212> DNA
   <213> Artificial Sequence
<220>
   <223> Description of Artificial Sequence: Synthetic oligonucleotide
<220>
   <221> modified_base
   <222> (7) .. (7)
   <223> 7-deaza-dG
<400> 32
   tctgtcgttc t 11
<210> 33
   <211> 11
   <212> DNA
   <213> Artificial Sequence
<220>
   <223> Description of Combined DNA/RNA molecule: Synthetic oligonucleotide
<220>
   <223> Description of Artificial Sequence: Synthetic oligonucleotide
<220>
   <221> modified_base
   <222> (3)..(3)
   <223> 7-deaza-dG
<220>
   <221> modified_base
   <222> (7)..(7)
   <223> 7-deaza-dG
<220>
   <221> modified_base
   <222> (8)..(9)
   <223> 2'-deoxy-U
<220>
   <221> modified_base
   <222> (11)..(11)
   <223> 2'-O-methylribonucleotide
<400> 33
   tcgaacguuc g 11
<210> 34
   <211> 11
   <212> DNA
   <213> Artificial Sequence
<220>
   <223> Description of Artificial Sequence: Synthetic oligonucleotide
<220>
   <221> modified_base
   <222> (3) .. (3)
   <223> 7-deaza-dG
<220>
   <221> modified_base
   <222> (7) .. (7)
   <223> 7-deaza-dG
<220>
   <221> modified_base
   <222> (11)..(11)
   <223> 2'-O-methylribonucleotide
<400> 34
   tcgaacgttc g 11
<210> 35
   <211> 11
   <212> DNA
   <213> Artificial Sequence
<220>
   <223> Description of Artificial Sequence: Synthetic oligonucleotide
<220>
   <221> modified_base
   <222> (3)..(3)
   <223> 7-deaza-dG
<220>
   <221> modified_base
   <222> (7)..(7)
   <223> 7-deaza-dG
<220>
   <221> modified_base
   <222> (11)..(11)
   <223> 7-deaza-dG
<400> 35
   tcgaacgttc g 11
<210> 36
   <211> 11
   <212> DNA
   <213> Artificial Sequence
<220>
   <223> Description of Artificial Sequence: Synthetic oligonucleotide
<220>
   <221> modified_base
   <222> (6)..(6)
   <223> AraG
<400> 36
   cagtcgttca g 11
<210> 37
   <211> 11
   <212> DNA
   <213> Artificial Sequence
<220>
   <223> Description of Artificial Sequence: Synthetic oligonucleotide
<220>
   <221> modified_base
   <222> (6)..(6)
   <223> AraG
<400> 37
   cagtcgttca g 11
<210> 38
   <211> 11
   <212> DNA
   <213> Artificial Sequence
<220>
   <223> Description of Artificial Sequence: Synthetic oligonucleotide
<220>
   <221> modified_base
   <222> (6)..(6)
   <223> AraG
<400> 38
   cagtcgttca g 11
<210> 39
   <211> 11
   <212> DNA
   <213> Artificial Sequence
<220>
   <223> Description of Artificial Sequence: Synthetic oligonucleotide
<220>
   <221> modified_base
   <222> (3)..(3)
   <223> 7-deaza-dG
<220>
   <221> modified_base
   <222> (7)..(7)
   <223> 7-deaza-dG
<220>
   <221> modified_base
   <222> (11)..(11)
   <223> 7-deaza-dG
<400> 39
   tcgaacgttc g 11
<210> 40
   <211> 11
   <212> DNA
   <213> Artificial Sequence
<220>
   <223> Description of Artificial Sequence: Synthetic oligonucleotide
<220>
   <221> modified_base
   <222> (3)..(3)
   <223> 7-deaza-dG
<220>
   <221> modified_base
   <222> (7)..(7)
   <223> 7-deaza-dG
<220>
   <221> modified_base
   <222> (11)..(11)
   <223> 2'-O-methylribonucleotide
<400> 40
   tcgaacgttc g 11
<210> 41
   <211> 11
   <212> DNA
   <213> Artificial Sequence
<220>
   <223> Description of Artificial Sequence: Synthetic oligonucleotide
<220>
   <221> modified_base
   <222> (3)..(3)
   <223> 7-deaza-dG
<220>
   <221> modified_base
   <222> (7)..(7)
   <223> 7-deaza-dG
<220>
   <221> modified_base
   <222> (11)..(11)
   <223> 2'-O-methylribonucleotide
<400> 41
   tcgaacgttc g 11
<210> 42
   <211> 11
   <212> DNA
   <213> Artificial Sequence
<220>
   <223> Description of Artificial Sequence: Synthetic oligonucleotide
<220>
   <221> modified_base
   <222> (3)..(3)
   <223> 7-deaza-dG
<220>
   <221> modified_base
   <222> (7)..(7)
   <223> 7-deaza-dG
<220>
   <221> modified_base
   <222> (11) .. (11)
   <223> 2'-O-methylribonucleotide
<400> 42
   tcgaacgttc g 11
<210> 43
   <211> 11
   <212> DNA
   <213> Artificial Sequence
<220>
   <223> Description of Artificial Sequence: Synthetic oligonucleotide
<220>
   <221> modified_base
   <222> (6)..(6)
   <223> AraG
<400> 43
   cagtcgttca g 11
<210> 44
   <211> 11
   <212> DNA
   <213> Artificial Sequence
<220>
   <223> Description of Artificial Sequence: Synthetic oligonucleotide
<220>
   <221> modified_base
   <222> (3) .. (3)
   <223> 7-deaza-dG
<220>
   <221> modified_base
   <222> (7)..(7)
   <223> 7-deaza-dG
<220>
   <221> modified_base
   <222> (11) .. (11)
   <223> 2'-O-methylribonucleotide
<400> 44
   tcgaacgttc g 11
<210> 45
   <211> 11
   <212> DNA
   <213> Artificial Sequence
<220>
   <223> Description of Artificial Sequence: Synthetic oligonucleotide
<220>
   <221> modified_base
   <222> (3) .. (3)
   <223> 7-deaza-dG
<220>
   <221> modified_base
   <222> (7)..(7)
   <223> 7-deaza-dG
<220>
   <221> modified_base
   <222> (11)..(11)
   <223> 7-deaza-dG
<400> 45
   tcgaacgttc g 11
<210> 46
   <211> 11
   <212> DNA
   <213> Artificial Sequence
<220>
   <223> Description of Artificial Sequence: Synthetic oligonucleotide
<220>
   <221> modified_base
   <222> (7)..(7)
   <223> 7-deaza-dG
<400> 46
   tcagtcgtta c 11
<210> 47
   <211> 11
   <212> DNA
   <213> Artificial Sequence
<220>
   <223> Description of Artificial Sequence: Synthetic oligonucleotide
<220>
   <221> modified_base
   <222> (7)..(7)
   <223> 7-deaza-dG
<400> 47
   tctgtcgttt t 11
<210> 48
   <211> 11
   <212> DNA
   <213> Artificial Sequence
<220>
   <223> Description of Artificial Sequence: Synthetic oligonucleotide
<220>
   <221> modified_base
   <222> (7)..(7)
   <223> 7-deaza-dG
<400> 48
   tcagtcgtta c 11
<210> 49
   <211> 11
   <212> DNA
   <213> Artificial Sequence
<220>
   <223> Description of Artificial Sequence: Synthetic oligonucleotide
<220>
   <221> modified_base
   <222> (7)..(7)
   <223> 7-deaza-dG
<400> 49
   tctgtcgttt t 11
<210> 50
   <211> 8
   <212> DNA
   <213> Artificial Sequence
<220>
   <223> Description of Artificial Sequence: Synthetic oligonucleotide
<220>
   <221> modified_base
   <222> (3)..(3)
   <223> 7-deaza-dG
<220>
   <221> modified_base
   <222> (6)..(6)
   <223> 7-deaza-dG
<400> 50
   tcgtcgtt 8
<210> 51
   <211> 9
   <212> DNA
   <213> Artificial Sequence
<220>
   <223> Description of Combined DNA/RNA molecule: Synthetic oligonucleotide
<220>
   <223> Description of Artificial Sequence: Synthetic oligonucleotide
<220>
   <221> modified_base
   <222> (3) .. (3)
   <223> 7-deaza-dG
<220>
   <221> modified_base
   <222> (6) .. (6)
   <223> 7-deaza-dG
<220>
   <221> modified_base
   <222> (9)..(9)
   <223> 2'-deoxy-U
<400> 51
   tcgtcgttu 9
<210> 52
   <211> 9
   <212> DNA
   <213> Artificial Sequence
<220>
   <223> Description of Combined DNA/RNA molecule: Synthetic oligonucleotide
<220>
   <223> Description of Artificial Sequence: Synthetic oligonucleotide
<220>
   <221> modified_base
   <222> (3)..(3)
   <223> 7-deaza-dG
<220>
   <221> modified_base
   <222> (6)..(6)
   <223> 7-deaza-dG
<220>
   <221> modified_base
   <222> (9)..(9)
   <223> 2'-deoxy-U
<400> 52
   tcgtcgttu 9
<210> 53
   <211> 9
   <212> DNA
   <213> Artificial Sequence
<220>
   <223> Description of Combined DNA/RNA molecule: Synthetic oligonucleotide
<220>
   <223> Description of Artificial Sequence: Synthetic oligonucleotide
<220>
   <221> modified_base
   <222> (3)..(3)
   <223> 7-deaza-dG
<220>
   <221> modified_base
   <222> (6)..(6)
   <223> 7-deaza-dG
<220>
   <221> modified_base
   <222> (9)..(9)
   <223> 2'-deoxy-U
<400> 53
   tcgtcgttu 9
<210> 54
   <211> 9
   <212> DNA
   <213> Artificial Sequence
<220>
   <223> Description of Combined DNA/RNA molecule: Synthetic oligonucleotide
<220>
   <223> Description of Artificial Sequence: Synthetic oligonucleotide
<220>
   <221> modified_base
   <222> (3)..(3)
   <223> 7-deaza-dG
<220>
   <221> modified_base
   <222> (6)..(6)
   <223> 7-deaza-dG
<220>
   <221> modified_base
   <222> (9)..(9)
   <223> 2'-deoxy-U
<400> 54
   tcgtcgttu 9
<210> 55
   <211> 9
   <212> DNA
   <213> Artificial Sequence
<220>
   <223> Description of Combined DNA/RNA molecule: Synthetic oligonucleotide
<220>
   <223> Description of Artificial Sequence: Synthetic oligonucleotide
<220>
   <221> modified_base
   <222> (3) .. (3)
   <223> AraG
<220>
   <221> modified_base
   <222> (6)..(6)
   <223> AraG
<220>
   <221> modified_base
   <222> (9)..(9)
   <223> 2'-deoxy-U
<400> 55
   tcgtcgttu 9
<210> 56
   <211> 9
   <212> DNA
   <213> Artificial Sequence
<220>
   <223> Description of Combined DNA/RNA molecule: Synthetic oligonucleotide
<220>
   <223> Description of Artificial Sequence: Synthetic oligonucleotide
<220>
   <221> modified_base
   <222> (3) .. (3)
   <223> AraG
<220>
   <221> modified_base
   <222> (6)..(6)
   <223> AraG
<220>
   <221> modified_base
   <222> (9)..(9)
   <223> 2'-deoxy-U
<400> 56
   tcgtcgttu 9
<210> 57
   <211> 9
   <212> DNA
   <213> Artificial Sequence
<220>
   <223> Description of Combined DNA/RNA molecule: Synthetic oligonucleotide
<220>
   <223> Description of Artificial Sequence: Synthetic oligonucleotide
<220>
   <221> modified_base
   <222> (3) .. (3)
   <223> AraG
<220>
   <221> modified_base
   <222> (6)..(6)
   <223> AraG
<220>
   <221> modified_base
   <222> (9)..(9)
   <223> 2'-deoxy-U
<400> 57
   tcgtcgttu 9
<210> 58
   <211> 9
   <212> DNA
   <213> Artificial Sequence
<220>
   <223> Description of Combined DNA/RNA molecule: Synthetic oligonucleotide
<220>
   <223> Description of Artificial Sequence: Synthetic oligonucleotide
<220>
   <221> modified_base
   <222> (3)..(3)
   <223> AraG
<220>
   <221> modified_base
   <222> (6)..(6)
   <223> AraG
<220>
   <221> modified_base
   <222> (9)..(9)
   <223> 2'-deoxy-U
<400> 58
   tcgtcgttu 9
<210> 59
   <211> 11
   <212> DNA
   <213> Artificial Sequence
<220>
   <223> Description of Artificial Sequence: Synthetic oligonucleotide
<220>
   <221> modified_base
   <222> (3) .. (3)
   <223> 7-deaza-dG
<220>
   <221> modified_base
   <222> (6)..(6)
   <223> 7-deaza-dG
<220>
   <221> modified_base
   <222> (9)..(9)
   <223> 7-deaza-dG
<400> 59
   tcgtcgacga t 11
<210> 60
   <211> 11
   <212> DNA
   <213> Artificial Sequence
<220>
   <223> Description of Artificial Sequence: Synthetic oligonucleotide
<220>
   <221> modified_base
   <222> (7) .. (7)
   <223> AraG
<400> 60
   tcagtcgtta c 11
<210> 61
   <211> 11
   <212> DNA
   <213> Artificial Sequence
<220>
   <223> Description of Artificial Sequence: Synthetic oligonucleotide
<220>
   <221> modified_base
   <222> (3)..(3)
   <223> 7-deaza-dG
<220>
   <221> modified_base
   <222> (7)..(7)
   <223> 7-deaza-dG
<220>
   <221> modified_base
   <222> (11)..(11)
   <223> 7-deaza-dG
<400> 61
   tcgatcgatc g 11
<210> 62
   <211> 11
   <212> DNA
   <213> Artificial Sequence
<220>
   <223> Description of Artificial Sequence: Synthetic oligonucleotide
<220>
   <221> modified_base
   <222> (3)..(3)
   <223> 7-deaza-dG
<220>
   <221> modified_base
   <222> (7)..(7)
   <223> 7-deaza-dG
<220>
   <221> modified_base
   <222> (11) .. (11)
   <223> 7-deaza-dG
<400> 62
   tcgaacgttc g 11
<210> 63
   <211> 11
   <212> DNA
   <213> Artificial Sequence
<220>
   <223> Description of Artificial Sequence: Synthetic oligonucleotide
<220>
   <221> modified_base
   <222> (3) .. (3)
   <223> 7-deaza-dG
<220>
   <221> modified_base
   <222> (7)..(7)
   <223> 7-deaza-dG
<220>
   <221> modified_base
   <222> (11) .. (11)
   <223> 2'-O-methylribonucleotide
<400> 63
   tcgaacgttc g 11
<210> 64
   <211> 11
   <212> DNA
   <213> Artificial Sequence
<220>
   <223> Description of Artificial Sequence: Synthetic oligonucleotide
<220>
   <221> modified_base
   <222> (3) .. (3)
   <223> 7-deaza-dG
<220>
   <221> modified_base
   <222> (7)..(7)
   <223> 7-deaza-dG
<220>
   <221> modified_base
   <222> (11)..(11)
   <223> 7-deaza-dG
<400> 64
   tcgaacgttc g 11
<210> 65
   <211> 11
   <212> DNA
   <213> Artificial Sequence
<220>
   <223> Description of Artificial Sequence: Synthetic oligonucleotide
<220>
   <221> modified_base
   <222> (6)..(6)
   <223> 7-deaza-dG
<400> 65
   tagtcgtttt t 11
<210> 66
   <211> 11
   <212> DNA
   <213> Artificial Sequence
<220>
   <223> Description of Artificial Sequence: Synthetic oligonucleotide
<400> 66
   tcgtcgttct t 11
<210> 67
   <211> 11
   <212> DNA
   <213> Artificial Sequence
<220>
   <223> Description of Artificial Sequence: Synthetic oligonucleotide
<220>
   <221> modified_base
   <222> (6)..(6)
   <223> AraG
<400> 67
   tggtcgttct t 11
<210> 68
   <211> 11
   <212> DNA
   <213> Artificial Sequence
<220>
   <223> Description of Artificial Sequence: Synthetic oligonucleotide
<220>
   <221> modified_base
   <222> (6)..(6)
   <223> AraG
<400> 68
   tagtcgttct c 11
<210> 69
   <211> 11
   <212> DNA
   <213> Artificial Sequence
<220>
   <223> Description of Artificial Sequence: Synthetic oligonucleotide
<220>
   <221> modified_base
   <222> (3)..(3)
   <223> 7-deaza-dG
<220>
   <221> modified_base
   <222> (6)..(6)
   <223> 7-deaza-dG
<400> 69
   tcgtcgtttt t 11
<210> 70
   <211> 11
   <212> DNA
   <213> Artificial Sequence
<220>
   <223> Description of Artificial Sequence: Synthetic oligonucleotide
<220>
   <221> modified_base
   <222> (3) .. (3)
   <223> 7-deaza-dG
<220>
   <221> modified_base
   <222> (6)..(6)
   <223> 7-deaza-dG
<400> 70
   tcgtcgtttt t 11
<210> 71
   <211> 11
   <212> DNA
   <213> Artificial Sequence
<220>
   <223> Description of Artificial Sequence: Synthetic oligonucleotide
<220>
   <221> modified_base
   <222> (3) .. (3)
   <223> 7-deaza-dG
<220>
   <221> modified_base
   <222> (6)..(6)
   <223> 7-deaza-dG
<400> 71
   tcgtcgtttt t 11
<210> 72
   <211> 11
   <212> DNA
   <213> Artificial Sequence
<220>
   <223> Description of Artificial Sequence: Synthetic oligonucleotide
<220>
   <221> modified_base
   <222> (8)..(8)
   <223> 7-deaza-dG
<400> 72
   tcttgtcgtt c 11
<210> 73
   <211> 11
   <212> DNA
   <213> Artificial Sequence
<220>
   <223> Description of Artificial Sequence: Synthetic oligonucleotide
<220>
   <221> modified_base
   <222> (8)..(8)
   <223> 7-deaza-dG
<400> 73
   tcttgtcgtt c 11
<210> 74
   <211> 11
   <212> DNA
   <213> Artificial Sequence
<220>
   <223> Description of Artificial Sequence: Synthetic oligonucleotide
<220>
   <221> modified_base
   <222> (7)..(7)
   <223> 7-deaza-araG
<400> 74
   tctgtcgttc t 11
<210> 75
   <211> 11
   <212> DNA
   <213> Artificial Sequence
<220>
   <223> Description of Artificial Sequence: Synthetic oligonucleotide
<220>
   <221> modified_base
   <222> (3)..(3)
   <223> 7-deaza-araG
<220>
   <221> modified_base
   <222> (7)..(7)
   <223> 7-deaza-araG
<220>
   <221> modified_base
   <222> (11)..(11)
   <223> 7-deaza-araG
<400> 75
   tcgaacgttc g 11
<210> 76
   <211> 11
   <212> DNA
   <213> Artificial Sequence
<220>
   <223> Description of Artificial Sequence: Synthetic oligonucleotide
<220>
   <221> modified_base
   <222> (3)..(3)
   <223> 7-deaza-dG
<220>
   <221> modified_base
   <222> (6)..(6)
   <223> 7-deaza-dG
<400> 76
   tcgtcgtttt t 11
<210> 77
   <211> 11
   <212> DNA
   <213> Artificial Sequence
<220>
   <223> Description of Artificial Sequence: Synthetic oligonucleotide
<220>
   <221> modified_base
   <222> (3)..(3)
   <223> 7-deaza-dG
<220>
   <221> modified_base
   <222> (6)..(6)
   <223> 7-deaza-dG
<400> 77
   tcgtcgtttt t 11
<210> 78
   <211> 11
   <212> DNA
   <213> Artificial Sequence
<220>
   <223> Description of Artificial Sequence: Synthetic oligonucleotide
<220>
   <221> modified_base
   <222> (3) .. (3)
   <223> 7-deaza-dG
<220>
   <221> modified_base
   <222> (6)..(6)
   <223> 7-deaza-dG
<400> 78
   tcgtcgtttt t 11
<210> 79
   <211> 11
   <212> DNA
   <213> Artificial Sequence
<220>
   <223> Description of Artificial Sequence: Synthetic oligonucleotide
<220>
   <221> modified_base
   <222> (7)..(7)
   <223> 7-deaza-dG
<400> 79
   tctgtcgttc t 11
<210> 80
   <211> 11
   <212> DNA
   <213> Artificial Sequence
<220>
   <223> Description of Artificial Sequence: Synthetic oligonucleotide
<220>
   <221> modified_base
   <222> (3) .. (3)
   <223> 7-deaza-dG
<220>
   <221> modified_base
   <222> (7)..(7)
   <223> 7-deaza-dG
<220>
   <221> modified_base
   <222> (11)..(11)
   <223> 7-deaza-dG
<400> 80
   tcgaacgttc g 11
<210> 81
   <211> 11
   <212> DNA
   <213> Artificial Sequence
<220>
   <223> Description of Artificial Sequence: Synthetic oligonucleotide
<220>
   <221> modified_base
   <222> (6) .. (6)
   <223> AraG
<400> 81
   cagtcgttca g 11
<210> 82
   <211> 11
   <212> DNA
   <213> Artificial Sequence
<220>
   <223> Description of Artificial Sequence: Synthetic oligonucleotide
<220>
   <221> modified_base
   <222> (6)..(6)
   <223> AraG
<400> 82
   cagtcgttca g 11
<210> 83
   <211> 11
   <212> DNA
   <213> Artificial Sequence
<220>
   <223> Description of Artificial Sequence: Synthetic oligonucleotide
<220>
   <221> modified_base
   <222> (6)..(6)
   <223> AraG
<400> 83
   cagtcgttca g 11
<210> 84
   <211> 11
   <212> DNA
   <213> Artificial Sequence
<220>
   <223> Description of Artificial Sequence: Synthetic oligonucleotide
<220>
   <221> modified_base
   <222> (6) .. (6)
   <223> AraG
<400> 84
   cagtcgttca g 11
<210> 85
   <211> 11
   <212> DNA
   <213> Artificial Sequence
<220>
   <223> Description of Artificial Sequence: Synthetic oligonucleotide
<220>
   <221> modified_base
   <222> (6)..(6)
   <223> AraG
<400> 85
   cagtcgttca g 11
<210> 86
   <211> 11
   <212> DNA
   <213> Artificial Sequence
<220>
   <223> Description of Artificial Sequence: Synthetic oligonucleotide
<220>
   <221> modified_base
   <222> (6)..(6)
   <223> AraG
<400> 86
   cagtcgttca g 11
<210> 87
   <211> 11
   <212> DNA
   <213> Artificial Sequence
<220>
   <223> Description of Artificial Sequence: Synthetic oligonucleotide
<220>
   <221> modified_base
   <222> (6)..(6)
   <223> AraG
<400> 87
   cagtcgttca g 11
<210> 88
   <211> 11
   <212> DNA
   <213> Artificial Sequence
<220>
   <223> Description of Artificial Sequence: Synthetic oligonucleotide
<220>
   <221> modified_base
   <222> (6)..(6)
   <223> AraG
<400> 88
   cagtcgttca g 11
<210> 89
   <211> 11
   <212> DNA
   <213> Artificial Sequence
<220>
   <223> Description of Artificial Sequence: Synthetic oligonucleotide
<220>
   <221> modified_base
   <222> (3) .. (3)
   <223> 7-deaza-dG
<220>
   <221> modified_base
   <222> (7)..(7)
   <223> 7-deaza-dG
<220>
   <221> modified_base
   <222> (11) .. (11)
   <223> 7-deaza-dG
<400> 89
   tcgaacgttc g 11
<210> 90
   <211> 11
   <212> DNA
   <213> Artificial Sequence
<220>
   <223> Description of Artificial Sequence: Synthetic oligonucleotide
<220>
   <221> modified_base
   <222> (3)..(3)
   <223> 7-deaza-dG
<220>
   <221> modified_base
   <222> (7)..(7)
   <223> 7-deaza-dG
<220>
   <221> modified_base
   <222> (11) .. (11)
   <223> 7-deaza-dG
<400> 90
   tcgaacgttc g 11
<210> 91
   <211> 11
   <212> DNA
   <213> Artificial Sequence
<220>
   <223> Description of Artificial Sequence: Synthetic oligonucleotide
<220>
   <221> modified_base
   <222> (3) .. (3)
   <223> 7-deaza-dG
<220>
   <221> modified_base
   <222> (7)..(7)
   <223> 7-deaza-dG
<220>
   <221> modified_base
   <222> (11)..(11)
   <223> 2'-O-methylribonucleotide
<400> 91
   tcgaacgttc g 11
<210> 92
   <211> 11
   <212> DNA
   <213> Artificial Sequence
<220>
   <223> Description of Artificial Sequence: Synthetic oligonucleotide
<220>
   <221> modified_base
   <222> (3)..(3)
   <223> 7-deaza-dG
<220>
   <221> modified_base
   <222> (7)..(7)
   <223> 7-deaza-dG
<220>
   <221> modified_base
   <222> (11)..(11)
   <223> 7-deaza-dG
<400> 92
   tcgaacgttc g 11
<210> 93
   <211> 11
   <212> DNA
   <213> Artificial Sequence
<220>
   <223> Description of Combined DNA/RNA molecule: Synthetic oligonucleotide
<220>
   <223> Description of Artificial Sequence: Synthetic oligonucleotide
<220>
   <221> modified_base
   <222> (3)..(3)
   <223> 7-deaza-dG
<220>
   <221> modified_base
   <222> (7)..(7)
   <223> 7-deaza-dG
<220>
   <221> modified_base
   <222> (8) .. (9)
   <223> 2'-deoxy-U
<220>
   <221> modified_base
   <222> (11)..(11)
   <223> 7-deaza-dG
<400> 93
   tcgaacguuc g 11
<210> 94
   <211> 11
   <212> DNA
   <213> Artificial Sequence
<220>
   <223> Description of Combined DNA/RNA molecule: Synthetic oligonucleotide
<220>
   <223> Description of Artificial Sequence: Synthetic oligonucleotide
<220>
   <221> modified_base
   <222> (3)..(3)
   <223> 7-deaza-dG
<220>
   <221> modified_base
   <222> (7)..(7)
   <223> 7-deaza-dG
<220>
   <221> modified_base
   <222> (8)..(9)
   <223> 2'-deoxy-U
<220>
   <221> modified_base
   <222> (11)..(11)
   <223> 2'-O-methylribonucleotide
<400> 94
   tcgaacguuc g 11
<210> 95
   <211> 11
   <212> DNA
   <213> Artificial Sequence
<220>
   <223> Description of Combined DNA/RNA molecule: Synthetic oligonucleotide
<220>
   <223> Description of Artificial Sequence: Synthetic oligonucleotide
<220>
   <221> modified_base
   <222> (3) .. (3)
   <223> 7-deaza-dG
<220>
   <221> modified_base
   <222> (7)..(7)
   <223> 7-deaza-dG
<220>
   <221> modified_base
   <222> (8)..(9)
   <223> 2'-deoxy-U
<220>
   <221> modified_base
   <222> (11)..(11)
   <223> 2'-O-methylribonucleotide
<400> 95
   tcgaacguuc g 11
<210> 96
   <211> 11
   <212> DNA
   <213> Artificial Sequence
<220>
   <223> Description of Combined DNA/RNA molecule: Synthetic oligonucleotide
<220>
   <223> Description of Artificial Sequence: Synthetic oligonucleotide
<220>
   <221> modified_base
   <222> (3) .. (3)
   <223> 7-deaza-dG
<220>
   <221> modified_base
   <222> (7)..(7)
   <223> 7-deaza-dG
<220>
   <221> modified_base
   <222> (8)..(9)
   <223> 2'-deoxy-U
<220>
   <221> modified_base
   <222> (11)..(11)
   <223> 2'-O-methylribonucleotide
<400> 96
   tcgaacguuc g 11
<210> 97
   <211> 11
   <212> DNA
   <213> Artificial Sequence
<220>
   <223> Description of Combined DNA/RNA molecule: Synthetic oligonucleotide
<220>
   <223> Description of Artificial Sequence: Synthetic oligonucleotide
<220>
   <221> modified_base
   <222> (3)..(3)
   <223> 7-deaza-dG
<220>
   <221> modified_base
   <222> (7) .. (7)
   <223> 7-deaza-dG
<220>
   <221> modified_base
   <222> (8)..(9)
   <223> 2'-deoxy-U
<220>
   <221> modified_base
   <222> (11) .. (11)
   <223> 2'-O-methylribonucleotide
<400> 97
   tcgaacguuc g 11
<210> 98
   <211> 11
   <212> DNA
   <213> Artificial Sequence
<220>
   <223> Description of Artificial Sequence: Synthetic oligonucleotide
<220>
   <221> modified_base
   <222> (3) .. (3)
   <223> 7-deaza-dG
<220>.
   <221> modified_base
   <222> (7)..(7)
   <223> 7-deaza-dG
<220>
   <221> modified_base
   <222> (11)..(11)
   <223> 7-deaza-dG
<400> 98
   tcgaacgttc g 11
<210> 99
   <211> 10
   <212> DNA
   <213> Artificial Sequence
<220>
   <223> Description of Artificial Sequence: Synthetic oligonucleotide
<220>
   <221> modified_base
   <222> (3)..(3)
   <223> 7-deaza-dG
<220>
   <221> modified_base
   <222> (6)..(6)
   <223> 7-deaza-dG
<400> 99
   tcgtcgttct 10
<210> 100
   <211> 11
   <212> DNA
   <213> Artificial Sequence
<220>
   <223> Description of Artificial Sequence: Synthetic oligonucleotide
<220>
   <221> modified_base
   <222> (4) .. (4)
   <223> 7-deaza-dG
<220>
   <221> modified_base
   <222> (7)..(7)
   <223> 7-deaza-dG
<220>
   <221> modified_base
   <222> (11) .. (11)
   <223> 7-deaza-dG
<400> 100
   tctgtcgttc g 11
<210> 101
   <211> 11
   <212> DNA
   <213> Artificial Sequence
<220>
   <223> Description of Artificial Sequence: Synthetic oligonucleotide
<220>
   <221> modified_base
   <222> (3)..(3)
   <223> 7-deaza-dG
<220>
   <221> modified_base
   <222> (7)..(7)
   <223> 7-deaza-dG
<220>
   <221> modified_base
   <222> (11)..(11)
   <223> 7-deaza-dG
<400> 101
   tcggtcgttc g 11
<210> 102
   <211> 11
   <212> DNA
   <213> Artificial Sequence
<220>
   <223> Description of Artificial Sequence: Synthetic oligonucleotide
<220>
   <221> modified_base
   <222> (7)..(7)
   <223> 7-deaza-dG
<400> 102
   tctgtcgttc t 11
<210> 103
   <211> 11
   <212> DNA
   <213> Artificial Sequence
<220>
   <223> Description of Artificial Sequence: Synthetic oligonucleotide
<220>
   <221> modified_base
   <222> (7)..(7)
   <223> 7-deaza-dG
<400> 103
   tctgtcgttc t 11
<210> 104
   <211> 11
   <212> DNA
   <213> Artificial Sequence
<220>
   <223> Description of Artificial Sequence: Synthetic oligonucleotide
<220>
   <221> modified_base
   <222> (7)..(7)
   <223> 7-deaza-dG
<400> 104
   tctgtcgttc t 11
<210> 105
   <211> 11
   <212> DNA
   <213> Artificial Sequence
<220>
   <223> Description of Artificial Sequence: Synthetic oligonucleotide
<220>
   <221> modified_base
   <222> (7)..(7)
   <223> 7-deaza-dG
<400> 105
   tctgtcgttc t 11
<210> 106
   <211> 11
   <212> DNA
   <213> Artificial Sequence
<220>
   <223> Description of Artificial Sequence: Synthetic oligonucleotide
<220>
   <221> modified_base
   <222> (6)..(6)
   <223> 7-deaza-araG
<400> 106
   cagtcgttca g 11
<210> 107
   <211> 11
   <212> DNA
   <213> Artificial Sequence
<220>
   <223> Description of Artificial Sequence: Synthetic oligonucleotide
<220>
   <221> modified_base
   <222> (7)..(7)
   <223> 7-deaza-araG
<400> 107
   tctgtcgttc t 11
<210> 108
   <211> 11
   <212> DNA
   <213> Artificial Sequence
<220>
   <223> Description of Artificial Sequence: Synthetic oligonucleotide
<220>
   <221> modified_base
   <222> (7)..(7)
   <223> 7-deaza-dG
<400> 108
   tctgtcgttc t 11
<210> 109
   <211> 11
   <212> DNA
   <213> Artificial Sequence
<220>
   <223> Description of Artificial Sequence: Synthetic oligonucleotide
<220>
   <221> modified_base
   <222> (7)..(7)
   <223> 7-deaza-dG
<400> 109
   tctgtcgttc t 11
<210> 110
   <211> 11
   <212> DNA
   <213> Artificial Sequence
<220>
   <223> Description of Artificial Sequence: Synthetic oligonucleotide
<220>
   <221> modified_base
   <222> (7)..(7)
   <223> 7-deaza-dG
<400> 110
   tctgtcgttc t 11
<210> 111
   <211> 11
   <212> DNA
   <213> Artificial Sequence
<220>
   <223> Description of Artificial Sequence: Synthetic oligonucleotide
<220>
   <221> modified_base
   <222> (7)..(7)
   <223> 7-deaza-dG
<400> 111
   tctgtcgttc t 11
<210> 112
   <211> 11
   <212> DNA
   <213> Artificial Sequence
<220>
   <223> Description of Artificial Sequence: Synthetic oligonucleotide
<220>
   <221> modified_base
   <222> (7)..(7)
   <223> 7-deaza-dG
<400> 112
   tctgtcgttc t 11
<210> 113
   <211> 11
   <212> DNA
   <213> Artificial Sequence
<220>
   <223> Description of Artificial Sequence: Synthetic oligonucleotide
<220>
   <221> modified_base
   <222> (3) .. (3)
   <223> 7-deaza-dG
<220>
   <221> modified_base
   <222> (7)..(7)
   <223> 7-deaza-dG
<220>
   <221> modified_base
   <222> (11) .. (11)
   <223> 7-deaza-dG
<400> 113
   tcgaacgttc g 11
<210> 114
   <211> 11
   <212> DNA
   <213> Artificial Sequence
<220>
   <223> Description of Artificial Sequence: Synthetic oligonucleotide
<220>
   <221> modified_base
   <222> (3) .. (3)
   <223> 7-deaza-dG
<220>
   <221> modified_base
   <222> (7)..(7)
   <223> 7-deaza-dG
<220>
   <221> modified_base
   <222> (11)..(11)
   <223> 7-deaza-dG
<400> 114
   tcgaacgttc g 11
<210> 115
   <211> 11
   <212> DNA
   <213> Artificial Sequence
<220>
   <223> Description of Artificial Sequence: Synthetic oligonucleotide
<220>
   <221> modified_base
   <222> (3)..(3)
   <223> 7-deaza-dG
<220>
   <221> modified_base
   <222> (7)..(7)
   <223> 7-deaza-dG
<220>
   <221> modified_base
   <222> (11) .. (11)
   <223> 7-deaza-dG
<400> 115
   tcgaacgttc g 11
<210> 116
   <211> 11
   <212> DNA
   <213> Artificial Sequence
<220>
   <223> Description of Artificial Sequence: Synthetic oligonucleotide
<220>
   <221> modified_base
   <222> (3) .. (3)
   <223> 7-deaza-dG
<220>
   <221> modified_base
   <222> (7)..(7)
   <223> 7-deaza-dG
<220>
   <221> modified_base
   <222> (11)..(11)
   <223> 7-deaza-dG
<400> 116
   tcgaacgttc g 11
<210> 117
   <211> 11
   <212> DNA
   <213> Artificial Sequence
<220>
   <223> Description of Artificial Sequence: Synthetic oligonucleotide
<220>
   <221> modified_base
   <222> (3)..(3)
   <223> 7-deaza-dG
<220>
   <221> modified_base
   <222> (7)..(7)
   <223> 7-deaza-dG
<220>
   <221> modified_base
   <222> (11) .. (11)
   <223> 7-deaza-dG
<400> 117
   tcgaacgttc g 11
<210> 118
   <211> 11
   <212> DNA
   <213> Artificial Sequence
<220>
   <223> Description of Artificial Sequence: Synthetic oligonucleotide
<220>
   <221> modified_base
   <222> (6) .. (6)
   <223> AraG
<400> 118
   cagtcgttca g 11
<210> 119
   <211> 11
   <212> DNA
   <213> Artificial Sequence
<220>
   <223> Description of Artificial Sequence: Synthetic oligonucleotide
<220>
   <221> modified_base
   <222> (6)..(6)
   <223> AraG
<400> 119
   cagtcgttca g 11
<210> 120
   <211> 11
   <212> DNA
   <213> Artificial Sequence
<220>
   <223> Description of Artificial Sequence: Synthetic oligonucleotide
<220>
   <221> modified_base
   <222> (6)..(6)
   <223> AraG
<400> 120
   cagtcgttca g 11
<210> 121
   <211> 11
   <212> DNA
   <213> Artificial Sequence
<220>
   <223> Description of Artificial Sequence: Synthetic oligonucleotide
<220>
   <221> modified_base
   <222> (6)..(6)
   <223> AraG
<400> 121
   cagtcgttca g 11
<210> 122
   <211> 11
   <212> DNA
   <213> Artificial Sequence
<220>
   <223> Description of Artificial Sequence: Synthetic oligonucleotide
<220>
   <221> modified_base
   <222> (3)..(3)
   <223> 7-deaza-dG
<220>
   <221> modified_base
   <222> (7)..(7)
   <223> 7-deaza-dG
<220>
   <221> modified_base
   <222> (11) .. (11)
   <223> 2'-O-methylribonucleotide
<400> 122
   tcgaacgttc g 11
<210> 123
   <211> 11
   <212> DNA
   <213> Artificial Sequence
<220>
   <223> Description of Artificial Sequence: Synthetic oligonucleotide
<220>
   <221> modified_base
   <222> (7)..(7)
   <223> 7-deaza-dG
<400> 123
   tcagtcgtta c 11
<210> 124
   <211> 11
   <212> DNA
   <213> Artificial Sequence
<220>
   <223> Description of Artificial Sequence: Synthetic oligonucleotide
<220>
   <221> modified_base
   <222> (7)..(7)
   <223> 7-deaza-dG
<400> 124
   tctgtcgtta g 11
<210> 125
   <211> 11
   <212> DNA
   <213> Artificial Sequence
<220>
   <223> Description of Artificial Sequence: Synthetic oligonucleotide
<220>
   <221> modified_base
   <222> (7)..(7)
   <223> 7-deaza-dG
<400> 125
   tctgtcgttt t 11
<210> 126
   <211> 11
   <212> DNA
   <213> Artificial Sequence
<220>
   <223> Description of Artificial Sequence: Synthetic oligonucleotide
<220>
   <221> modified_base
   <222> (7)..(7)
   <223> 7-deaza-dG
<400> 126
   tctgtcgttg t 11
<210> 127
   <211> 11
   <212> DNA
   <213> Artificial Sequence
<220>
   <223> Description of Artificial Sequence: Synthetic oligonucleotide
<220>
   <221> modified_base
   <222> (3)..(3)
   <223> 7-deaza-dG
<220>
   <221> modified_base
   <222> (7)..(7)
   <223> 7-deaza-dG
<220>
   <221> modified_base
   <222> (11)..(11)
   <223> 2'-O-methylribonucleotide
<400> 127
   tcgaacgttc g 11
<210> 128
   <211> 11
   <212> DNA
   <213> Artificial Sequence
<220>
   <223> Description of Artificial Sequence: Synthetic oligonucleotide
<220>
   <221> modified_base
   <222> (3)..(3)
   <223> 7-deaza-dG
<220>
   <221> modified_base
   <222> (7)..(7)
   <223> 7-deaza-dG
<220>
   <221> modified_base
   <222> (11)..(11)
   <223> 7-deaza-dG
<400> 128
   tcgaacgttc g 11
<210> 129
   <211> 11
   <212> DNA
   <213> Artificial Sequence
<220>
   <223> Description of Artificial Sequence: Synthetic oligonucleotide
<220>
   <221> modified_base
   <222> (3)..(3)
   <223> 7-deaza-dG
<220>
   <221> modified_base
   <222> (7)..(7)
   <223> 7-deaza-dG
<220>
   <221> modified_base
   <222> (11)..(11)
   <223> 7-deaza-dG
<400> 129
   tcgaacgttc g 11
<210> 130
   <211> 11
   <212> DNA
   <213> Artificial Sequence
<220>
   <223> Description of Artificial Sequence: Synthetic oligonucleotide
<220>
   <221> modified_base
   <222> (3)..(3)
   <223> 7-deaza-dG
<220>
   <221> modified_base
   <222> (7)..(7)
   <223> 7-deaza-dG
<220>
   <221> modified_base
   <222> (11)..(11)
   <223> 2'-O-methylribonucleotide
<400> 130
   tcgaacgttc g 11
<210> 131
   <211> 11
   <212> DNA
   <213> Artificial Sequence
<220>
   <223> Description of Artificial Sequence: Synthetic oligonucleotide
<220>
   <221> modified_base
   <222> (3)..(3)
   <223> 7-deaza-dG
<220>
   <221> modified_base
   <222> (7)..(7)
   <223> 7-deaza-dG
<220>
   <221> modified_base
   <222> (11)..(11)
   <223> 2'-O-methylribonucleotide
<400> 131
   tcgaacgttc g 11
<210> 132
   <211> 11
   <212> DNA
   <213> Artificial Sequence
<220>
   <223> Description of Artificial Sequence: Synthetic oligonucleotide
<220>
   <221> modified_base
   <222> (3)..(3)
   <223> 7-deaza-dG
<220>
   <221> modified_base
   <222> (6)..(6)
   <223> 7-deaza-dG
<400> 132
   tcgtcgtttt t 11
<210> 133
   <211> 11
   <212> DNA
   <213> Artificial Sequence
<220>
   <223> Description of Artificial Sequence: Synthetic oligonucleotide
<220>
   <221> modified_base
   <222> (3)..(3)
   <223> 7-deaza-dG
<220>
   <221> modified_base
   <222> (6)..(6)
   <223> 7-deaza-dG
<400> 133
   tcgtcgtttt t 11
<210> 134
   <211> 9
   <212> DNA
   <213> Artificial Sequence
<220>
   <223> Description of Combined DNA/RNA molecule: Synthetic oligonucleotide
<220>
   <223> Description of Artificial Sequence: Synthetic oligonucleotide
<220>
   <221> modified_base
   <222> (3)..(3)
   <223> AraG
<220>
   <221> modified_base
   <222> (6)..(6)
   <223> AraG
<220>
   <221> modified_base
   <222> (9)..(9)
   <223> 2'-deoxy-U
<400> 134
   tcgtcgttu 9
<210> 135
   <211> 11
   <212> DNA
   <213> Artificial Sequence
<220>
   <223> Description of Artificial Sequence: Synthetic oligonucleotide
<220>
   <221> modified_base
   <222> (3)..(3)
   <223> 7-deaza-dG
<220>
   <221> modified_base
   <222> (7)..(7)
   <223> 7-deaza-dG
<220>
   <221> modified_base
   <222> (11)..(11)
   <223> 7-deaza-dG
<400> 135
   tcgaacgttc g 11
<210> 136
   <211> 11
   <212> DNA
   <213> Artificial Sequence
<220>
   <223> Description of Artificial Sequence: Synthetic oligonucleotide
<220>
   <221> modified_base
   <222> (7)..(7)
   <223> 7-deaza-dG
<400> 136
   tctgtcgtta c 11
<210> 137
   <211> 11
   <212> DNA
   <213> Artificial Sequence
<220>
   <223> Description of Artificial Sequence: Synthetic oligonucleotide
<220>
   <221> modified_base
   <222> (7)..(7)
   <223> 7-deaza-dG
<400> 137
   tctgtcgttg t 11
<210> 138
   <211> 11
   <212> DNA
   <213> Artificial Sequence
<220>
   <223> Description of Artificial Sequence: Synthetic oligonucleotide
<220>
   <221> modified_base
   <222> (7)..(7)
   <223> 7-deaza-dG
<400> 138
   tcagtcgttc t 11
<210> 139
   <211> 11
   <212> DNA
   <213> Artificial Sequence
<220>
   <223> Description of Artificial Sequence: Synthetic oligonucleotide
<220>
   <221> modified_base
   <222> (3)..(3)
   <223> 7-deaza-dG
<220>
   <221> modified_base
   <222> (7)..(7)
   <223> 7-deaza-dG
<220>
   <221> modified_base
   <222> (11)..(11)
   <223> 7-deaza-dG
<400> 139
   tcgaacgttc g 11
<210> 140
   <211> 11
   <212> DNA
   <213> Artificial Sequence
<220>
   <223> Description of Artificial Sequence: Synthetic oligonucleotide
<220>
   <221> modified_base
   <222> (3)..(3)
   <223> 7-deaza-dG
<220>
   <221> modified_base
   <222> (7)..(7)
   <223> 7-deaza-dG
<220>
   <221> modified_base
   <222> (11)..(11)
   <223> 7-deaza-dG
<400> 140
   tcgaacgttc g 11
<210> 141
   <211> 11
   <212> DNA
   <213> Artificial Sequence
<220>
   <223> Description of Artificial Sequence: Synthetic oligonucleotide
<220>
   <221> modified_base
   <222> (7)..(7)
   <223> 7-deaza-dG
<400> 141
   tctgtcgtta c 11
<210> 142
   <211> 11
   <212> DNA
   <213> Artificial Sequence
<220>
   <223> Description of Artificial Sequence: Synthetic oligonucleotide
<220>
   <221> modified_base
   <222> (7)..(7)
   <223> 7-deaza-dG
<400> 142
   tctgtcgttg t 11
<210> 143
   <211> 11
   <212> DNA
   <213> Artificial Sequence
<220>
   <223> Description of Artificial Sequence: Synthetic oligonucleotide
<220>
   <221> modified_base
   <222> (7)..(7)
   <223> 7-deaza-dG
<400> 143
   tcagtcgtta g 11
<210> 144
   <211> 11
   <212> DNA
   <213> Artificial Sequence
<220>
   <223> Description of Artificial Sequence: Synthetic oligonucleotide
<220>
   <221> modified_base
   <222> (7)..(7)
   <223> 7-deaza-dG
<400> 144
   tcagtcgtta c 11
<210> 145
   <211> 11
   <212> DNA
   <213> Artificial Sequence
<220>
   <223> Description of Artificial Sequence: Synthetic oligonucleotide
<220>
   <221> modified_base
   <222> (7)..(7)
   <223> 7-deaza-dG
<400> 145
   tctgtcgtta g 11
<210> 146
   <211> 11
   <212> DNA
   <213> Artificial Sequence
<220>
   <223> Description of Artificial Sequence: Synthetic oligonucleotide
<220>
   <221> modified_base
   <222> (7)..(7)
   <223> 7-deaza-dG
<400> 146
   tctgtcgttt t 11
<210> 147
   <211> 11
   <212> DNA
   <213> Artificial Sequence
<220>
   <223> Description of Artificial Sequence: Synthetic oligonucleotide
<220>
   <221> modified_base
   <222> (7)..(7)
   <223> 7-deaza-dG
<400> 147
   tctgtcgttg t 11
<210> 148
   <211> 11
   <212> DNA
   <213> Artificial Sequence
<220>
   <223> Description of Artificial Sequence: Synthetic oligonucleotide
<220>
   <221> modified_base
   <222> (6)..(6)
   <223> AraG
<400> 148
   cagtcgttca g 11
<210> 149
   <211> 11
   <212> DNA
   <213> Artificial Sequence
<220>
   <223> Description of Artificial Sequence: Synthetic oligonucleotide
<220>
   <221> modified_base
   <222> (7)..(7)
   <223> 7-deaza-dG
<400> 149
   tcagtcgtta c 11
<210> 150
   <211> 11
   <212> DNA
   <213> Artificial Sequence
<220>
   <223> Description of Artificial Sequence: Synthetic oligonucleotide
<220>
   <221> modified_base
   <222> (7)..(7)
   <223> 7-deaza-dG
<400> 150
   tctgtcgtta g 11
<210> 151
   <211> 11
   <212> DNA
   <213> Artificial Sequence
<220>
   <223> Description of Artificial Sequence: Synthetic oligonucleotide
<220>
   <221> modified_base
   <222> (7)..(7)
   <223> 7-deaza-dG
<400> 151
   tctgtcgttt t 11
<210> 152
   <211> 11
   <212> DNA
   <213> Artificial Sequence
<220>
   <223> Description of Artificial Sequence: Synthetic oligonucleotide
<220>
   <221> modified_base
   <222> (7)..(7)
   <223> 7-deaza-dG
<400> 152
   tctgtcgttg t 11
<210> 153
   <211> 11
   <212> DNA
   <213> Artificial Sequence
<220>
   <223> Description of Artificial Sequence: Synthetic oligonucleotide
<220>
   <221> modified_base
   <222> (3)..(3)
   <223> 7-deaza-dG
<220>
   <221> modified_base
   <222> (7)..(7)
   <223> 7-deaza-dG
<220>
   <221> modified_base
   <222> (11)..(11)
   <223> 7-deaza-dG
<400> 153
   tcgaacgttc g 11
<210> 154
   <211> 11
   <212> DNA
   <213> Artificial Sequence
<220>
   <223> Description of Artificial Sequence: Synthetic oligonucleotide
<220>
   <221> modified_base
   <222> (3)..(3)
   <223> 7-deaza-dG
<220>
   <221> modified_base
   <222> (7)..(7)
   <223> 7-deaza-dG
<220>
   <221> modified_base
   <222> (11)..(11)
   <223> 7-deaza-dG
<400> 154
   tcgaacgttc g 11
<210> 155
   <211> 11
   <212> DNA
   <213> Artificial Sequence
<220>
   <223> Description of Artificial Sequence: Synthetic oligonucleotide
<220>
   <221> modified_base
   <222> (3)..(3)
   <223> 7-deaza-dG
<220>
   <221> modified_base
   <222> (7)..(7)
   <223> 7-deaza-dG
<220>
   <221> modified_base
   <222> (11)..(11)
   <223> 7-deaza-dG
<400> 155
   tcgtacgtac g 11
<210> 156
   <211> 11
   <212> DNA
   <213> Artificial Sequence
<220>
   <223> Description of Artificial Sequence: Synthetic oligonucleotide
<220>
   <221> modified_base
   <222> (3)..(3)
   <223> 7-deaza-dG
<220>
   <221> modified_base
   <222> (7)..(7)
   <223> 7-deaza-dG
<220>
   <221> modified_base
   <222> (11)..(11)
   <223> 7-deaza-dG
<400> 156
   tcgtacgtac g 11
<210> 157
   <211> 11
   <212> DNA
   <213> Artificial Sequence
<220>
   <223> Description of Artificial Sequence: Synthetic oligonucleotide
<220>
   <221> modified_base
   <222> (3)..(3)
   <223> 7-deaza-dG
<220>
   <221> modified_base
   <222> (7)..(7)
   <223> 7-deaza-dG
<220>
   <221> modified_base
   <222> (11)..(11)
   <223> 7-deaza-dG
<400> 157
   tcgtacgtac g 11
<210> 158
   <211> 11
   <212> DNA
   <213> Artificial Sequence
<220>
   <223> Description of Artificial Sequence: Synthetic oligonucleotide
<220>
   <221> modified_base
   <222> (6)..(6)
   <223> AraG
<400> 158
   cagtcgttca g 11
<210> 159
   <211> 11
   <212> DNA
   <213> Artificial Sequence
<220>
   <223> Description of Artificial Sequence: Synthetic oligonucleotide
<220>
   <221> modified_base
   <222> (6)..(6)
   <223> 7-deaza-dG
<400> 159
   tagtcgtttt t 11
<210> 160
   <211> 11
   <212> DNA
   <213> Artificial Sequence
<220>
   <223> Description of Artificial Sequence: Synthetic oligonucleotide
<220>
   <221> modified_base
   <222> (7)..(7)
   <223> 7-deaza-dG
<400> 160
   tctgtcgttc t 11
<210> 161
   <211> 11
   <212> DNA
   <213> Artificial Sequence
<220>
   <223> Description of Artificial Sequence: Synthetic oligonucleotide
<220>
   <221> modified_base
   <222> (7)..(7)
   <223> 7-deaza-dG
<400> 161
   tctgtcgttc t 11
<210> 162
   <211> 11
   <212> DNA
   <213> Artificial Sequence
<220>
   <223> Description of Artificial Sequence: Synthetic oligonucleotide
<220>
   <221> modified_base
   <222> (7)..(7)
   <223> 7-deaza-dG
<400> 162
   tctgtcgttc t 11
<210> 163
   <211> 11
   <212> DNA
   <213> Artificial Sequence
<220>
   <223> Description of Artificial Sequence: Synthetic oligonucleotide
<220>
   <221> modified_base
   <222> (3)..(3)
   <223> 7-deaza-dG
<220>
   <221> modified_base
   <222> (7)..(7)
   <223> 7-deaza-dG
<220>
   <221> modified_base
   <222> (11)..(11)
   <223> 7-deaza-dG
<400> 163
   tcgaacgttc g 11
<210> 164
   <211> 11
   <212> DNA
   <213> Artificial Sequence
<220>
   <223> Description of Artificial Sequence: Synthetic oligonucleotide
<220>
   <221> modified_base
   <222> (3)..(3)
   <223> 7-deaza-dG
<220>
   <221> modified_base
   <222> (7)..(7)
   <223> 7-deaza-dG
<220>
   <221> modified_base
   <222> (11)..(11)
   <223> 7-deaza-dG
<400> 164
   tcgaacgttc g 11
<210> 165
   <211> 11
   <212> DNA
   <213> Artificial Sequence
<220>
   <223> Description of Artificial Sequence: Synthetic oligonucleotide
<220>
   <221> modified_base
   <222> (3)..(3)
   <223> 7-deaza-dG
<220>
   <221> modified_base
   <222> (7)..(7)
   <223> 7-deaza-dG
<220>
   <221> modified_base
   <222> (11)..(11)
   <223> 7-deaza-dG
<400> 165
   tcgaacgttc g 11
<210> 166
   <211> 11
   <212> DNA
   <213> Artificial Sequence
<220>
   <223> Description of Artificial Sequence: Synthetic oligonucleotide
<220>
   <221> modified_base
   <222> (3)..(3)
   <223> 7-deaza-dG
<220>
   <221> modified_base
   <222> (7)..(7)
   <223> 7-deaza-dG
<400> 166
   tcgaacgttc g 11
<210> 167
   <211> 11
   <212> DNA
   <213> Artificial Sequence
<220>
   <223> Description of Combined DNA/RNA molecule: Synthetic oligonucleotide
<220>
   <223> Description of Artificial Sequence: Synthetic oligonucleotide
<220>
   <221> modified_base
   <222> (3)..(3)
   <223> 7-deaza-dG
<220>
   <221> modified_base
   <222> (7)..(7)
   <223> 7-deaza-dG
<220>
   <221> modified_base
   <222> (8)..(9)
   <223> 2'-deoxy-U
<220>
   <221> modified_base
   <222> (11)..(11)
   <223> 2'-O-methylribonucleotide
<400> 167
   tcgaacguuc g 11
<210> 168
   <211> 11
   <212> DNA
   <213> Artificial Sequence
<220>
   <223> Description of Artificial Sequence: Synthetic oligonucleotide
<220>
   <221> modified_base
   <222> (3)..(3)
   <223> 7-deaza-dG
<220>
   <221> modified_base
   <222> (6)..(6)
   <223> 7-deaza-dG
<220>
   <221> modified_base
   <222> (9)..(9)
   <223> 7-deaza-dG
<400> 168
   tcgtcgacga t 11
<210> 169
   <211> 11
   <212> DNA
   <213> Artificial Sequence
<220>
   <223> Description of Artificial Sequence: Synthetic oligonucleotide
<220>
   <221> modified_base
   <222> (3)..(3)
   <223> 7-deaza-dG
<220>
   <221> modified_base
   <222> (7)..(7)
   <223> 7-deaza-dG
<220>
   <221> modified_base
   <222> (11)..(11)
   <223> 7-deaza-dG
<400> 169
   tcgatcgatc g 11
<210> 170
   <211> 11
   <212> DNA
   <213> Artificial Sequence
<220>
   <223> Description of Artificial Sequence: Synthetic oligonucleotide
<220>
   <221> modified_base
   <222> (7)..(7)
   <223> AraG
<400> 170
   tctgtcgtta g 11
<210> 171
   <211> 11
   <212> DNA
   <213> Artificial Sequence
<220>
   .<223> Description of Combined DNA/RNA molecule: Synthetic oligonucleotide
<220>
   <223> Description of Artificial Sequence: Synthetic oligonucleotide
<220>
   <221> modified_base
   <222> (3)..(3)
   <223> 2'-O-methylribonucleotide
<220>
   <221> modified_base
   <222> (7)..(7)
   <223> 7-deaza-dG
<400> 171
   tcugtcgttc t 11
<210> 172
   <211> 15
   <212> DNA
   <213> Artificial Sequence
<220>
   <223> Description of Artificial Sequence: Synthetic oligonucleotide
<220>
   <221> modified_base
   <222> (3)..(3)
   <223> 7-deaza-dG
<220>
   <221> modified_base
   <222> (6)..(6)
   <223> 2'-O-methylribonucleotide
<220>
   <221> modified_base
   <222> (11)..(11)
   <223> 7-deaza-dG
<400> 172
   tcgttctgtc gttct 15
<210> 173
   <211> 11
   <212> DNA
   <213> Artificial Sequence
<220>
   <223> Description of Artificial Sequence: Synthetic oligonucleotide
<220>
   <221> modified_base
   <222> (7)..(7)
   <223> 7-deaza-dG
<220>
   <221> modified_base
   <222> (10)..(11)
   <223> 2'-O-methylribonucleotide
<400> 173
   tctgtcgaca g 11
<210> 174
   <211> 11
   <212> DNA
   <213> Artificial Sequence
<220>
   <223> Description of Combined DNA/RNA molecule: Synthetic oligonucleotide
<220>
   <223> Description of Artificial Sequence: Synthetic oligonucleotide
<220>
   <221> modified_base
   <222> (2)..(3)
   <223> 2'-O-methylribonucleotide
<220>
   <221> modified_base
   <222> (7)..(7)
   <223> 7-deaza-dG
<400> 174
   tcugtcgttc t 11
<210> 175
   <211> 15
   <212> DNA
   <213> Artificial Sequence
<220>
   <223> Description of Combined DNA/RNA molecule: Synthetic oligonucleotide
<220>
   <223> Description of Artificial Sequence: Synthetic oligonucleotide
<220>
   <221> modified_base
   <222> (3)..(3)
   <223> 7-deaza-dG
<220>
   <221> modified_base
   <222> (6)..(7)
   <223> 2'-O-methylribonucleotide
<220>
   <221> modified_base
   <222> (11)..(11)
   <223> 7-deaza-dG
<400> 175
   tcgttcugtc gttct 15
<210> 176
   <211> 14
   <212> DNA
   <213> Artificial Sequence
<220>
   <223> Description of Combined DNA/RNA molecule: Synthetic oligonucleotide
<220>
   <223> Description of Artificial Sequence: Synthetic oligonucleotide
<220>
   <221> modified_base
   <222> (3)..(3)
   <223> 7-deaza-dG
<220>
   <221> modified_base
   <222> (5)..(6)
   <223> 2'-O-methylribonucleotide
<220>
   <221> modified_base
   <222> (11)..(11)
   <223> AraG
<400> 176
   tcgtucagtc gttc 14
<210> 177
   <211> 14
   <212> DNA
   <213> Artificial Sequence
<220>
   <223> Description of Artificial Sequence: Synthetic oligonucleotide
<220>
   <221> modified_base
   <222> (3)..(3)
   <223> 7-deaza-dG
<220>
   <221> modified_base
   <222> (12)..(12)
   <223> AraG
<400> 177
   tcgttcagtc gttc 14
<210> 178
   <211> 15
   <212> DNA
   <213> Artificial Sequence
<220>
   <223> Description of Artificial Sequence: Synthetic oligonucleotide
<220>
   <221> modified_base
   <222> (3)..(3)
   <223> 7-deaza-dG
<220>
   <221> modified_base
   <222> (6).. (6)
   <223> 2'-O-methylribonucleotide
<220>
   <221> modified_base
   <222> (11)..(11)
   <223> 7-deaza-dG
<400> 178
   tcgttctgtc gttgt 15
<210> 179
   <211> 11
   <212> DNA
   <213> Artificial Sequence
<220>
   <223> Description of Artificial Sequence: Synthetic oligonucleotide
<220>
   <221> modified_base
   <222> (6)..(6)
   <223> 7-deaza-dG
<400> 179
   tatgcgtttt t 11
<210> 180
   <211> 11
   <212> DNA
   <213> Artificial Sequence
<220>
   <223> Description of Artificial Sequence: Synthetic oligonucleotide
<220>
   <221> modified_base
   <222> (2)..(2)
   <223> 2'-O-methylribonucleotide
<220>
   <221> modified_base
   <222> (7)..(7)
   <223> 7-deaza-dG
<400> 180
   tctgtcgttc t 11
<210> 181
   <211> 15
   <212> DNA
   <213> Artificial Sequence
<220>
   <223> Description of Artificial Sequence: Synthetic oligonucleotide
<220>
   <221> modified_base
   <222> (6)..(6)
   <223> 2'-O-methylribonucleotide
<220>
   <221> modified_base
   <222> (11)..(11)
   <223> 7-deaza-dG
<400> 181
   tccttctgtc gttct 15
<210> 182
   <211> 11
   <212> DNA
   <213> Artificial Sequence
<220>
   <223> Description of Artificial Sequence: Synthetic oligonucleotide
<220>
   <221> modified_base
   <222> (6)..(6)
   <223> AraG
<400> 182
   cattcgttca g 11
<210> 183
   <211> 11
   <212> DNA
   <213> Artificial Sequence
<220>
   <223> Description of Artificial Sequence: Synthetic oligonucleotide
<220>
   <221> modified_base
   <222> (6)..(6)
   <223> AraG
<400> 183
   cattcgttca g 11
<210> 184
   <211> 11
   <212> DNA
   <213> Artificial Sequence
<220>
   <223> Description of Artificial Sequence: Synthetic oligonucleotide
<220>
   <221> modified_base
   <222> (3)..(3)
   <223> 7-deaza-dG
<220>
   <221> modified base
   <222> (7)..(7)
   <223> 7-deaza-dG
<400> 184
   tcggtcgttc t 11
<210> 185
   <211> 11
   <212> DNA
   <213> Artificial Sequence
<220>
   <223> Description of Artificial Sequence: Synthetic oligonucleotide
<220>
   <221> modified_base
   <222> (7)..(7)
   <223> 7-deaza-dG
<220>
   <221> modified_base
   <222> (11)..(11)
   <223> 7-deaza-dG
<400> 185
   tctgtcgttc g 11
<210> 186
   <211> 14
   <212> DNA
   <213> Artificial Sequence
<220>
   <223> Description of Artificial Sequence: Synthetic oligonucleotide
<220>
   <221> modified_base
   <222> (3)..(3)
   <223> 7-deaza-dG
<220>
   <221> modified_base
   <222> (6)..(6)
   <223> 2'-O-methylribonucleotide
<220>
   <221> modified_base
   <222> (11)..(11)
   <223> AraG
<400> 186
   tcgttcagtc gttc 14
<210> 187
   <211> 14
   <212> DNA
   <213> Artificial Sequence
<220>
   <223> Description of Artificial Sequence: Synthetic oligonucleotide
<220>
   <221> modified_base
   <222> (3)..(3)
   <223> 7-deaza-dG
<220>
   <221> modified_base
   <222> (11)..(11)
   <223> AraG
<400> 187
   tcgttcagtc gttc 14
<210> 188
   <211> 15
   <212> DNA
   <213> Artificial Sequence
<220>
   <223> Description of Artificial Sequence: Synthetic oligonucleotide
<220>
   <221> modified_base
   <222> (3)..(3)
   <223> 7-deaza-dG
<220>
   <221> modified_base
   <222> (6)..(6)
   <223> 2'-O-methylribonucleotide
<220>
   <221> modified_base
   <222> (11)..(11)
   <223> 7-deaza-dG
<400> 188
   tcgttctgtc gttac 15
<210> 189
   <211> 15
   <212> DNA
   <213> Artificial Sequence
<220>
   <223> Description of Combined DNA/RNA molecule: Synthetic oligonucleotide
<220>
   <223> Description of Artificial Sequence: Synthetic oligonucleotide
<220>
   <221> modified_base
   <222> (3)..(3)
   <223> 7-deaza-dG
<220>
   <221> modified_base
   <222> (6)..(7)
   <223> 2'-O-methylribonucleotide
<220>
   <221> modified_base
   <222> (11)..(11)
   <223> 7-deaza-dG
<400> 189
   tcgttcugtc gttgt 15
<210> 190
   <211> 15
   <212> DNA
   <213> Artificial Sequence
<220>
   <223> Description of Combined DNA/RNA molecule: Synthetic oligonucleotide
<220>
   <223> Description of Artificial Sequence: Synthetic oligonucleotide
<220>
   <221> modified_base
   <222> (3)..(3)
   <223> 7-deaza-dG
<220>
   <221> modified_base
   <222> (6)..(7)
   <223> 2'-O-methylribonucleotide
<220>
   <221> modified_base
   <222> (11)..(11)
   <223> 7-deaza-dG
<400> 190
   tcgttcugtc gttga 15
<210> 191
   <211> 15
   <212> DNA
   <213> Artificial Sequence
<220>
   <223> Description of Combined DNA/RNA molecule: Synthetic oligonucleotide
<220>
   <223> Description of Artificial Sequence: Synthetic oligonucleotide
<220>
   <221> modified_base
   <222> (3)..(3)
   <223> 7-deaza-dG
<220>
   <221> modified_base
   <222> (6)..(7)
   <223> 2'-O-methylribonucleotide
<220>
   <221> modified_base
   <222> (11)..(11)
   <223> 7-deaza-dG
<400> 191
   tcgttcugtc gttac 15